(19) Europäisches Patentamt / European Patent Office / Office européen des brevets

(11) EP 2 734 212 B1

(12) **EUROPEAN PATENT SPECIFICATION**

(45) Date of publication and mention of the grant of the patent:
**06.09.2017 Bulletin 2017/36**

(21) Application number: **12814382.3**

(22) Date of filing: **19.07.2012**

(51) Int Cl.:
***A61K 31/196*** *(2006.01)* ***A61K 31/724*** *(2006.01)*
***A61K 9/00*** *(2006.01)* ***A61K 9/08*** *(2006.01)*
***A61K 47/40*** *(2006.01)* ***A61P 25/00*** *(2006.01)*
***A61P 43/00*** *(2006.01)*

(86) International application number:
**PCT/US2012/047453**

(87) International publication number:
**WO 2013/013076 (24.01.2013 Gazette 2013/04)**

# (54) COMPOSITIONS COMPRISING DICLOFENAC FOR THE TREATMENT OF POST-OPERATIVE PAIN

ZUSAMMENSETZUNGEN ENTHALTEND DICLOFENAC ZUR BEHANDLUNG VON POSTOPERATIVEN SCHMERZEN

COMPOSITIONS CONTENANT DU DICLOFENAC DESTINÉES AU TRAITEMENT DES DOULEURS POSTOPERATIVES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.07.2011 US 201161509886 P**

(43) Date of publication of application:
**28.05.2014 Bulletin 2014/22**

(60) Divisional application:
**17179857.2**

(73) Proprietor: **Hospira, Inc.**
**Lake Forest, Illinois 60045 (US)**

(72) Inventors:
- **LACOUTURE, Peter**
  **Gurnee, IL 60031 (US)**
- **GARCIA DE ROCHA, Marcelo**
  **Buffalo Grove, IL 60089 (US)**
- **CARR, Daniel, B.**
  **Chestnut Hill, MA 02467 (US)**

(74) Representative: **Atkinson, Jennifer**
**Barker Brettell LLP**
**100 Hagley Road**
**Edgbaston**
**Birmingham B16 8QQ (GB)**

(56) References cited:
**EP-A1- 0 647 451** **EP-A1- 1 574 221**
**WO-A1-2009/089269** **US-A1- 2007 232 566**
**US-A1- 2007 232 567** **US-A1- 2009 292 022**

- **AMMON ET AL.: 'Diclofenac does not Interact with Codeine Metabolism in Vivo: A Study of Healthy Volunteers' BMC CLINICAL PHARMACOLOGY vol. 2, no. 2, 27 February 2002, pages 1 - 10, XP021017385**
- **CHRISTENSEN ET AL.: 'A Double-Blind Placebo-Controlled Comparison of a Novel Formulation of Intravenous Diclofenac and Ketorolac for Postoperative Third Molar Extraction Pain' ANESTH. PROG. vol. 58, 22 June 2011, pages 73 - 81, XP055142439**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The presently disclosed subject matter is directed to a pharmaceutical composition for use in treating post-operative moderate and severe pain by administering diclofenac or a pharmaceutically acceptable diclofenac salt formulated with hydroxypropyl-beta-cyclodextrin (HPβCD).

## BACKGROUND OF THE INVENTION

Opioids are commonly used for pain management, but this class of drugs has significant deleterious effects. Excessive reliance on opioids for postoperative analgesia may increase morbidity due to dose-related side effects and because of the potential for rapid development of acute tolerance and hyperalgesia.

Recently, multimodal analgesia, in which a nonsteroidal anti-inflammatory drug (NSAID) is paired with an opioid, has become standard clinical practice for the control of moderate to severe acute postoperative pain. Multimodal analgesia results in less opioid consumption, and consequent decreases in postoperative nausea, vomiting, and sedation.

NSAIDs are integral to the management of acute postoperative pain. This is especially true in the context of "fast-track" surgery, which combines epidural or regional anesthesia, minimally invasive techniques, optimal pain control, and aggressive postoperative rehabilitation, to significantly enhance recovery and reduce morbidity. Most surgeries currently being performed are ambulatory or performed on a short-stay basis. Unrelieved pain may delay patient discharge, and it is a common reason for unplanned admissions and re-admissions.

Parenteral NSAIDs are often preferred when patients cannot tolerate or are unable to take oral medications and when they require rapid onset of analgesia. Multimodal anesthesia is important to accelerate discharge and reduce opioid-related side effects in fast-track surgery. A recent systematic review concluded that only NSAIDs and ketamine decrease postoperative pain intensity while reducing concurrent opioid requirements. See Liu et al. Anesth. Analg. 104(3): 689-702 (2007).

Not all NSAIDs are efficacious for moderate to severe pain, and not all NSAIDs are able to achieve clinically meaningful reductions in opioid requirements. Some formulations of acetaminophen and NSAIDs require preparation and/or slow infusion of each dose. Ketorolac is one of the most commonly used injectable non-narcotic analgesics in North America. Ketorolac, although efficacious as a single agent given by rapid intravenous (IV) bolus, interferes with platelet aggregation and increases the risk of bleeding to such extents that dosage reductions are mandatory in at-risk populations such as the elderly.

Diclofenac is a well-known non-steroidal anti-inflammatory drug ("NSAID") used in acute and chronic pain in both parenteral and oral dosage forms. Oral dosages range from 100-200 mg/day, while parenteral dosages range from 75-150 mg/day (1-2 mg/kg/day) by either infusion or intermittent (divided) doses. Toxicity of oral and parenteral forms of diclofenac are well known, with gastrointestinal, hemorrhagic, renal, hepatic, cardiovascular and allergic (anaphylactic and severe dermal allergy) adverse events being most significant.

A currently approved formulation of diclofenac is Voltarol™, an injectable form of diclofenac. The use of this product is limited by the preparation and administration requirements including dilution, buffering with sodium bicarbonate solution, instability and consequent need for immediate administration following preparation, with administration times ranging from 30 minutes to 2 hours. The poor solubility of diclofenac has limited the parenteral use of Voltarol™ to intramuscular (IM) use and/or slow IV administration of diluted (100-500 ml diluent) product. In addition, this formulation employs organic solvents, such as propylene glycol and benzyl alcohol, in order to increase the solubility of diclofenac. Each of these excipients is a known vascular irritant and can cause pain on injection.

U.S. 5,679,660 and U.S. 2005/0238674 A1, disclose novel formulations of diclofenac with hydroxypropyl-beta-cyclodextrin (HPβCD). These formulations allow a more concentrated preparation and thus rapid intravenous administration.

WO2009/089269 discloses methods of treating pain while minimizing effects on platelet function that include the step of administering to the subject a pharmaceutical composition comprising a diclofenac compound and a beta-cyclodextrin.

US2007/232566 discloses a pharmaceutical composition containing a unit dose of a diclofenac compound effective to induce analgesia; and a beta-cyclodextrin compound; wherein the dose of the diclofenac compound is less than 10 mg.

EP1574221 discloses stable parenteral aqueous solutions comprising either (a) diclofenac or a pharmaceutically acceptable diclofenac salt and a cyclodextrin, or (b) an inclusion complex of diclofenac or a pharmaceutically acceptable diclofenac salt and a cyclodextrin, or a mixture of (a) and (b), which are suitable for intramuscular and intravenous administration.

There remains a need in the art for an NSAID formulation effective at a low dose to avoid toxic effects and to be suitable for administration to high-risk populations requiring specialized dosing regimens. Such populations include, but are not limited to elderly patients, obese patients, and cancer patients. There is also a need for an NSAID that is

effective to treat severe pain as well as moderate pain and to reduce the need for opioid treatment. In addition, it would be advantageous to provide an NSAID with at least the same efficacy in treating pain as ketorolac, but with fewer side effects.

The presently disclosed subject matter arises, in part, from the discovery that a parenteral formulation of diclofenac sodium with beta-cyclodextrin can treat moderate to severe cancer pain and post-operative pain. The presently disclosed subject matter also arises from the discovery that diclofenac doses below the minimum approved doses are both effective and safe in specialized patient populations.

## SUMMARY OF THE INVENTION

The presently disclosed subject matter provides a pharmaceutical composition comprising 50mg or less of diclofenac or a pharmaceutically acceptable diclofenac salt; and hydroxypropyl-beta-cyclodextrin (HPβCD); for use in treating post-operative moderate to severe pain assessed by a visual analog scale (VAS), resulting from orthopedic surgery in a mammal having hepatic or renal impairment, wherein the composition is intended for parenteral administration, wherein moderate to severe pain is a pain intensity of≥50 mm on a 1-100 mm VAS.

According to another aspect of the invention, there is provided a presently disclosed subject matter provides a pharmaceutical composition comprising 50mg or less of diclofenac or a pharmaceutically acceptable diclofenac salt; and hydroxypropyl-beta-cyclodextrin (HPβCD); for use in treating post-operative moderate to severe pain assessed by a visual analog scale (VAS), resulting from abdominal or pelvic surgery in a mammal having hepatic or renal impairment, wherein the composition is intended for parenteral administration, wherein moderate to severe pain is a pain intensity of ≥ 50 mm on a 1-100 mm VAS.

Pain relief may be provided to the mammal within about ten minutes after administration. In certain embodiments, the pharmaceutical composition is intended to be administered f with an amount of rescue medication. The amount of rescue medication may be reduced by at least about 40% within a time period selected from the group consisting of about 24 hours post-administration, about 48 hours post-administration, about 72 hours post-administration, about 96 hours post-administration, and about 120 hours post-administration.

The description also discloses a method of treating post-operative pain resulting from abdominal or pelvic surgery in a mammal, wherein the method comprises parenterally administering to the mammal in need thereof a pharmaceutical composition of about 50 mg or less of a diclofenac compound and a beta-cyclodextrin compound. In a specific embodiment, the mammal is a human subject who weighs at least about 210 lbs. (95 kg). In one embodiment, the mammal has a high-risk of an adverse reaction to analgesia. In particular embodiments, the mammal is a human subject who is about 65 years or older. In particular embodiments, the mammal is suffering from moderate to severe pain.

In one embodiment, the pharmaceutical composition comprises 37.5 mg or less of diclofenac or a pharmaceutically acceptable diclofenac salt. In another embodiment, the pharmaceutical composition comprises 18.75 mg or less of diclofenac or a pharmaceutically acceptable diclofenac salt. In certain embodiments, the pharmaceutical composition comprises 9.375 mg or less of diclofenac or a pharmaceutically acceptable diclofenac salt.

In one embodiment, the presently disclosed subject matter provides a a pharmaceutical composition which comprises 37.5 mg or less of a diclofenac or a pharmaceutically acceptable diclofenac salt.

## DESCRIPTION OF THE FIGURES

Figure 1 depicts a graphical representation of the mean sum of pain intensity differences over time. The tested formulations include placebo (dark grey bar), ketorolac (light grey bar), and diclofenac (black bar). "*" represents that the P value is < 0.0001 versus placebo.

Figure 2 depicts the cumulative amount of rescue medication administered over time. The tested formulations include placebo (dark grey bar), ketorolac (light grey bar), and diclofenac (black bar). "*" represents that the P value is < 0.0001 versus placebo. "#" represents that the P value is < 0.05 versus ketorolac.

Figure 3 depicts the percentage of subjects in each treatment group who did not require rescue medication over time. The tested formulations include placebo, ketorolac, and diclofenac.

Figure 4 depicts the patient global evaluation at last assessment. The graph shows the percentage of total patients expressing evaluations of "excellent," "very good," "good," "fair," and "poor" for each of placebo, ketorolac, and diclofenac.

Figure 5 depicts the mean amount of rescue morphine administered (mg) per day post surgery for the first three days for each of placebo, ketorolac, 18.75 mg diclofenac, and 37.5 mg diclofenac.

Figure 6 represents the study timeline in Example 10.

Figure 7 represents the distribution of patients in study groups and reasons for study withdrawal in Example 10.

Figure 8 depicts a graphical representation of the mean sum of pain intensity differences over time in Example 10. The tested formulations include placebo, ketorolac 30 mg, diclofenac 18.75 mg, and diclofenac 37.5 mg.

Figure 9 depicts the mean amount of rescue morphine administered over time in Example 10. The tested formulations include placebo, ketorolac 30 mg, diclofenac 18.75 mg, and diclofenac 37.5 mg.

Figure 10 represents the distribution of patients randomized in Example 11.

Figure 11 depicts the patient global evaluation at last assessment in Example 11. The graph shows the percentage of total patients expressing evaluations of "excellent," "very good," "good," "fair," and "poor" for each of placebo, ketorolac, and diclofenac.

Figure 12 depicts a graphical representation of the mean sum of pain intensity differences over time in Example 11. The tested formulations include placebo (dark grey bar), ketorolac (light grey bar), and diclofenac (black bar). "*" represents that the P value is < 0.05 versus placebo. "#" represents that the P value is < 0.05 versus ketorolac.

Figures 13A and B depict the efficacy of ketorolac in high-risk elderly patients over time compared to that of placebo and diclofenac as shown in Example 11. Figure 13A depicts the percentage of patients attaining at least 30% reduction in pain intensity across treatments. The tested formulations include placebo (dark grey bar), ketorolac (light grey bar), and diclofenac (black bar). "*" represents the P value is < 0.05 versus placebo. Figure 13B depicts the mean amount of rescue medication by treatment versus placebo over time. "#" represents the P value is < 0.05 versus ketorolac.

Figure 14 represents the distribution of patients in study groups and reasons for study withdrawal in Example 12.

Figure 15 represents the distribution of patients assigned to age and weight cohorts in Study 1 of Example 13.

Figure 16A and B depict the effect of age and weight on diclofenac PK as shown in Example 13. Figure 16A depicts the mean plasma concentrations over time of diclofenac after IV administration of 18.75 mg of HPβCD-diclofenac to three age-based cohorts. Figure 16B depicts the mean plasma concentrations over time of diclofenac after IV administration of 37.5 mg of HPβCD-diclofenac to five weight-based cohorts.

Figure 17A and B depict the relationships between age, and diclofenac PK parameters as shown in Example 13. Figure 17A depicts the relationship between volume of distribution and age after intravenous administration of 18.75 mg or 37.5 mg of HPβCD-diclofenac of Example 13. Figure 17B depicts the relationship between terminal elimination half-life and age after intravenous administration of 18.75 mg or 37.5 mg of HPβCD-diclofenac.

Figure 18A and B depict the pharmacokinetics of HPβCD-diclofenac in subjects with renal impairment as shown in Example 13. Figure 18A depicts the mean plasma concentrations over time of diclofenac after IV administration of 37.5 mg of HPβCD-diclofenac to subjects with mild or moderate renal impairment and to healthy subjects. Figure 18B shows the mean plasma concentrations over time of the excipient, HPβCD, after IV administration of 37.5 mg of HPβCD-diclofenac to subjects with mild or moderate renal impairment and to healthy subjects.

Figure 19A and B depict the pharmacokinetics of HPβCD-diclofenac in subjects with hepatic impairment as shown in Example 13. Figure 19A shows the mean plasma concentrations over time of diclofenac after IV administration of 37.5 mg of HPβCD-diclofenac to subjects with mild hepatic impairment and to healthy subjects. Figure 19B shows the mean plasma concentrations over time of the excipient, HPβCD, after IV administration of 37.5 mg of HPβCD-diclofenac to healthy subjects.

## DETAILED DESCRIPTION OF THE INVENTION

The presently disclosed subject matter provides methods of treating specialized populations of mammals in need of analgesia by administering a combination of a low dose of diclofenac compound and beta-cyclodextrin. In specific embodiments, the mammals have post-operative pain. In particular embodiments, the specialized patient populations are high-risk and/or obese mammals.

The presently disclosed subject matter is based, in part, on the results of a comparison of the efficacy of diclofenac solubilized with HPβCD to ketorolac and placebo for the treatment of moderate-to-severe post-surgical pain. The efficacy of diclofenac solubilized with HPβCD at several dose levels suggests a faster onset of action. Most notably, diclofenac formulated with HPβCD provides single-dose efficacy at about 67%, 50%, about 25%, about 12.5% and about 5% of the current recommended doses of diclofenac. This, in combination with the human pharmacokinetic results for the formulation, supports reduced total daily doses of this NSAID with anticipated lower risk of toxicity by reducing the extent and duration of drug exposure. This finding holds clinical importance.

### Definitions

The "pharmaceutical composition" as used in accordance with the presently disclosed subject matter relates to compositions that can be formulated in any conventional manner using one or more pharmaceutically acceptable carriers or excipients. A "pharmaceutically acceptable" carrier or excipient, as used herein, means approved by a regulatory agency of the Federal or a state government, or as listed in the U.S. Pharmacopoeia or other generally recognized

pharmacopoeia for use in mammals, and more particularly in humans.

The term "dosage" is intended to encompass a formulation expressed in terms of mg/kg/day. The dosage is the amount of an ingredient administered in accordance with a particular dosage regimen. A "dose" is an amount of an agent administered to a mammal in a unit volume or mass, *e.g.*, an absolute unit dose expressed in mg of the agent. The dose depends on the concentration of the agent in the formulation, *e.g.*, in moles per liter (M), mass per volume (m/v), or mass per mass (m/m). The two terms are closely related, as a particular dosage results from the regimen of administration of a dose or doses of the formulation. The particular meaning in any case will be apparent from context.

The term "mammal" is intended to include, any warm-blooded vertebrate having the skin more or less covered with hair. The mammal can be a non-human animal. Examples of animals include a domestic pet, such as a canine or feline, a farm animal, a work animal, or an animal in a circus or zoological garden. Most preferably, the mammal is a human.

As used herein, the term "high-risk" refers to mammals with a high risk of an adverse reaction to analgesia. Examples of high-risk human subjects include those who weigh less than about 110 lbs. (50 kg), are about 65 years or older, are undergoing medical ulcer therapy, have cancer, are immunocompromised, or who have a Child-Pugh score of from about 6 to about 9, a serum creatinine of from about 1.9 to about 3.0 mg/dL, a history of gastrointestinal bleeding or perforation, renal impairment, hepatic impairment, or concomitant anticoagulant use. As used herein, the term "renal impairment" refers to a screening serum creatinine value greater than the normal range, which is 1.1 mg/dL for women and 1.3 mg/dL for men, or a urine cratinine value greater than the normal range, which is about 300 mg/dL. As used herein, the term "hepatic impairment" refers to a screening total bilirubin value greater than the normal range for the laboratory, which is 1.2 mg/dL for both women and men.

The term "PGE" refers to the patient's global estimate of improvement. PGE refers to the patient's global estimate of treatment. PGE is an assessment that is made by the patient which is intended to capture the overall experience with the treatment received. It is designed to allow the patient to consider factors such as effectiveness, side effects, convenience, and ease of treatment application.

The term "minimum approved dose" refers to the minimum dosage that has received full regulatory approval by the appropriate United States or foreign regulatory authority as safe and effective for human or veterinary use.

The term "therapeutically effective" as applied to dose or amount refers to that quantity of a compound or pharmaceutical composition that is sufficient to result in a desired activity upon administration to a mammal in need thereof As used herein, the term "therapeutically effective amount/dose" refers to the amount/dose of a compound or pharmaceutical composition that is sufficient to produce an analgesic response upon administration to a mammal.

The term "amount" as used herein refers to quantity or to concentration as appropriate to the context. In the presently disclosed subject matter, the effective amount of a compound refers to an amount sufficient to treat a patient/mammal in need of analgesia. The effective amount of a drug that constitutes a therapeutically effective amount varies according to factors such as the potency of the particular drug, the route of administration of the formulation, and the mechanical system used to administer the formulation. A therapeutically effective amount of a particular drug can be selected by those of ordinary skill in the art with due consideration of such factors.

The term "about" or "approximately" means within an acceptable error range for the particular value as determined by one of ordinary skill in the art, which will depend in part on how the value is measured or determined, *i.e.,* the limitations of the measurement system. For example, "about" can mean within 3 or more than 3 standard deviations, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value.

As used herein, the term "treat" is used herein to mean to at least partially relieve, alleviate, reduce the extent of, and/or reduce the duration of at least one symptom of a pain in a mammal. Within the meaning of the presently disclosed subject matter, the term "treat" also denotes to arrest, delay the onset (*i.e.,* the period prior to clinical manifestation of a disease) and/or reduce the risk of developing or worsening at least one symptom of a pain in a mammal.

**Pharmaceutical Compositions**

The pharmaceutical compositions of the presently disclosed subject matter comprise diclofenac or a pharmaceutically acceptable diclofenac salt. A pharmaceutically acceptable salt of diclofenac, can be an alkali metal salt, for example the sodium or the potassium salt, or the salt formed with an amine, *e.g.,* a mono-, di- or tri-$C_1$-$C_4$ alkylamine, for example diethyl- or triethyl-amine, hydroxy-$C_2$-$C_4$ alkylamine, for example ethanolamine, or hydroxy-$C_2$-$C_4$ alkyl-$C_1$-$C_4$ alkylamine, for example dimethylethanolamine, or a quaternary ammonium salt, for example the tetramethylammonium salt or the choline salt of diclofenac (see, *e.g.,* U.S. Pat. No. 5,389,681). Preferably the diclofenac salt is diclofenac sodium.

In certain embodiments, the pharmaceutical composition comprises 50 mg or less of diclofenac or a pharmaceutically acceptable diclofenac salt. In particular embodiments, the pharmaceutical composition comprises 45 mg or

less of diclofenac or a pharmaceutically acceptable diclofenac salt. In certain embodiments, the pharmaceutical composition comprises 40 mg or less of diclofenac or a pharmaceutically acceptable diclofenac salt. In particular embodiments, the pharmaceutical composition comprises 37.5 mg or less of diclofenac or a pharmaceutically acceptable diclofenac salt. In certain embodiments, the pharmaceutical composition comprises 30 mg or less of diclofenac or a pharmaceutically acceptable diclofenac salt. In particular embodiments, the pharmaceutical composition comprises 25 mg or less of diclofenac or a pharmaceutically acceptable diclofenac salt. In one embodiment, the pharmaceutical composition comprises 20 mg or less of diclofenac or a pharmaceutically acceptable diclofenac salt. In certain embodiments, the pharmaceutical composition comprises 18.75 mg or less of diclofenac or a pharmaceutically acceptable diclofenac salt. In one embodiment, the pharmaceutical composition comprises 15 mg or less of diclofenac or a pharmaceutically acceptable diclofenac salt. In certain embodiments, the pharmaceutical composition comprises 10 mg or less of diclofenac or a pharmaceutically acceptable diclofenac salt. In particular embodiments, the pharmaceutical composition comprises 9.375 mg or less of diclofenac or a pharmaceutically acceptable diclofenac salt. In one embodiment, the pharmaceutical composition comprises 5 mg or less of diclofenac or a pharmaceutically acceptable diclofenac salt. The 18.75 mg and 50 mg diclofenac doses are about 25% and 67%, respectively, of the minimum approved dose and about 12.5% or 33%, respectively, of the approved daily dosage

Suitable formulations of the presently disclosed subject matter for parenteral administration include cyclodextrin inclusion complexes. One or more modified or unmodified cyclodextrins can be employed to stabilize and increase the water solubility and efficacy of compounds of the presently disclosed subject matter. A useful cyclodextrin for this purpose includes Hydroxypropyl-beta-cyclodextrin (HPβCD). The formulations can be prepared as described in U.S. Patent Nos. 5,679,660 and 5,674,854.

Hydroxypropyl-beta-cyclodextrin (HPβCD) is a cyclic, glucose-derived oligomer consisting of linked alpha-1,4-glucose units. These glucose oligomers form a cone-like cavity into which poorly water-soluble compounds can enter, forming a water-soluble complex. The advantages of solubilizing diclofenac with HPβCD include a reduction in dosing volume, reduction in irritation from the high or low pH or the use of organic solvents needed for solubilization, and avoiding direct venous irritation from the drug itself. In one embodiment, the ratio of diclofenac sodium to HPβCD is about 1:0.5 to about 1:10. In one embedment, the ratio of diclofenac sodium to HPβCD is about 1:8.9. In particular embodiments, the ratio of diclofenac sodium to HPβCD is about 1:1.5 to about 1:5. In certain embodiments, the ratio of diclofenac sodium to HPβCD is about 1:1.5 to 1:2.5.

The pharmacokinetics of diclofenac sodium solubilized with HPβCD have been evaluated. The maximum HPβCD plasma concentration after administration of Dyloject™, which is an IV solution containing diclofenac sodium and HPβCD, was determined to be 70 μg/ml in normal healthy human subjects and 106 μg/ml in subjects with moderate renal impairment. The calculated diclofenac/cyclodextrin complex stability constant was 116 $M^{-1}$, the calculated drug fraction bound to protein was 99.5%, and the calculated drug fraction bound to cyclodextrin in plasma was 0.00%. Based on these values, HPβCD has a negligible effect on the fraction of diclofenac sodium plasma protein bound or on the fraction of the drug unbound in plasma.

Pharmaceutical compositions include solid dosage forms, *e.g.,* for perioral, transnasal (powder), or rectal (suppository) administration; and liquid dosage forms, *e.g.,* for parenteral administration, transnasal (spray), or perioral administration. In a specific embodiment, the pharmaceutical compositions of the presently disclosed subject matter are formulated for parenteral administration, including intravenous and intramuscular administration.

The pharmaceutical compositions of diclofenac or a pharmaceutically acceptable diclofenac salt and HPβCD suitable for parenteral administration can be in the form of suspensions, solutions, or emulsions, in oily or aqueous vehicles, and can contain formulatory agents such as suspending, stabilizing, solubilizing, and/or dispersing agents. The form can be sterile and can be fluid. It can be stable under the conditions of manufacture and storage and can be preserved against the contaminating action of microorganisms such as bacteria and fungi. Alternatively, the diclofenac or a pharmaceutically acceptable diclofenac salt and HPβCD can be in sterile powder form for reconstitution with a suitable vehicle before use. The pharmaceutical compositions can be presented in unit dose form, in ampoules, or other unit-dose containers, or in multi-dose containers. Alternatively, the pharmaceutical compositions can be stored in a freeze-dried (lyophilized) condition requiring only the addition of sterile liquid carrier, for example water for injections, immediately prior to use. Extemporaneous injection solutions and suspensions can be prepared from sterile powders, granules or tablets.

Excipients that is used include preservatives, suspending agents, stabilizers, dyes, buffers, antibacterial agents, antifungal agents, and isotonic agents, for example, sugars or sodium chloride. As used herein, the term "stabilizer" refers to a compound optionally used in the pharmaceutical compositions of the presently disclosed subject matter in order to avoid the need for sulphite salts and increase storage life. Examples of stabilizers include antioxidants, preferably monothioglycerol and those described in U.S. Patent Publication 2005/0238674.

The pharmaceutical composition can comprise one or more pharmaceutically acceptable carriers. The carrier can be a solvent or dispersion medium. Examples of pharmaceutically acceptable carriers include water, ethanol, polyol (*e.g.,* glycerol, propylene glycol and liquid polyethylene glycol), oils, and suitable mixtures thereof.

The parenteral formulation can be sterilized. Examples of sterilization techniques include filtration through a bacterial-retaining filter, terminal sterilization, incorporation of sterilizing agents, irradiation, heating, vacuum drying, and freeze drying.

**Routes of Administration**

The diclofenac-HPβCD combination for use in the presently disclosed subject matter can be administered via any suitable parenteral route. Examples of parenteral routes of administration include intravenous, such as a bolus injection or infusion, as well as intramuscular, subcutaneous, intraperitoneal or intrathecal. Examples of parenteral delivery systems include intravenous (IV) bags, syringes, bioerodable implants, osmotic pumps, implantable infusion systems, pump delivery, injection pens, and needle-less injection devices.

**Methods of Treatment**

As noted above, the compositions disclosed are for use in administering diclofenac to treat post-operative pain. Examples of post-operative pain include pain resulting from or following orthopedic, abdominal, pelvic, dental, plastic, cosmetic, neurological, urological, bariatric, gastric, cardiac, orthoscopic, vascular, endovascular, laparoscopic, oncological, colorectal, podiatric, ocular, otoplastic, rhinoplastic, throat surgery, or any other surgery that would qualify for ≥ two days of scheduled parentarlly-administered NSAIDs over multiple days. In particular embodiments, the post-operative pain is pain resulting from or following orthopedic surgery, abdominal surgery, or pelvic surgery. Examples of orthopedic surgery include total hip, total knee, bilateral total knee, spine, shoulder, ankle, soft tissue surgeries, spinal fusion, rotator cuff repair, laminectomy, fracture repair, and discectomyExamples of abdominal/pelvic surgery include abdominal hysterectomy, abdominal laparotomy, cholecystectomy, vaginal hysterectomy, ventral or inguinal hernia repair, myomectomy, salpingo-oophorectomy, bariatric, partial colectomy surgeries, and gynecologic or genitourinary surgery.

The compositions disclosed are suitable in the treatment of all severities of pain, including moderate to severe pain. Moderate or severe pain is defined as a pain intensity of ≥ about 50 mm on a 0-100 mm visual analog scale (VAS). In one embodiment, moderate pain is characterized as a pain intensity of ≥ about 50 mm and < about 70 mm on a 1-100 mm VAS. Severe pain is characterized as a pain intensity of ≥ about 70 mm on a 1-100 mm VAS. An advantage of the presently disclosed subject matter results from the surprising discovery that diclofenac can be used for severe pain, since it was previously believed that NSAIDs could only be used to treat mild to moderate pain. The pharmaceutical composition comprises 50 mg or less of diclofenac compound. In particular embodiments, the pharmaceutical composition comprises 45 mg or less of diclofenac compound. In certain embodiments, the pharmaceutical composition comprises 40 mg or less of diclofenac compound. In particular embodiments, the pharmaceutical composition comprises 37.5 mg or less of diclofenac compound. In certain embodiments, the pharmaceutical composition comprises 30 mg or less of diclofenac compound. In particular embodiments, the pharmaceutical composition comprises 25 mg or less of diclofenac compound. In one embodiment, the pharmaceutical composition comprises 20 mg or less of diclofenac compound. In certain embodiments, the pharmaceutical composition comprises 18.75 mg or less of diclofenac compound. In one embodiment, the pharmaceutical composition comprises 15 mg or less of diclofenac compound. In certain embodiments, the pharmaceutical composition comprises 10 mg or less of diclofenac compound. In particular embodiments, the pharmaceutical composition comprises 9.375 mg or less of diclofenac compound. In one embodiment, the pharmaceutical composition comprises 5 mg or less of diclofenac compound. The 18.75 mg and 50 mg diclofenac doses are 25% and 67%, respectively, of the minimum approved dose and about 12.5% or 33%, respectively, of the approved daily dosage.

In certain embodiments, the presently disclosed subject matter provides for titrating the dose reduction of diclofenac and beta-cyclodextrin by decreasing the unit dose to achieve an analgesic effect that is sufficient, even at a reduced level, for the patient's needs, which can be met by increasing the dosing frequency to achieve an effective daily dosage that is still lower than the minimum approved dose. The term "effect" means that there is a statistically significant difference in a response in patients taking the formulation containing the diclofenac relative to patients taking a placebo.

The pharmaceutical compositions of the presently disclosed subject matter are suitable for mammals. Preferably, the mammal is a human. In certain embodiments, the mammal is a human subject who is a member of a specialized patient population. In particular embodiments, the human subject is a high-risk subject or obese.

In one embodiment, the human subject is a high-risk subject. In particular embodiments, the high-risk subject is undergoing medical ulcer therapy, weighs less than 110 lbs. (50 kg), has a Child-Pugh score of from about 6 to about 9, has a serum creatinine of from about 1.9 to about 3.0 mg/dL, has a history of gastrointestinal bleeding or perforation, has renal impairment, has hepatic impairment, or has concomitant anticoagulant use. In some embodiments, the subject receives concomitant anticoagulant for deep vein thrombosis (DVT) prophylaxis, for example, heparin, low molecular weight heparin (LMWH), aspirin, or clopidogrel.

In certain embodiments, the high-risk subject is elderly. In particular embodiments, the high-risk subject is at least 65 years old. In particular embodiments, the high-risk subject is at least 65 years old and weighs at least about

210 lbs. (95 kg). Elderly patients are likely to have renal or hepatic deficiencies, which leads to higher plasma levels of administered drugs. Elderly patients may also have weakened immune systems. There is also a risk of drug-drug interactions since these patients are often on multiple medications.

In certain embodiments, the high-risk human subject has cancer. In certain embodiments, the human subject is immunocompromised. Human subjects with cancer may have weakened immune systems, renal deficiencies, or hepatic deficiencies due to the disease or cancer treatments such as chemotherapy, radiation, and surgery. As with other high-risk patients, human subjects with cancer may also be at risk of drug-drug interactions since these subjects are often on multiple medications.

High-risk subjects are at greater risk of opioid and NSAID induced side effects, so lower dosages of these drugs are therefore are preferable in order to minimize their exposure and risk. A significant advantage of the presently disclosed subject matter results from the ability to achieve efficacy with lower doses and overall daily dosing of diclofenac. Another advantage is the ability to reduce the amount and frequency of rescue medication. Consequently, it is possible to reduce the dosages, and thus reduce toxicities of these medications.

In particular embodiments, the human subject is obese. Obesity is a medical condition in which a subject has excess body fat. Obesity is associated with a number of medical ailments, including atherosclerosis, hypertension, arrhythmia, type II diabetes, pancreatitis, hypercholesterolemia and hyperlipidemia, insulin resistance, osteoarthritis, respiratory complications, and a higher risk of cancer. One measure of obesity is the body mass index (BMI), which compares weight and height. A BMI of over 30 is generally considered obese. In certain embodiments of the presently disclosed subject matter, the human subject weighs at least about 210 lbs. (95 kg). A human subject who weighs 210 lbs. has a BMI of at least about 30 if he or she is 5'10" or shorter. Obese patients sometimes require higher doses of analgesics. Falagas et al. The Lancet 375(9710): 248-251 (2010). An advantage of the presently disclosed subject matter results from the ability to achieve efficacy with lower doses and overall daily dosing of diclofenac even in obese patients. The presently disclosed subject matter also provides for a reduction in the amount and frequency of rescue medication. The lower dosages reduce the potential of NSAID and opioid toxicities in obese patients.

As noted above, the mammal can also be a non-human animal. Thus, the presently disclosed subject matter is useful in veterinary medicine as well, *e.g.,* for treating pain in a domestic pet, such as a canine or feline, a farm animal, a work animal, or an animal in a circus or zoological garden. The presently disclosed subject matter has particular value in treating pain in a horse, particularly in sport, such as thoroughbred and other race horses, rodeo horses, circus horses, and dressage horses. A particular advantage of the presently disclosed subject matter is that, by increasing the efficacy of a dosage of diclofenac, it is possible to administer a therapeutic dosage that is below a maximum allowed dose permitted by the particular regulatory authorities of the sport.

Pain relief to the subject may be provided within about one hour after administration. Pain relief may be provided within about 45 minutes after administration, or30 minutes after administration, or within about 15 minutes after administration, or within about 10 minutes after administration, or within about 5 minutes after administration.

## EXAMPLES

### EXAMPLE 1: IV Diclofenac for Treatment of Acute Moderate to Severe Pain after Orthopedic Surgery

A 277-patient, multicenter, multiple-dose, multiple-day, randomized, double-blind, active- and placebo-controlled, parallel-group study was conducted on patients who had undergone orthopedic surgery. Patients were randomly assigned to diclofenac, ketorolac, or placebo (2:1:1 ratio). The patients selected for the study had moderate to severe pain within 6 hours postoperatively, defined as pain intensity of at least 50 mm on a 0-100 visual analog scale (VAS).

Randomization was stratified by risk group at baseline. The groups were high-risk, non-high-risk, or higher-weight, which was greater than 210 lbs., or 95 kg. Randomization was also stratified by anticipated stay between a long stay, which was longer than 24 hours, versus a shorter stay. The high-risk patients weighed less than 110 lbs. (50 kg), were 65 years or older, were undergoing medical ulcer therapy, or had a Child-Pugh score 6-9, a serum creatinine of 1.9-3.0 mg/dL, or a history of gastrointestinal bleeding or perforation.

The diclofenac dose was 37.5 mg in the non-high-risk group, 18.75 mg in the high-risk group, and 50 mg in the higher-weight group. The diclofenac formulation was Dyloject™. The ketorolac dose was 30 mg in the non-high risk and higher-weight groups and 15 mg in the high-risk group. Patients in all treatment groups received a bolus IV injection every 6 hours until discharge, or discontinued sooner if the patient withdrew from the active treatment phase of the study owing to an adverse event, inadequate pain control, noncompliance with the protocol, or at the investigator's discretion (*e.g.*, to address intercurrent illness or because parenteral NSAID treatment was no longer required). Patients were observed for at least 24 hours from baseline (study drug initiation) and for up to 5 days. Adverse events (adverse effects) were recorded between surgery and randomization, not just after baseline, in order to distinguish treatment-emergent adverse events.

Rescue medication was IV morphine, given every three hours as needed in 2.5-mg increments up to a total of

7.5 mg. Patients were encouraged to wait at least 30 minutes after study drug initiation to request morphine.

Patients assessed their pain at baseline and at specified time points through the next 24 hours. Those who remained at the site longer assessed their pain every three hours until discharged. Patients returned for follow-up 5-9 days after baseline, and they received a follow-up telephone call 30-37 days after baseline.

A sample of 120 patients on diclofenac and 60 patients on placebo were needed to provide 95% power to detect a clinically significant difference for each time interval in the primary efficacy measure. This calculation was based on an estimated standard deviation of 468 for the 0 to 24 hour interval obtained from a randomized, placebo-controlled pivotal trial the sponsor conducted in an orthopedic surgical population. Standard deviations for the other intervals were obtained by extrapolation.

Efficacy analyses were conducted using Statistical Analysis Software®, and unless otherwise noted refer to the intent-to-treat population. The analysis of area under the pain intensity difference curve, pain intensity difference, area under the pain relief curve, patient global evaluation, and amount of rescue medication were based on analysis of covariance (ANCOVA) models, with treatment and center as factors and baseline pain as a covariate. Confidence intervals were based on the pooled standard deviation obtained from an ANCOVA model. Treatment differences were tested with linear contrasts.

All testing of statistical significance was 2-sided unless the test performed was inherently 1-sided. Interaction P-values <0.1 were considered significant; otherwise, P-values <0.05 were considered significant. For area under the pain intensity difference curve, the five comparisons between diclofenac and placebo were performed in the following order: 0 to 24, 0 to 48, 0 to 72, 0 to 96, and 0 to 120 hours. If any of the comparisons failed to demonstrate statistical significance, no further comparisons were made. The area under the pain relief curve was analyzed similarly.

The proportion of patients attaining at least 30% reduction in pain intensity and the frequency of rescue medication use were analyzed with Cochran-Mantel-Haenszel tests, with center as a stratification variable.

For area under the curve calculations, evaluations after the administration of rescue medication or after withdrawal due to adverse events (adverse effects) or lack of efficacy were imputed in accordance with prespecified rules. For post hoc analyses of pain intensity, an even more conservative and simplified set of imputation rules was applied.

There were no significant differences across treatment groups for any baseline characteristic, either overall or within any risk group. The distribution of surgeries differed by length of stay and by risk group. Bunionectomy or other foot bone surgery was the most common in the short-stay population (39%) and the non-high-risk group (50%). Knee replacement was most common in the long-stay population (49%) and the high-risk group (57%). In the higher-weight group, the most common procedures were bunionectomy (25%), knee replacement (22%), and knee surgery other than replacement (18%). There were no significant differences among treatment groups in duration of surgery, duration of anesthesia, or time from end of surgery until study dose initiation.

Overall, 78% of patients reported at least one adverse event, with similar incidence in the active treatment groups. The incidence of adverse effects was also similar across the risk groups compared with the overall population. Most adverse effects (92%) were mild to moderate in severity. Nausea was the most commonly reported adverse effect overall and in each risk group. Within the higher-weight group, use of diclofenac 50 mg did not increase the risk of an adverse effect compared with placebo. No deaths were reported.

The incidence of bleeding-related adverse effects was similar in the active treatment groups (diclofenac, 21%; ketorolac, 23%) and not significantly greater than in the placebo group (17%). Likewise, in the subset of patients who received anticoagulants (n=197), there were no clinically meaningful differences in bleeding-related adverse effects across treatment groups. There was one report of marked elevation (greater than eight times the upper limit of normal) of alanine aminotransferase in the diclofenac group.

The overall mean pain intensity on VAS was 69 mm at baseline; 57% of patients had moderate pain (50 mm $\leq$ VAS < 70 mm) and 43% had severe pain (VAS $\geq$ 70 mm). There were no significant differences in baseline pain intensity across treatment groups or risk groups.

Eleven patients were assigned to the wrong risk group at baseline. In four cases, site personnel discovered the error and adjusted the dose of study drug. The post hoc analyses presented here are based on dose levels received.

The sum of pain intensity differences is shown in Figure 1. Pain intensity was assessed on a 100-mm visual analog scale at baseline and at scheduled time points over at least 24 hours and for up to 120 hours (five days). Mean SPID scores are shown for each of the five time intervals in the primary efficacy measure for placebo (dark grey bars), ketorolac (light grey bars), and diclofenac (black bars). Larger values indicate greater reduction of pain intensity from postoperative baseline values. The percentages of imputed SPID values over 0 to 48, 0 to 72, 0 to 96, and 0 to 120 hours were 47%, 54%, 62%, and 68%, respectively. *P < 0.0001 vs. placebo. In all time intervals, the mean sum of pain intensity differences (SPID) was significantly better for diclofenac and ketorolac than for placebo (P < 0.0001). Those results were consistent across baseline pain intensities. Starting at 0 to 48 hours, diclofenac was consistently numerically superior to ketorolac.

The total pain relief over 0 to 24, 0 to 48, 0 to 72, 0 to 96, and 0 to 120 hours was significantly better with diclofenac and ketorolac than with placebo (P < 0.0001). During each interval, diclofenac was numerically superior to

ketorolac. Clinically meaningful pain reduction was achieved by 81% and 75% of patients on diclofenac and ketorolac, respectively, compared with 43% on placebo. The proportion of patients who had meaningful pain reduction with diclofenac was significantly superior to the proportion in the ketorolac group at 10 minutes, 42 hours, 48 hours, and 60 hours ($P \leq 0.05$ for all comparisons).

The onset of analgesia was faster with diclofenac than with the comparators. For the pain intensity difference, which is the baseline pain intensity minus pain intensity at each scheduled assessment, there was a significant difference compared with placebo. This difference first occurred at 10 minutes with diclofenac (P = 0.03) and at 30 minutes with ketorolac (P = 0.006). For both active treatments, statistical separation from placebo was maintained for 120 hours.

Figure 2 shows the cumulative amount or rescue medication (mg) required over the first five days. Rescue medication (IV morphine) was given every three hours as needed in 2.5-mg increments up to a total of 7.5 mg. The total morphine requirement over the first five days was 42% lower with diclofenac than with placebo (11.8 vs. 20.5 mg), and in every time interval the opioid-sparing effect was at least 40% with diclofenac compared with placebo. Diclofenac-treated patients consistently required significantly less morphine than those receiving placebo ($P < 0.0001$). Over the five days of treatment, patients on diclofenac also used significantly less morphine compared with patients on ketorolac (11.8 v.s. 18.1 mg, 35% reduction v.s. ketorolac, P = 0.008).

The cumulative proportions of patients in each treatment group who required rescue medication are shown in Figure 3. The median time from administration of study drug to administration of rescue medication was greatest for diclofenac, which had a median time of 220.0 minutes. The median time from administration of study drug to administration of rescue medication for ketorolac was 137.0 minutes. The median time from administration of study drug to administration of rescue medication for placebo was 51.0 minutes. The time to rescue was significantly greater for both diclofenac and ketorolac groups compared to placebo ($P < 0.0001$ using log-rank test comparing treatment groups to placebo). Of the patients who required rescue, more than half required rescue two or fewer times in the diclofenac and ketorolac groups compared with six or fewer times in the placebo group (data not shown).

Patient global evaluations were done every 24 hours post start time of initial dosing and at completion/early termination. Subjects were asked how they would rate the study drug on a 5-point scale ranging from "poor" to "excellent." The results are shown in Figure 4. At last assessment (completion or early termination), 70.2% of patients on diclofenac and 57.6% on ketorolac rated their drug as "very good" or "excellent", versus 22.9% of those on placebo. Overall, the mean scores for the active treatments were significantly higher than for placebo ($P < 0.0001$). In the non-high-risk group, mean SPID scores for the 0 to 6, 0 to 24, 0 to 48, 0 to 72, 0 to 96, and 0 to 120 hour intervals were better with diclofenac 37.5 mg than with placebo ($P \leq 0.001$), with no significant differences between active treatments. Similarly, in the high-risk group, SPID scores for all time intervals were better with diclofenac 18.75 mg than with placebo ($P < 0.05$). Compared with patients receiving ketorolac 15 mg, those who received diclofenac 18.75 mg had better SPID scores over 0 to 24 hours (P = 0.02) and 0 to 48 hours (P = 0.02).

Most patients received the study drug for three days or less. Of these, 55% received the study drug for one day, 6% for two days, and 30% for three days. Essentially all patients expected to have a short stay did stay only one day, and nearly all received four doses of study drug. Of the 155 long-stay patients, 54% had 9 to 12 doses, 30% had 1 to 8, and 16% had 13 or more doses.

In the higher-weight group, SPID scores for all time intervals were better with both diclofenac 50 mg and ketorolac 30 mg compared with placebo ($P < 0.05$), with no significance differences between active treatments.

Elderly patients who received diclofenac had a 1.3-fold greater response rate compared with ketorolac and 2.4- to 3.5-fold greater response rates compared with placebo, as defined by 30% reduction in pain intensity. Analgesic effectiveness over 0 to 24 and 0 to 48 hours, measured by SPID scores and 30% reduction in pain intensity, was better with diclofenac than ketorolac ($P < 0.05$), and diclofenac-treated patients needed less rescue medication than those given ketorolac or placebo (P = 0.05). The amount of morphine used was 35.5% less in diclofenac treated elderly patients versus ketorolac treated elderly patients (9.6 vs. 14.9 mg), and the frequency of morphine use was also less in the diclofenac group (data not shown).

In the non-high-risk group, mean SPID scores for the 0 to 6, 0 to 24, 0 to 48, 0 to 72, 0 to 96, and 0 to 120 hour intervals were better with diclofenac 37.5 mg than with placebo ($P \leq 0.001$), with no significant differences between active treatments. Similarly, in the high-risk group, SPID scores for all time intervals were better with diclofenac 18.75 mg than with placebo ($P < 0.05$). Compared with patients receiving ketorolac 15 mg, those who received diclofenac 18.75 mg had better SPID scores over 0 to 24 hours (P = 0.02) and 0 to 48 hours (P = 0.02).

This study establishes the safety and efficacy of diclofenac for managing acute moderate to severe pain alone or in combination with an opioid in patients recovering from painful orthopedic surgery. Administration of diclofenac 37.5 mg every 6 hours was found to provide significantly superior analgesia than placebo and numerically greater analgesic effects than those receiving ketorolac beginning at 0 to 48 hours, although this did not reach the level of statistical significance. During the trial, all treatment groups received rescue medication as needed and experienced satisfactory analgesia. Importantly, diclofenac treated patients required 35% less rescue morphine than those treated with ketorolac, a difference that was clinically and statistically significant at p=0.008. The median time to rescue morphine administration

with IV diclofenac was 220 minutes, four times longer than those receiving placebo and 83 minutes longer than those receiving ketorolac.

Elderly patients treated with a half dose of diclofenac (18.75 mg) had superior outcomes compared with those who received a half dose of ketorolac (15 mg), including a higher likelihood of analgesic response, significantly better analgesic efficacy, and a lower opioid requirement. This finding is important since elderly people are at greater risk of opioid and NSAID induced side effects and lower dosages are warranted of each in order to minimize their exposure and risk.

In the perioperative period, side effects of most concern with nonselective NSAIDs are renal impairment, interference with platelet function, wound and bone healing, and peptic ulceration or bronchospasm in individuals at risk. The patient on diclofenac who developed renal failure in the present study had multiple risk factors for acute renal failure after general surgery: over 59 years old, male, obesity, active congestive heart failure, hypertension, mild preoperative chronic renal insufficiency, and perioperative vasopressor and diuretic use. The acute worsening of his renal insufficiency promptly began to revert toward baseline upon correction of physiological disturbances, and he did not require dialysis. An open-label study has demonstrated the safety of postoperative HPβCD diclofenac in hundreds of patients with known NSAID risk factors, including advanced age, renal or hepatic impairment, or routine anticoagulation. See Chelly J et al. Presentation at the International Association for the Study of Pain 13th World Congress on Pain (2010).

Blood loss in this study was slightly greater with the active treatments than with placebo. However, hip replacement surgery, which often results in substantial blood loss, was more frequent in the diclofenac group (13%) than in the ketorolac (10%) or placebo groups (10%). Thus, overrepresentation of this procedure in the diclofenac group would be expected to exaggerate any tendencies toward blood loss.

In a single-dose crossover trial in healthy volunteers, HPβCD diclofenac 37.5 mg resulted in significantly less platelet function disruption compared with analgesic doses of oral diclofenac, IV ketorolac, and acetylsalicylic acid. See Bauer KA et al. J Clin Anesth. 2010; 22(7):510-518. Both sets of findings are important for hip and knee arthroplasty and hip fracture surgery, which are associated with a high risk of thromboembolism. Although insufficient as the sole means of thromboprophylaxis, neuraxial analgesia is recognized for its ability to attenuate the hypercoagulable response. The American Society of Regional Anesthesia and Pain Medicine has concluded that NSAIDs seem to represent no added significant risk for the development of spinal hematoma in patients having epidural or spinal anesthesia and should not obviate the performance of neuraxial blocks.

The decreased use of rescue opioid medication observed in this study is fundamental to the concept of multimodal analgesia with an NSAID. Not every NSAID molecule has the same mechanisms of action, and it is conceivable that unique actions of diclofenac enhance its efficacy after orthopedic surgery. Without being bound to any theory, the mechanisms are believed to include stimulation of adenosine triphosphate-sensitive potassium channels and central effects such as increasing plasma β-endorphin levels and inhibiting the N-methyl-D-aspartate pathway.

Even after fast-track hip and knee arthroplasty, most patients have moderate to severe pain for the first 48 hours. The vast majority of the literature on NSAID use for multimodal acute postoperative pain control describes NSAIDs as generally useful for mild to moderate, but not severe pain. In this study, diclofenac demonstrated the unexpected result that it was effective in treating severe pain as well as moderate pain. This extends the clinical applicability of NSAIDs to a pain severity not previously thought to be routinely controllable with an NSAID plus minimal amounts of rescue opioid medication.

Diclofenac demonstrated the unexpected result that it was effective in treating severe pain as well as moderate pain. This study further demonstrates that a novel IV formulation of diclofenac, a long-trusted NSAID with a well-characterized safety profile, is safe and efficacious for treatment of acute moderate to severe pain after major orthopedic surgery. Furthermore, the data demonstrates that HPβCD diclofenac can be used as the default postoperative analgesic, with morphine added as necessary, rather than the reverse.

## EXAMPLE 2: IV Diclofenac for Treatment of Acute Moderate to Severe Pain after Abdominal or Pelvic Surgery.

A 331 multicenter, multiple-dose, multiple-day, randomized, double-blind, parallel-group study was conducted on patients who had undergone abdominal or pelvic surgery. Patients were randomly assigned to 18.75 mg of diclofenac, 37.5 mg of diclofenac, 30 mg ketorolac, or placebo (1:1:1:1 ratio). The diclofenac formulation was Dyloject™. The patients selected for the study had moderate to severe pain within 6 hours postoperatively, defined as pain intensity of at least 50 mm on a 0-100 VAS.

Over the first 48 hours after study drug initiation, the mean sum of pain intensity differences was significantly better for each of the active treatments compared with those receiving placebo and rescue morphine, as shown in Table 1. The results were consistent regardless of baseline pain intensity. There were no significant differences between the low dose 18.75 mg HPβCD diclofenac group and those that received the standard ketorolac dose of 30 mg or 37.5 mg of HPβCD diclofenac, although the diclofenac 37.5 mg group had a numerically greater SPID than the diclofenac 18.75 mg group.

**Table 1: Sum of the Pain Intensity Differences Over 0-48 Hours**

| | Placebo (N=76) | Ketorolac 30 mg (N=82) | Diclofenac 18.75 mg (N=86) | Diclofenac 37.5 mg (N=87) |
|---|---|---|---|---|
| Mean (SD) | 936.0 (1076.56) | 1583.2 (982.74) | 1303.6 (1029.50) | 1573.5 (1060.34) |
| 95% Confidence Interval (CI) for difference from diclofenac 18.75 mg | --- | -7.6, 590.4 | --- | -30.3, 562.4 |
| 95% CI for difference from diclofenac 37.5 mg | --- | -274.1, 324.8 | -30.3, 562.4 | --- |
| *P*-value vs. placebo | --- | <.0001 | .03 | .0001 |

The sum of pain intensity differences over the 0 to 24 hour interval was significantly better with HPβCD diclofenac 37.5 mg and ketorolac than with placebo (P < 0.0001). Only a small number of patients, 15 of 331, needed to stay longer than 48 hours, so data were insufficient to evaluate longer time intervals. The mean pain intensity difference (baseline pain intensity minus pain intensity at each scheduled assessment) was consistently better with the active treatments than with placebo over the first 48 hours.

The proportions of patients with clinically meaningful pain reduction were greater with active treatments than with placebo. At 45 minutes after study drug initiation, 34% of patients receiving placebo and rescue morphine had at least a 30% reduction in pain intensity, compared with 57% of patients on ketorolac, 42% of patients on 18.75 mg, and 46% of patients on 37.5 mg HPβCD diclofenac (P = 0.02 for the active treatments versus placebo).

The mean total pain relief scores over the 0 to 24 and 0 to 48 hour time intervals were significantly better for the active treatments than for those receiving placebo (P< 0.0008). During the first 24 hour time interval the mean score was significantly better for each active treatment than for placebo (P < 0.05), with no significant differences among active treatments.

The time to administration of rescue medication was longer with each of the active treatments than with placebo. The median time to rescue morphine administration was 2 hours 7 minutes for placebo, 4 hours 15 minutes for ketorolac, 3 hours 14 minutes for low dose 18.75 mg HPβCD diclofenac, and 2 hours 24 minutes for the standard 37.5 mg dose of HPβCD diclofenac.

Over all time intervals studied, patients on active treatments required significantly less morphine and significantly less frequent administrations of morphine compared with the placebo control group, as shown in Table 2 and Figure 5. Over the first 24 hours, patients receiving the low dose 18.75 mg HPβCD diclofenac or the standard 30 mg dose of ketorolac experienced 39% and 40% reductions in morphine dosages, respectively, as compared to those treated with placebo and rescue morphine. Patients receiving the 37.5 mg dose of HPβCD diclofenac experienced a 44% reduction over the same 24 hour time period. Overall, patients on active treatments required approximately half as much morphine as those receiving placebo.

**Table 2: Cumulative Amount of Rescue Medication, mg**

| Time interval, hours | Placebo (N=76) | Ketorolac 30 mg (N=82) | Diclofenac 18.75 mg (N=86) | Diclofenac 37.5 mg (N=87) |
|---|---|---|---|---|
| 0 to 24 | | | | |
| Mean (SD) | 11.2 (7.61) | 6.7 (7.74) | 6.8 (6.8 0) | 6.3 (7.33) |
| Opioid sparing vs. placebo, % | --- | 40 | 39 | 44 |
| P-value vs. placebo | --- | <0.0001 | 0.0001 | <0.0001 |
| 0 to 48 | | | | |
| Mean (SD) | 15.6 (12.61) | 8.5 (10.00) | 8.4 (9.95) | 7.3 (9.29) |
| Opioid sparing vs. placebo, % | --- | 46 | 46 | 53 |
| P-value vs. placebo | --- | <0.0001 | <0.0001 | <0.0001 |

(continued)

| Time interval, hours | Placebo (N=76) | Ketorolac 30 mg (N=82) | Diclofenac 18.75 mg (N=86) | Diclofenac 37.5 mg (N=87) |
|---|---|---|---|---|
| 0 to 72 | | | | |
| Mean (SD) | 15.9 (13.28) | 8.5 (10.04) | 8.8(10.44) | 7.4 (9.59) |
| Opioid sparing vs. placebo, % | --- | 47 | 45 | 53 |
| P-value vs. placebo | --- | <0.0001 | <0.0001 | <0.0001 |

The patient global evaluations in each of the active treatment groups were significantly superior to placebo (P < 0.001) for both the 0 to 24 hour and 0 to 48 hour intervals, with no significant differences between those receiving low dose 18.75 mg HPβCD diclofenac and those receiving the standard 30 mg dosage of ketorolac or 37.5 mg dosages of HPβCD diclofenac. Altogether, 83% to 87% of patients in the active treatment groups assessed their study drug as "good," "very good," or "excellent" at 48 hours.

Overall, 85% of patients experienced at least one adverse event, with similar rates among the active treatment groups. Most events were mild to moderate in severity. Nausea, flatulence, and injection site pain or irritation were the most commonly reported adverse events among patients receiving active treatments. There was no indication of increased risk of cardiovascular thrombosis.

This study demonstrate the safety and efficacy of HPβCD diclofenac 18.75 and 37.5 mg for treatment of acute moderate to severe pain following major abdominal or pelvic surgery. It also demonstrated that the delivery every 6 hours of lower dosages of 37.5 mg or 18.75 mg diclofenac solubilized in HPβCD was able to provide analgesia for moderate to severe pain comparable to a standard dose of 30 mg ketorolac, the most commonly used injectable non-narcotic analgesic in North America. The HPβCD diclofenac 37.5 mg and 18.75 mg doses were significantly more effective than placebo as measured by the sum of pain intensity differences, total pain relief, proportion of patients with at least a 30% reduction in pain intensity, proportion of patients requiring rescue morphine, and average amount of rescue morphine and comparable to that demonstrated for with a standard 30 mg dose of ketorolac.

The incidence and severity of treatment-emergent and treatment-related adverse events demonstrated in this clinical trial were comparable to the active treatment control. No treatment-related serious adverse events were reported in either diclofenac dose group. HPβCD diclofenac was also not associated with an increased incidence of postoperative bleeding-related adverse events, even when given concomitantly with anticoagulants.

Pain and the need for analgesia are typically greatest during the first day after surgery and decline quickly thereafter. Confirming clinical experience, the use of rescue medication in this study was greatest in the first 24 hours postoperatively. Furthermore, the opioid-sparing effect of the active treatments, compared with placebo, was at least 40% during every time interval studied. These results are important because, according to a meta-analysis, morphine reduction of this magnitude is associated with a significant decrease in the incidence of post operative vomiting and sedation.

As in the previous Examples, diclofenac demonstrated the unexpected result that it was effective in treating severe pain as well as moderate pain. This study further supports the clinical applicability of NSAIDs to a pain severity not previously thought to be routinely controllable with an NSAID plus minimal amounts of rescue opioid medication.

This study demonstrates that diclofenac, a well established NSAID molecule with a well-characterized and known safety profile, maintains a high degree of intrinsic efficacy and safety for the treatment of acute moderate to severe pain when administered in lower dosages within a diclofenac sodium-HPβCD IV formulation following major abdominal or pelvic surgery. In addition, this study supports the ability of these lower dosage formulations to be used as a foundational analgesic for patients arriving in the postanesthesia care unit with moderate to severe pain.

**EXAMPLE 3: IV Diclofenac for Treatment of Post-operative Pain in a Broadly Representative Population.**

A 971-patient, open-label, single-arm prospective trial was conducted to evaluate the safety of delivering small-volume bolus injections of HPβCD diclofenac over the course of two to three days in patients with acute postoperative pain following major orthopedic surgery, abdominal/pelvic surgery, or other surgery. The major orthopedic surgeries were total hip, total knee, spine, shoulder, ankle, and soft tissue surgeries. The major abdominal/pelvic surgeries were hysterectomy, laparotomy, colectomy, salping-oophorectomy, inguinal hernia, and myomectomy surgeries. Of the 971 patients, 765 (78.8% of enrolled subjects) patients were concomitantly on anticoagulants such as heparin, low-molecular-weight heparin, warfarin, and aspirin. Over a third of the subjects were at least 65 years old (37.8% of enrolled subjects).

Approximately a third of the subjects, 335 patients, weighed at least 210 lbs., or 95 kg. The patient population reflected the types of patients commonly seen in post-operative settings.

Fifty seven subjects (5.9% of enrolled subjects) had renal impairment at baseline and thirty one subjects (3.2% of enrolled subjects) had hepatic impairment at baseline. For the laboratory analyses, the baseline values were the most recent value obtained postoperatively and prior to first dose of study drug. Any values obtained at screening were not used as baseline values. If screening values were missing, the values obtained at the baseline visit were used.

The diclofenac doses were 37.5 mg and 50 mg. All but one of the subjects who weighed less than 210 lbs., or 95 kg, was administered a dose of 37.5 mg diclofenac. The majority of the subjects who weighed at least 210 lbs., or 95 kg, were administered a dose of 50 mg. Fourteen subjects who weighed at least 210 lbs., or 95 kg, were administered a dose of 37.5 mg. In total, 634 patients received at least one does of 37.5 mg diclofenac and 335 patients received 50 mg diclofenac. The diclofenac formulation was Dyloject™.

Of the 971 subjects who were dosed, 943 (89.7% of enrolled subjects) completed the study. Among the 28 subjects who were withdrawn, the most common reasons were: lost to follow-up, withdrawal of consent, and noncompliance with study procedures. Two subjects who weighed over 210 lbs., or 95 kg, were treated with 18.75 mg in error and therefore do not appear in the summary data for either the 37.5 mg or 50 mg diclofenac doses. As a result, the sum of subjects from the two dose groups is 969, while the number of total subjects enrolled was 971.

The total exposure to HPβCD diclofenac was 2,359 subject days. Most subjects received study treatment for three days or less. Forty one patients (4.2%) were treated for one day, 607 patients (62.5%) were treated for two days, and 220 patients (22.7%) were treated for days. There were no notable differences in duration and frequency of administration between the 37.5 mg and 50 mg dosing groups.

All statistical analyses were performed using the SAS™ Statistical Software System (SAS Institute, Inc., Cary, NC). For continuous variables, data were summarized by sample size, mean, standard deviation, median, minimum, and maximum. For categorical or ordinal variables, data were summarized as frequency counts and percentages. Missing data were treated as a separate category in the categorical data summaries. No imputation methods were applied to missing data for safety or patient global evaluation.

Of the 958 subjects who completed the 5-point PGE, 97.3% indicated that their experience with study medication was "Excellent," "Very Good," or "Good" and 86.4% assessed their experience with study medication as "Very Good" or "Excellent."

For the elderly patient group, those who were at least 65 years old, the mean age was 72 years old. In this group, 268 of the 367 elderly subjects (73.0%) received the recommended dose of 37.5 mg diclofenac and 99 elderly subjects were at least 210 lbs. and received the adjusted dose of 50 mg diclofenac. The overall incidence of subjects reporting adverse events was similar for the two dosing groups. The overall incidence of subjects reporting adverse events was also similar for the two age groups of elderly and non-elderly patients. The slightly higher percentage of adverse effects in elderly subjects (86.4%) compared to subjects under 65 years of age (83.8%) is likely to be due not only to age but also to differences in comorbidities, postoperative complications, and intrusiveness of surgical procedure. For instance, elderly subjects had a small but higher incidence of renal failure acute (1.6% vs. 0.2%) and renal failure (0.8% vs. 0%) that was likely associated with fluid shifts that occur in more extensive surgical procedures.

A total of 765 subjects (78.8%) received anticoagulant therapy, including 602 subjects (62.0%) who received heparin, low-molecular-weight heparin, or warfarin on the last day of study drug administration. In the entire study population, 56 subjects reported one or more treatment-emergent bleeding-related adverse events. Most of these events (85.7%) occurred in subjects who received anticoagulant treatment. The incidence of treatment-emergent bleeding events was similar in the 37.5 mg diclofenac group and 50 mg diclofenac group for both subjects who received anticoagulant treatment and those in the overall population.

Twenty eight of the subjects who had had renal impairment at screening weighed at least 210 lbs. Of those, 23 received 50 mg diclofenac and 5 received 37.5 mg diclofenac. Overall, fewer subjects in the renal impairment group (79%) had at least one adverse effect than did the non-renally impaired subjects (85%).

Eight of the subjects who had hepatic impairment at screening weighed at least 210 lbs and received 50 mg diclofenac. Twenty nine of the subjects with hepatic impairment (93.5%) experienced at least one treatment-emergent adverse effect, compared with 84.5% of subjects in the non-hepatically impaired group.

The majority of the subjects (95.9%) who received 50 mg diclofenac weighed more than 95 kg, or 209 lbs. The incidence of pulmonary embolism was higher in the higher-weight cohort, 1.4% compared with 0.3% of the overall study population. The incidence of most other adverse effects was lower or similar for subjects receiving 50 mg diclofenac compared with the rest of the study population. The exceptions were pyrexia (8%), peripheral edema (6%), hypokalemia (5%), muscle spasms (4%), asthenia (3%), and hyperglycemia (2%). The reports of treatment-related adverse effects were similar in the overall safety population and the high-weight subgroup.

Overall, the majority of treatment-emergent adverse effects were mild or moderate in severity. No drug-related cardiovascular adverse effects were reported. Two drug-related peripheral vascular adverse effects were reported. Only one drug-related cardiac arrhythmia was reported (vasovagal syncope). No subject noted palpable swelling or redness

beyond the length of the cannula, and none developed overt infection. At the study discharge/early termination assessment, the vast majority of subjects (95.3%) had no reaction.

In general, wound healing was not affected by HPβCD diclofenac, with 90% to 95% of subjects having normal or better than expected outcomes. The extent of healing, extent and degree of inflammation, and extent of drainage were "normal" or "better than expected" at the time of study discharge/early termination in 95%, 95%, and 93% of subjects, respectively. In all cases, "slower" or "much slower than expected" results were observed in 3% or less of subjects. In 83% of subjects there was no wound separation, and in 96% of subjects there was no localized separation. In 95% of subjects there was no sign of infection at the wound site at the time of study discharge/early termination. One subject (0.1%) received systemic antibiotics for treatment of infection.

In general, no significant changes in laboratory outcomes were observed, with the exception that mean hematocrit, hemoglobin, red blood cell, and white blood cell values tended to decrease by at least 10% from baseline to study discharge/early termination. Vital signs, including ECG results, and physical examination results showed little change from baseline. In this study, the only serious gastrointestinal adverse effect thought to be related to NSAIDs was one case of upper gastrointestinal hemorrhage (0.1%). NSAID class labeling for chronic use cites gastrointestinal adverse effects in 1% of patients treated for three to six months and about 2%-4% of patients treated for one year. For subjects who were over 65 years old, current national quality indicators include a rate of postoperative hemorrhage or hematoma of 0.3%, which is higher than that seen in this study.

The degree to which drug-drug interactions, type of surgery, or other factors affect the incidence of bleeding-related adverse effects could not be assessed in this study due to the lack of placebo control. However, the incidence of bleeding-related adverse effects was similar to or lower than what would be expected in a population of subjects receiving postoperative prophylactic anticoagulation, irrespective of whether the study drug was administered.

The observed incidence of postoperative renal failure in this study also compares favorably with national surgical data sets. For example, the American College of Surgeons-National Surgical Quality Improvement Program reports an incidence of postoperative acute renal failure of 1.0% across all subjects, with a higher incidence in subgroups undergoing more extensive procedures or with risk factors such as increasing age.

Although the study population was at risk for fluid shifts, compromised hemodynamic balance, and possibly impaired renal function, subjects treated with HPβCD diclofenac had a low incidence of the development of transient renal insufficiency. HPβCD diclofenac was also well tolerated by subjects with pre-existing impairment of renal function.

The subjects treated in this study are likely to be representative of the target patient population for HPβCD diclofenac. Consistent with the results of two phase 3 multidose, multiday randomized controlled trials, this study demonstrates the safety of HPβCD diclofenac injection for the treatment of acute moderate to severe pain following major abdominal/pelvic and orthopedic surgery. At a dose of 37.5 mg, 50 mg for subjects weighing 95 kg or more, HPβCD diclofenac was safe and well tolerated by the overall population and by patients with known NSAID risk factors, including advanced age, renal or hepatic impairment, or use of an anticoagulant.

**EXAMPLE 4: Two Dose Levels of IV Diclofenac for Treatment of Post-Operative Pain in Elderly Patients.**

A randomized, double-blind, placebo-controlled multicenter study is conducted on the efficacy, safety, and opioid-sparing effects of two doses of diclofenac sodium in elderly patients with postoperative pain.

Patients are randomly assigned to placebo, 18.75 mg diclofenac sodium or 37.5 mg diclofenac sodium (1:1:1 ratio). The diclofenac formulation is Dyloject™. Baseline pain intensity is recorded and then an IV bolus administration of the assigned treatment is administered. The assigned dose is administered every 6 hours for three days for a total of 12 doses. The patients selected for the study are 65 years or older with moderate to severe pain following surgical operations. The surgical outcomes, functionality, and satisfaction of the patients are recorded twice-daily. A quality of life assessment is determined on post-surgical day three.

Rescue medication is an IV opioid and is allowed for patients with inadequate pain control as determined by the patient. Investigator determined rescue opioid is used according to current practice.

Pain intensity and pain relief scores are obtained hourly after the first dose and then twice-daily thereafter. The pain intensity is measured by SPID and the pain relief is measured by TOTPAR. An elicited opioid-related adverse effect assessment is made twice-daily. Spontaneous adverse effects, changes in vital signs and changes in clinical laboratory findings are recorded. Bleeding indices are recorded prior to and upon completion of the trial. Thrombophlebitis and wound-healing are examined daily. Safety follow-up assessments are made with the patient between day 4 and 10 postoperatively and with the patients 30-37 days postoperatively.

**EXAMPLE 5: IV Diclofenac for Treatment of Post-Operative Bariatric Surgery Pain in Obese Patients.**

A randomized, double-blind, placebo-controlled multicenter study is conducted on the efficacy, safety, and opioid-sparing effects of two doses of diclofenac sodium obese patients with postoperative pain resulting from bariatric

surgery.

Patients are randomly assigned to placebo, 50 mg diclofenac sodium or 37.5 mg diclofenac sodium (1:1:1 ratio). The diclofenac formulation is Dyloject™. Baseline pain intensity is recorded and then an IV bolus administration of the assigned treatment is administered. The assigned dose is administered every 6 hours for three days for a total of 12 doses. The patients selected for the study are 18 years or older, weigh at least 95 kg (210 lbs.), and have moderate to severe pain following bariatric surgery. Some of the patients are over 65 years old. The surgical outcomes, functionality, and satisfaction of the patients are recorded twice-daily. A quality of life assessment is determined on post-surgical day three.

Rescue medication is an IV opioid and is allowed for patients with inadequate pain control as determined by the patient. Investigator determined rescue opioid is used according to current practice.

Pain intensity and pain relief scores are obtained hourly after the first dose and then twice-daily thereafter. The pain intensity is measured by SPID and the pain relief is measured by TOTPAR. An elicited opioid-related adverse effect assessment is made twice-daily. Spontaneous adverse effects, changes in vital signs and changes in clinical laboratory findings are recorded. Bleeding indices are recorded prior to and upon completion of the trial. Thrombophlebitis and wound-healing are examined daily. Safety follow-up assessments are made with the patient between day 4 and 10 postoperatively and 30-37 days postoperatively.

**EXAMPLE 6: Three Dose Levels of IV Diclofenac for Treatment of Post-Operative Pain.**

A randomized, double-blind, placebo-controlled multicenter study is conducted on the safety and opioid-sparing effects of three doses of diclofenac sodium in patients with postoperative pain following major orthopedic surgery, abdominal surgery, or thoracic surgery.

Patients are randomly assigned to placebo, 9.375 mg diclofenac sodium, 18.75 mg diclofenac sodium or 37.5 mg diclofenac sodium (1:1:1:1 ratio). The diclofenac formulation is Dyloject™. Baseline pain intensity is recorded and then an IV bolus administration of the assigned treatment is administered. The assigned dose is administered every 6 hours for three days for a total of 12 doses. The patients selected for the study are 18 years or older with moderate to severe pain following orthopedic, thoracic or abdominal surgical operations.

Rescue medication is an IV opioid and is allowed for patients with inadequate pain control as determined by the patient. Investigator determined rescue opioid is used according to current practice.

Pain intensity and pain relief scores are obtained hourly after the first dose and then twice-daily thereafter. The pain intensity is measured by SPID and the pain relief is measured by TOTPAR. An elicited opioid-related adverse effect assessment is made twice-daily. Spontaneous adverse effects, changes in vital signs and changes in clinical laboratory findings are recorded. Bleeding indices are recorded prior to and upon completion of the trial. Thrombophlebitis and wound-healing are examined daily. Safety follow-up assessments are made with the patient between day 4 and 10 postoperatively and with the patients 30-37 days postoperatively.

**REFERENCE EXAMPLE 7: IV Diclofenac for Treatment of Bone Pain in Cancer Patients.**

A randomized, double-blind, multicenter study is conducted on the efficacy, safety, and opioid-sparing effects of diclofenac sodium in cancer patients with moderate to severe bone pain.

Patients are randomly assigned to 37.5 mg diclofenac sodium or 7.5 mg morphine. The diclofenac formulation is Dyloject™. Baseline pain intensity is recorded and then an IV bolus administration of the assigned treatment is administered. The assigned dose is administered every 6 hours for five days for a total of 20 doses. The patients selected for the study are 18 years or older with moderate to severe pain subsequent to metastatic bone involvement.

Pain intensity and pain relief scores are obtained hourly after the first dose and then twice-daily thereafter. The pain intensity is measured by SPID and the pain relief is measured by TOTPAR. An elicited opioid-related adverse effect assessment is made twice-daily. Spontaneous adverse effects, changes in vital signs and changes in clinical laboratory findings are recorded. Functionality and satisfaction will be also recorded twice-daily. A quality of life assessment is determined on post-surgical day five. Patients are examined for the quality (burning, throbbing, sharp, electric, etc) and severity (11-point numerical pain rating scale) of pain daily. Safety follow-up assessments are made with the patient.

Rescue medication is an IV opioid and is allowed for patients with inadequate pain control as determined by the patient. Investigator determined rescue opioid is used according to current practice.

Demonstrating the safety and efficacy of Dyloject™ in cancer patients is important because the potential for adverse effects of NSAIDs may be greater in patients with cancer and those who are immunocompromised. The opioid-sparing effect of Dyloject™, both by frequency and by duration, is another important factor since cancer and immunocompromised patients are also likely to be at greater risk for adverse reactions to opioids.

**REFERENCE EXAMPLE 8: IV Diclofenac for Extended Treatment of Pain in Patients Under Hospice or Home Care.**

A randomized, double-blind, multicenter study is conducted on the efficacy of low-dose diclofenac sodium in patients in hospice or home care settings over an extended period of time.

Patients are randomly assigned to 18.75 mg diclofenac sodium plus saline every 6 hours, 18.75 mg diclofenac sodium plus 5 mg morphine every 6 hours, or 18.75 mg diclofenac sodium plus 5 mg morphine every 12 hours. The diclofenac formulation is Dyloject™. Baseline pain intensity is recorded and then an IV bolus administration of the assigned treatment is administered. The assigned dose is administered every 6 or 12 hours for up to 30 days. The patients selected for the study are 18 years or older in hospice or home care settings.

Rescue medication is an IV opioid and is allowed for patients with inadequate pain control as determined by the patient. Investigator determined rescue opioid is used according to current practice.

Pain intensity and pain relief scores are obtained hourly after the first dose and then twice-daily thereafter. The pain intensity is measured by SPID and the pain relief is measured by TOTPAR. Patients are examined for the quality (burning, throbbing, sharp, electric, etc.) and severity (on an 11-point numerical pain rating scale) of the pain daily. An elicited opioid-related adverse effect assessment is made twice-daily for the first five days and then twice a week thereafter. Safety follow-up assessments are made with the patient. A quality of life assessment is determined on days 15 and 30 of the treatment.

**EXAMPLE 9: Analysis of the Pharmacokinetic Effect of HPβCD in a Diclofenac Sodium-HPβCD Complex.**

**I. Methods**

The role of cyclodextrins, such as HPβCD, in parenteral medicines is mainly solubilization of lipophilic poorly soluble drugs in water either via inclusion complex formation or via alternative mechanisms such as formation of non-inclusion complexes and nanoparticles. HPβCD represents sort of a vessel which improves transport properties of a drug and thus promotes its efficiency. After intravenous injection, a drug molecule will be subjected to two competing processes: interaction with cyclodextrin molecules and interaction with plasma components, the latter of which is mainly with HSA. Depending on the balance between drug affinity to HPβCD and HSA, a hypothetical risk exists that the cyclodextrin might cause a dramatic alteration of the pharmacokinetics of co-administered medication that can lead to drug inactivation and other adverse reactions. The stronger the drug/cyclodextrin interaction is and the weaker the drug/plasma components interaction is, the less likely that therapeutic failure or increased safety risks will manifest. In addition, when the drug/cyclodextrin complex stability constant, $K_{1:1}$, is high enough to resist the dilution counter-effect, a drug is less available for interaction with plasma components.

In order to predict the possibility of unfavorable cyclodextrin influence upon drug pharmacokinetics, a relatively simple theoretical model has been proposed. It is based on the suggestion that two kinds of equilibriums are applicable for drug/cyclodextrin solutions intended for intravenous (IV) injection. The first equilibrium takes place in the water prior to parenteral injection and can be characterized by the following equation:

*Pure water:*

$$\text{Drug} + \text{HP}\beta\text{CD} \xrightleftharpoons{K_{1:1}} \text{Drug/HP}\beta\text{CD complex}$$

$$K_{1:1} = \frac{[D/CD]}{[D] \cdot [CD]}$$

$$f_{CD} = \frac{[D/CD]}{[D] + [D/CD]}$$

where $K_{1:1}$ is the stability constant of the drug/HPβCD 1:1 complex in the aqueous formulation, [D] is the concentration of free drug, [CD] is the concentration of free HPβCD, and [D/CD] is the concentration of the drug/HPβCD complex, and $f_{CD}$ is the fraction bound to HPβCD in the aqueous solution

As soon as the solution has been injected into blood system, another competitive equilibrium process, *i.e.*

drug/plasma proteins interaction, takes place:

*Plasma:*

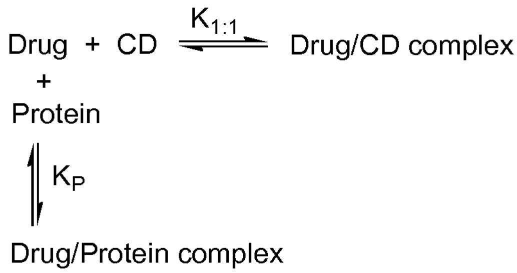


where $K_P$ is the stability constant for the drug/plasma protein 1:1 complex. In this case the fraction bound will be:

$$K_P = \frac{f_P}{[P]\cdot(1-f_P)}$$

$$K_P = \frac{[D/P]}{[D]\cdot[P]}$$

$$f_{CD\,plasma} = \frac{[D/CD]}{[D]+[D/CD]+[D/P]}$$

where fp is the fraction bound to plasma protein, [P] is the concentration of free plasma protein, $f_{CD}$ plasma is the fraction bound to HPβCD in plasma and [D/P] is the concentration of the drug/plasma protein complex.

The follow assumptions were made. First, the cyclodextrin/plasma components interactions are ignored as insignificant with respect to drug pharmacokinetics. This was based on literature data indicating that the very hydrophilic HPβCD displays negligible plasma protein binding (Sideris et al., Pharm Res 1994;11:90-5). Second, the only plasma component taken into account is HSA, which is the main component of plasma proteins. Although other components, such as globulin and cholesterol, are present in human plasma, their contribution to drug binding effect is thought to be negligible in comparison to HSA. Furthermore, the presence of such components should reduce rather than enhance the effect of HPβCD.

The maximum HPβCD plasma concentration after administration of Dyloject™ was determined to be 70 μg/ml in normal healthy subjects and 106 μg/ml in subjects with moderate renal impairment. In the following calculations, the plasma concentration of HPβCD is assumed to be 106 μg/ml (corresponding to 0.106 g/liter or $7.57\times10^{-5}$ M). The HSA concentration in plasma is between $5\times10^{-4}$ and $7.5\times10^{-4}$ M. The average plasma HSA concentration was assumed to be $6\times10^{-4}$ M.

$K_{1:1}$ values for drug/HPβCD complexes were extracted from the available literature. When $K_{1:1}$ values for a given drug/HPβCD complex were available from more than one source, the most reliable value, based on scientific quality of the source and use of experimental conditions (temperature, composition of the media etc.) closest to those in plasma, was used.

**II. Results**

Data for close to 200 different drugs was sought, of which 64 had sufficient information to permit calculations. As shown in Table 3, $K_{1:1}$ values for the drugs tested ranged from 2 $M^{-1}$ (fenofibrate) to 40,000 $M^{-1}$ (telmisartan). Plasma protein binding ranged from about 20% to over 99% although 68% were ≥90% protein bound and over 40% were ≥97% protein bound. Even for telmisartan with its $K_{1:1}$ value of 40,000 $M^{-1}$ only 2.9% will be bound to HPβCD in plasma. This small degree of binding will have a minimal effect upon the free or bound (to plasma proteins) fractions of telmisartan, and thus HPβCD will have virtually no effect on the pharmacokinetics of the drug. None of the 63 drugs tested have

sufficiently high $K_{1:1}$ values to affect the plasma protein binding sufficiently to alter their pharmacokinetics. In all cases less than 10% of the drug was bound to HPβCD in plasma. In summary, in all cases HPβCD will result in less than 10% decrease in the concentrations of free and protein-bound drug in plasma at the plasma peak HPβCD concentration of 106 μg/ml.

The calculations in Table 3 are based on competitive binding between HPβCD and HSA. As mentioned above the contribution of other plasma proteins is ignored, as is HPβCD binding to additional circulating compounds such as cholesterol. However, the effect of other compounds in plasma that can compete with the drug for HPβCD can only be to reduce the $f_{CD}$ plasma values displayed in Table 3. Thus the calculated values in Table 3 for fractional binding to HPβCD in plasma ($f_{CD}$ plasma) are maximum values, and actual values are expected to be lower. Table 3 shows the calculation of the diclofenac sodium drug fraction bound to cyclodextrin in pure water ($f_{CD}$ pure water) and in plasma ($f_{CD}$ plasma) at HPβCD concentration of 106 μg/ml (corresponding to 0.106 g/liter or $7.57 \times 10^{-5}$ M).

**Table 3: Calculation of diclofenac sodium fraction bound to cyclodextrin**

| **Drug** | **$V_d$[a]** (l/kg) | **MW[b]** (Da) | **$C_{max}$[c]** (M) | **$K_{1:1}$[d]** ($M^{-1}$) | **$f_{CD}$ pure water[e]** (%) | **Prot. bind [f]** (%) | **$f_{CD}$ plasma[g]** (%) |
|---|---|---|---|---|---|---|---|
| Acetaminophen | 0.95 | 151 | $5.0 \times 10^{-5}$ | 400[h] | 3.0 | 20 | 0.6 |
| Acetylsalicylic acid | 0.15 | 180 | $5.3 \times 10^{-4}$ | 100[1] | 0.8 | 80 | 0.2 |
| Acetazolamide | 0.2 | 222 | $1.6 \times 10^{-4}$ | 60[h] | 0.5 | 95 | 0.02 |
| Acyclovir | 0.70 | 225 | $7.2 \times 10^{-6}$ | 770[2] | 5.5 | 33 | 3.8 |
| Allegra-D (fexofenadine) | 3.5 | 373 | $1.1 \times 10^{-6}$ | 410[3] | 3.0 | 70 | 0.9 |
| Amoxicillin | 0.3 | 365 | $6.5 \times 10^{-5}$ | 2.5[h] | 0.02 | 20 | 0.02 |
| Atropine | 2.0 | 289 | $7.4 \times 10^{-9}$ | 65[h] | 0.49 | 50 | 0.00 |
| Bactrim (sulfamethoxazole ) | 0.25 | 253 | $2.3 \times 10^{-4}$ | 360[6] | 2.7 | 70 | 0.8 |
| Bimatoprost | 0.67 | 416 | $1.9 \times 10^{-10}$ | 1,900[i] | 13 | 88 | 1.7 |
| Budesonide | 3.0 | 431 | $5.4 \times 10^{-6}$ | 3,300[4] | 20 | 90 | 2.4 |
| Bupivacaine | 1.0 | 288 | $6.9 \times 10^{-6}$ | 13[5] | 0.1 | 95 | 0.00 |
| Carbamazepine | 1.4 | 236 | $3.4 \times 10^{-5}$ | 630[6] | 4.5 | 75 | 1.1 |
| Carvedilol | 1.7 | 407 | $4.0 \times 10^{-4}$ | 580[7] | 4.2 | 98 | 0.1 |
| Cefdinir | 0.35 | 395 | $7.3 \times 10^{-6}$ | 59[8] | 0.4 | 70 | 0.1 |
| Cefixime | 1.0 | 453 | $3.6 \times 10^{-4}$ | 1,400[9] | 9.5 | 65 | 3.5 |
| Celecoxib | 6.5 | 381 | $1.9 \times 10^{-6}$ | 630[10] | 4.5 | 97 | 0.1 |
| Cephalothin | 0.26 | 396 | $7.6 \times 10^{-5}$ | 70[11] | 0.5 | 80 | 0.1 |
| Chloramphenicol | 0.80 | 323 | $1.3 \times 10^{-4}$ | 120[12] | 0.9 | 80 | 0.2 |
| Chlordiazepoxide | 0.50 | 300 | $9.6 \times 10^{-6}$ | 380[13] | 2.8 | 96 | 0.1 |
| Chlortalidone (chlorthalidone) | 4.0 | 339 | $1.9 \times 10^{-5}$ | 115[14] | 0.9 | 75 | 0.2 |
| Cyclosporin | 3.0 | 1203 | $7.3 \times 10^{-4}$ | 70[15] | 0.5 | 80 | 0.1 |
| Cilest (betamethasone) | 1.2 | 393 | $2.9 \times 10^{-7}$ | 3,000[16] | 18 | 64 | 7.5 |
| Clomipramine | 12 | 315 | $1.3 \times 10^{-4}$ | 9,600[17] | 42 | 97 | 2.1 |
| Diazepam | 1.1 | 285 | $3.5 \times 10^{-6}$ | 400[18] | 2.9 | 98 | 0.06 |
| Diclofenac Na | 0.17 | 318 | $1.3 \times 10^{-5}$ | 116[h] | 0.88 | 99.5 | 0.00 |
| Diphenhydramine | 7.0 | 255 | $6.2 \times 10^{-7}$ | 1,600[19] | 10 | 80 | 2.4 |
| Econazole nitrate | Topica l | 445 | $5.5 \times 10^{-6}$ | 13[20] | 0.1 | (96) | 0.00 |
| Etodolac | 0.4 | 287 | $1.7 \times 10^{-4}$ | 60[21] | 0.5 | 99 | 0.00 |
| Estradiol | 1 | 272 | $5.3 \times 10^{-10}$ | 2,000[22] | 13 | 97 | 0.5 |
| Ezetimibe | 1.5 | 409 | $1.2 \times 10^{-8}$ | 1,300[23] | 9.0 | 90 | 1.0 |
| Fenofibrate | 0.9 | 361 | $2.8 \times 10^{-5}$ | 2[24] | 0.02 | 99 | 0.00 |
| Fentanyl | 4 | 337 | $1.4 \times 10^{-8}$ | 72[25] | 0.5 | 85 | 0.1 |
| Finasteride | 1 | 373 | $1.0 \times 10^{-7}$ | 3,800[h] | 22 | 90 | 2.8 |

(continued)

| Drug | $V_d$[a] (l/kg) | MW[b] (Da) | $C_{max}$[c] (M) | $K_{1:1}$[d] ($M^{-1}$) | $f_{CD}$ pure water[e] (%) | Prot. bind[f] (%) | $f_{CD}$ plasma[g] (%) |
|---|---|---|---|---|---|---|---|
| Fluoxetine | 27 | 309 | $1.8 \times 10^{-7}$ | 310[h] | 2.3 | 95 | 0.1 |
| Flurbiprofen | 0.12 | 244 | $6.2 \times 10^{-5}$ | 7,500[26] | 36 | 99 | 0.6 |
| Furosemide | 0.2 | 331 | $8.6 \times 10^{-6}$ | 2,100[27] | 14 | 99 | 0.2 |
| Glimepride | 0.11 | 491 | $1.1 \times 10^{-6}$ | 630[28] | 4.6 | 99.5 | 0.02 |
| Glipizide | 0.2 | 446 | $1.4 \times 10^{-6}$ | 360[29] | 2.7 | 99 | 0.03 |
| Haloperidol | 18 | 376 | $8.8 \times 10^{-8}$ | 2,100[30] | 14 | 92 | 1.3 |
| Ibuprofen | 0.18 | 206 | $2.8 \times 10^{-4}$ | 5,000[31] | 27 | 99 | 0.4 |
| Indomethacin | 1 | 358 | $5.6 \times 10^{-6}$ | 700[32] | 5.0 | 99 | 0.05 |
| Ketoprofen | 0.15 | 254 | $1.9 \times 10^{-5}$ | 110[h] | 0.8 | 99 | 0.001 |
| Ketorolac | 0.21 | 376 | $1.8 \times 10^{-6}$ | 270[h] | 2.0 | 99 | 0.02 |
| Lidocaine | 1.1 | 234 | $5.6 \times 10^{-6}$ | 17[h] | 0.13 | 70 | 0.04 |
| Medroxyprogester one acetate | - | 387 | $1.8 \times 10^{-6}$ | 260[33] | 1.9 | 90 | 0.2 |
| Meloxicam | 2 | 351 | $3.0 \times 10^{-6}$ | 1,600[34] | 11 | 99 | 0.1 |
| Miconazole | 20 | 416 | $2.9 \times 10^{-8}$ | 940[h] | 6.6 | 92 | 0.6 |
| Midazolam | 2 | 326 | $4.5 \times 10^{-7}$ | 400[h] | 2.9 | 97 | 0.1 |
| Nabumetone | 0.2 | 228 | $1.6 \times 10^{-4}$ | 3,000[35] | 19 | 99 | 0.2 |
| Naproxen | 0.16 | 230 | $3.4 \times 10^{-4}$ | 2,000[36] | 13 | 99 | 0.2 |
| Nicardipine | 8.3 | 480 | $5.3 \times 10^{-7}$ | 16[37] | 0.1 | 95 | 0.00 |
| Omeprazole | 0.35 | 354 | $4.1 \times 10^{-6}$ | 70[38] | 0.5 | 96 | 0.03 |
| Ondansetron | 1.7 | 293 | $9.0 \times 10^{-7}$ | 180[39] | 1.3 | 75 | 0.3 |
| Oxaprozin | 0.15 | 293 | $7.8 \times 10^{-4}$ | 1,400[40] | 9.5 | 99 | 0.1 |
| Pantoprazole | 0.15 | 383 | $6.5 \times 10^{-6}$ | 420[41] | 3.1 | 98 | 0.06 |
| Prednisolone | 1.5 | 360 | $1.3 \times 10^{-6}$ | 2,400[42] | 15 | 90 | 1.8 |
| Propofol | 60 | 178 | $1.4 \times 10^{-5}$ | 1,600[43] | 11 | 99 | 0.1 |
| Sertaline | 20 | 306 | $4.6 \times 10^{-7}$ | 36[44] | 0.3 | 98 | 0.00 |
| Sildenafil | 1.5 | 475 | $9.3 \times 10^{-7}$ | 70[45] | 0.5 | 96 | 0.02 |
| Tadalafil | 0.9 | 389 | $1.3 \times 10^{-6}$ | 360[46] | 2.7 | 94 | 0.2 |
| Telmisartan | 7 | 515 | $2.8 \times 10^{-6}$ | 40,000 [47] | 75 | 99 | 2.9 |
| Testosterone | 122 | 288 | $6.8 \times 10^{-10}$ | 12,000 [48] | 48 | 98 | 1.8 |
| Zolpidem | 0.54 | 307 | $4.4 \times 10^{-7}$ | 150[49] | 1.1 | 93 | 0.08 |

[a]$V_D$ - volume of distribution
[b]MW - molecular weight
[c]$C_{max}$ - maximum drug concentration in plasma based on the drug's pharmacokinetics and administration of a normal drug dose
[d]$K_{1:1}$ - stability constant of 1:1 inclusion complex (with superscripted reference number)
[e]$f_{CD}$ pure water - drug fraction bound to cyclodextrin in pure water
[f]Prot. bind. - drug fraction bound to protein ($f_P$)
[g]$f_{CD}$ plasma - drug fraction bound to cyclodextrin in plasma
[h]based on unpublished data
[i]U.S. Patent No. 6,933,289

The results indicated that none of the 63 drugs commonly co-administered perioperatively had their plasma protein binding altered by as much as 10% when given at the same time as Dyloject™. Hence, no dosing adjustments would appear to be required for a broad range of perioperative drugs concurrently administered with Dyloject™.

The contribution of other plasma proteins and the contribution of HPβCD binding of other plasma compounds such as cholesterol are ignored in this calculation. In plasma, other compounds that can compete with the drug for

HPβCD will reduce the $f_{CD}$ plasma values displayed in Table 3. Thus, the calculated value in Table 3 for fraction bound to HPβCD in plasma ($f_{CD}$ plasma) is a maximum value. Since none of the diclofenac sodium was bound to HPβCD in plasma, HPβCD does not result in a decrease in the concentration of free diclofenac sodium and protein bound diclofenac sodium in plasma at the plasma peak HPβCD concentration of 106 μg/ml. The stability constant for diclofenac sodium is 116 $M^{-1}$. This low binding amount will have a negligible effect on the fraction of diclofenac sodium that is plasma protein bound or on the fraction of the drug unbound in plasma. Thus, HPβCD will have virtually no effect on the pharmacokinetics of the drug.

The following references are noted in Table 3. [1] Choudhury et al., Pharm Res 1993;10:156-9. [2] Aicart et al., J Incl Phenom Macroc Chem 2003;47:161-5. [3]Al Omari et al., Drug Dev Ind Pharm 2007;33:1205-15. [4] Vozone et al., J Incl Phenom Macroc Chem 2003 (Volume Date 2002);44:111-5. [5]Moraes et al., Int J Pharm 2007;331:99-106. [6] Loftsson et al., Int J Pharm. 2005;302:18-28. Bhutani et al., JSci Ind Res 2007;66830-4. [8] Aleem et al., J Pharm Biomed Anal 2008;47:535-40. [9]WO/2008/110080;pp28. [10]Chowdary et al., AAPS Pharm Sci Tech 2006;7:79. [11]Loftsson et al., Pharmazie 1994;49:292-3. [12] Hirayama et al., Eur J Pharm Sci 1997;5:23-30.[13]Loftsson et al., Acta Pharm Nord 1989;1:185-94.[14] Soica et al., Revista de Chimie (Bucharest, Romania) 2007;58:606-11.[15] Malaekeh-Nikouei et al., J Incl Phenom Macroc Chem 2007;59:245-50. [16] Piel et al., Int J Pharm 2006;312:75-82. [17]Misiuk et al., Anal Lett 2008;41:543-60. [18]Loftsson et al., J Incl Phenom Macroc Chem 2007;57:545-52. [19] Le Corre et al., Int J Pharm 1998;169:221-8. [20]Morin et al., J Liq Chrom Rel Tech 2000;23:727-39. [21]Loftsson et al., J Pharm Sci 2002;91:2307-16. [22]Cappello et al., Drug Dev Ind Pharm 2009;35:877-86. [23]Patel et al., J Incl Phenom Macroc Chem 2008;60:241-51. [24] Palmieri et al., S.T.P. Parma Sci 1997;7:174-81. [25]Holvoet et al., Int J Pharm 2003;265:13-26. [26]Maitre et al., Drug Dev Ind Pharm 2007;33:311-26. [27]Vlachou et al., J Biomater Appl 2003;17:197-206. [28] Ammar et al., Int J Pharm 2006;309:129-38. [29]Zhang et al., Guangpuxue Yu Guangpu Fenxi 2008;28:711-4. [30]Loukas et al., J Pharm Biomed Anal 1997;16:263-8. [31]Mura et al., Int J Pharm 1998;166:189-203. [32]Masson et al., Int J Pharm 1998;164:45-55. [33]Loftsson et al., Int J Pharm 1993;98:225-30. [34]Baboota et al., J Incl Phenom Macroc Chem 2005;51:219-24. [35] Valero et al., J Incl Phenom Macroc Chem 1999;35:663-77. [36]Mura P et al., Int J Pharm 2003;260(2):293-302. [37]Fernandes et al., Eur J Pharm Sci 2002; 15:79-88. [38] Loftsson et al., Int J Pharm 2005;302:18-28. [39]Cho et al., Int J Pharm 2008;349:101-7. [40]Maestrelli et al., J Incl Phenom Macroc Chem 2009;63:17-25. [41] WO/2003/059393;pp16. [42]Larsen et al., J Pharm Sci 2005;94:507-15. [43]Loftsson et al., Int J Pharm 2005;302:18-28. [44]Chen et al., Sepu 2004;22:595-600. [45]Al Omari et al., J Pharm Biomed Anal 2006;41:857-65. [46]Badr-Eldin et al., Eur J Pharm Biopharm 2008;70:819-27. [47]Shewale et al., Int J Chem Sci 2008;6:1449-54. [48]Zia et al., Pharm Res 2001;18:667-73. [49]Trapani et al., J Pharm Sci 2000;89:1443-51.

**EXAMPLE 10: Novel injectable formulation of diclofenac compared with intravenous ketorolac or placebo for acute moderate-to-severe pain after abdominal or pelvic surgery: a multicenter double-blind, randomized, multiple-dose study.**

Example 2 was further developed as provided below.

**I. Methods**

*1. Patients*

At 16 U.S. sites, adults 18-65 years old were screened if they were scheduled for abdominal or pelvic surgery within 2 weeks. Key inclusion criteria were moderate-to-severe postoperative pain, defined as intensity ≥50 mm on a 0-100 mm visual analog scale (VAS) within 6 hours after surgery, and weight >50 kg. Exclusion criteria were applied to the preoperative, intraoperative, and postoperative periods. Key preoperative exclusion criteria were a history of chronic disease or severe asthma, a recent (≤6 months) cardiovascular event or clinically-significant abnormal electrocardiogram (ECG) at screening, consumption of aspirin (except <325 mg/day for antiplatelet cardiac protection), opioids, other NSAIDs, other common analgesics, major and minor tranquilizers, or antihistamines ≤24 hours before study drug initiation (except if administered during surgery), consumption of a monoamine oxidase inhibitor, tryptophan, carbamazepine, or valproate ≥2 weeks before baseline, any clinically-significant laboratory abnormality, and previous or present peptic ulceration, gastrointestinal (GI) bleeding, or any bleeding diathesis. In addition, long-acting NSAIDs or COX-2 inhibitors were to be discontinued 3 days before surgery. Subjects were also excluded in the event of a known allergy to diclofenac, NSAIDs, morphine, anesthetics, or any excipient of the study preparation, receipt of any other investigational medication within 3 months prior to administration of the study drug, known or suspected alcohol or drug abuse, and unwillingness to remain in the clinical research center for 2 nights or return within 5-9 days for a safety follow-up visit. Female subjects with a positive pregnancy test within 24 hours of surgery or who were lactating at screening were also excluded. Intraoperative exclusion criteria were subcostal incision during surgery and concomitant use of NSAIDs or acetaminophen (other intraoperative medications were not restricted). Subjects with abnormal postoperative baseline ECG were excluded

from study participation. In addition, patient-controlled analgesia (PCA) was not permitted before or during study drug dosing. Nitrous oxide and very short-acting barbiturates or benzodiazepines were allowed, provided that there was a ≥1.5-hour washout period before study drug administration, to avoid residual effects on pain intensity assessments. If insufficient washout time (<1.5 hours) preceded scheduled study drug dosage, the patient was not enrolled in the study, and did not receive the study drug. Postoperative regional anesthesia was not allowed.

2. *Study Design*

This was a multicenter, multiple-dose, multiple-day, randomized, double-blind, active- and placebo-controlled, parallel-group phase 3 study. Within 6 hours of completing surgery, patients who reported a VAS pain score ≥50 mm and met all other eligibility requirements were randomly assigned to one of four treatment groups (1:1:1:1 ratio): HPβCD diclofenac, 18.75 mg or 37.5 mg; ketorolac tromethamine 30 mg; or placebo. Assignments were made according to a computer-generated random-number code, and clinical staff and patients were blinded to study drug assignment. The first dose of study medication (1 mL IV bolus) was received by patients in all treatment arms within this first 6-hour period (Table 4).

**Table 4. Baseline Demographic and Clinical Characteristics[a]**

| Parameter, n=331 | Placebo, n=76<br>n (%) | Ketorolac 30 mg, n=82<br>n (%) | Diclofenac 18.75 mg, n=86<br>n (%) | Diclofenac 37.5 mg, n=87<br>n (%) |
|---|---|---|---|---|
| Age, years | | | | |
| Mean (SD) | 42.8 (9.66) | 42.9 (11.42) | 42.6(11) | 43.3 (10.83) |
| Gender | | | | |
| Male | 15 (19.7%) | 15 (18.3%) | 13 (15.1%) | 19 (21.8%) |
| Female | 61 (80.3%) | 67 (81.7%) | 73 (84.9%) | 68 (78.2%) |
| Ethnicity | | | | |
| Caucasian | 62 (81.6) | 60 (73.2) | 68 (79.1) | 65 (74.7) |
| Asian | 0 | 2 (2.4) | 0 | 2 (2.3) |
| Hispanic | 8 (10.5) | 10 (12.2) | 10(11.6) | 10 (11.5) |
| Black | 6 (7.9) | 10 (12.2) | 6 (7.0) | 9 (10.3) |
| Other | 0 | 0 | 2 (2.3) | 1 (1.1) |
| Height, cm | | | | |
| Mean (SD) | 166.6 (8.13) | 167.6 (9.76) | 165.5 (10.3) | 167.2 (9.62) |
| Weight, kg | | | | |
| Mean (SD) | 82.6 (19.29) | 84.2 (23.9) | 83.4(18.3) | 83.9 (18.67) |
| Range | 46-142 | 41-157 | 47-150 | 53-155 |
| Time to first dose, min | | | | |
| Mean (SD) | 132.8 (101.5) | 123.3 (96.2) | 128.2 (93.8) | 136.2 (110.1) |
| Median | 93.5 | 85.0 | 89.0 | 92.0 |
| Range | 5-417 | 7-373 | 5-376 | 12-371 |
| Surgical procedure[b] | | | | |
| Abdominal hysterectomy | 25 (18 (72.0%), 7 (28.0%)) | 20 (17 (85.0%), 3 (15.0%) | 29 (22 (75.9%), 7 (24.1%)) | 18 (15 (83.3%), 3 (16.7%)) |
| Vaginal hysterectomy | 9 (0 (0.0%), 6 (66.7%)) | 15 (0 (0.0%), 10 (66.7%)) | 13 (0 (0.0%), 6 (46.2%)) | 20 (0 (0.0%), 13 (65.0%)) |
| Abdominal surgery | 14(3 (21.4%), 11 (78.6%)) | 12 (2 (16.7%), 10 (83.3%)) | 12 (2 (16.7%), 10 (83.3%)) | 12 (4 (33.3%), 8 (66.7%)) |
| Inguinal hernia repair | 9 (8 (88.9%), 1 (11.1%)) | 14 (13 (92.9%), 1 (7.1%)) | 10 (9 (90.0%), 1 (10.0%)) | 11 (10 (90.9%), 1 (9.1%)) |
| Myomectomy | 3 (3 (100.0%),0 (0.0%)) | 5 (5 (100.0%), 0(0.0%)) | 3 (3 (100.0%), 0 (0.0%)) | 6 (6 100.0%), 0 (0.0%)) |

(continued)

| Parameter, n=331 | Placebo, n=76 n (%) | Ketorolac 30 mg, n=82 n (%) | Diclofenac 18.75 mg, n=86 n (%) | Diclofenac 37.5 mg, n=87 n (%) |
|---|---|---|---|---|
| Partial colectomy | 3 (2 (66.7%), 1 (33.3%)) | 3 (3 (100.0%), 0 (0.0%)) | 1 (0 (0.0%), 1 (100.0%)) | 2 (0 (0.0%), 2 (100.0%)) |
| Pelvic surgery | 4 (1 (25.0%), 3 (75.0%)) | 5 (1 (20.0%), 4 (80.0%)) | 6 (0 (0.0%), 6 (100.0%)) | 6 (0 (0.0%), 6 (100.0%)) |
| Salpingo-oophorectomy | 2 (0 (0.0%), 2 (100.0%)) | 3 (2 (66.7%), 1 (33.3%)) | 5 (3 (60.0%), 2 (40.0%)) | 2 (2 (100.0%), 0 (0.0%)) |
| Ventral hernia repair | 1 (1 (100.0%),0 (0.0%)) | 1 (1 (100.0%), 0 (0.0%)) | 3 (3 (100.0%), 0 (0.0%)) | 3 (2 (66.7%), 1 (33.3%)) |
| Other | 6 (2 (33.3%), 4 (66.7%)) | 4 (2 (50.0%), 2 (50.0%)) | 4 (3 (75.0%), 1 (25.0%)) | 7 (3 (42.9%), 4 (57.1%)) |
| Baseline pain intensity, VAS[c] | | | | |
| n[d] | 76 | 80 | 85 | 86 |
| Mean (SD) | 67.7 (14.12) | 67.8 (13.81) | 67.0 (12.58) | 70.8 (15.64) |
| Median | 65.5 | 65.0 | 65.0 | 69.0 |
| Range | 50-98 | 50-99 | 50-100 | 50-100 |

[a] Number of patients per study center ranged from 1-80, with 10 centers providing 1-20 subjects, 4 centers providing 21-40 subjects, and 2 centers providing >40 subjects.

[b]Total (n open procedures (% of total), n laparoscopic procedures (% of total)); note that for some patients, open vs. laparoscopic was not specified

[c] VAS = visual analog scale (0-100 mm).

[d] Four randomized subjects did not have baseline pain intensity values and were not included in this assessment

Administration of the first dose of study drug was taken as time 0, and all subsequent dosing and evaluation time points were in relation to time of first study drug dose. Subsequent injections were received every 6 hours until discharge or until patient withdrawal/discontinuation from the study, due either to an adverse event (AE), inadequate pain control, noncompliance with the study protocol, or at the investigator's discretion. Patients were observed for at least 48 hours from baseline (study drug initiation), unless discharged earlier, and for up to 5 days.

Rescue medication (bolus IV morphine 5 mg, titrated up to 7.5 mg after 30 minutes if analgesia was inadequate) was available upon patient request, up to once every 3 hours any time after administration of the initial dose of study drug, but patients were encouraged to wait at least 1 hour following study medication injection. Patients were not denied rescue medication, and if adequate analgesia was not achieved with morphine, the patient was withdrawn from the study and given pain medication in accordance with the investigator's usual practice.

3. *Outcome Measures and Assessments*

Pain intensity was assessed at rest and recorded by subjects on a 0-100 mm VAS (0 = "no pain"; 100 = "worst pain imaginable") at specified time points (5, 10, 15, 30, 45 minutes, and 1, 2, 3, 5, 6, 9, 12, 15, 18, 21, 24, 27, 30, 33, 36, 39, 42, 45, and 48 hours; see study timeline as shown in Figure 6) over the 48 hours following the first dose of study medication. Figure 6 shows that upon meeting screening and baseline qualifying criteria, patients were to receive study drug for 2-5 days. Key efficacy assessment and safety evaluation time points are indicated.Patients remaining at the site longer had their pain assessed every 6 hours until discharge. The primary efficacy measure was SPID (in mm·hours) over the 0-48 hour time interval after the first dose of study drug. Assessments were reported by patients and scored using standardized tools. Pain intensity difference (PID) was calculated at each time point by subtracting recorded pain intensity from baseline pain intensity. SPID was calculated as the area under the curve of the PID scores. Secondary efficacy measures were:

- SPID over 0-24 hours
- Total pain relief (area under the pain relief curve for the 0-24 and 0-48-hour intervals (0-72, 0-96, and 0-120 hours as well, if data permitted); pain relief was recorded using a 0-100 mm VAS (0 = "no relief'; 100 = "complete pain

relief') at the same time points at which pain intensity was recorded (excluding baseline)

- Proportion of patients with clinically-meaningful (≥ 30%) reduction in pain intensity (vs. baseline, using 0-100 mm VAS)
- PID at each scheduled assessment
- Time from administration of study drug to administration of rescue medication
- Frequency and amount of rescue medication
- Patient-reported global evaluation of the study drug at 24 and 48 hours on a 5-point categorical scale ("excellent," "very good," "good," "fair," "poor").

Patients returned for a safety follow-up 5-9 days after baseline, and received a follow-up telephone call 30 days post-baseline. Safety assessments included physical examinations, laboratory testing, vital signs, 12-lead ECG , and evaluation of thrombophlebitis at the site of study drug injection using a 6-point scale (Table 5 and Figure 6). AEs were recorded throughout the study.

**Table 5. Injection site thrombophlebitis scale**

| Grade / scale | Severity / description |
|---|---|
| 0 | No reaction |
| 1 | Tenderness along vein |
| 2 | Continuous tenderness or pain with redness |
| 3 | Palpable swelling or thrombosis within the length of cannula |
| 4 | Palpable swelling or thrombosis beyond the length of the cannula |
| 5 | As for Grade 4, with overt infection |

Statistical Analysis

Study sample size was based on the calculation that eighty patients in the intent-to-treat (ITT) population per treatment group would provide 80% power to detect a difference in pain intensity of 540 mm·hours between placebo and diclofenac groups over a 48-hour period. This calculation was based on an estimated standard deviation of 1,200 mm·hours projected from Christensen et al., Anesth Prog (2011);58:73-81.

Efficacy analyses were conducted using Statistical Analysis Software®, and unless otherwise noted refer to the intent-to-treat population. SPID efficacy measures and pain relief scores were calculated using the trapezoidal rule. For SPID calculations, evaluations after administration of rescue medication or after withdrawal due to AEs or lack of efficacy were imputed in accordance with prespecified rules. If rescue medication was required, pain intensity and relief assessments were obtained before rescue analgesic administration. If rescue medication was administered within 3 hours of the next scheduled assessment, the worst assessment over the preceding 6 hours was carried forward. If the assessments necessary to do this were unavailable, assessments were imputed with the baseline score. For patients discontinuing due to AEs or lack of efficacy, baseline scores were carried forward. The same rules were applied to pain relief assessments.

PID, amount of rescue medication, and patient global evaluation were analyzed using analysis of covariance models with treatment and center as factors and baseline pain intensity as a covariate. Differences between active treatments and placebo were tested with linear contrasts. Comparisons with respect to the primary efficacy measure were performed as follows: diclofenac 37.5 mg vs. placebo at the 0.05 level of significance; if the result was significant, diclofenac 18.75 mg was tested versus placebo at the 0.05 level of significance. Ketorolac was employed as an active comparator to confirm assay sensitivity. Comparisons between the diclofenac and ketorolac groups were not performed, as the study was not powered to discern significant differences between active treatments.

The proportion of patients reporting ≥30% reduction from baseline in pain intensity was analyzed with the Cochran-Mantel-Haenszel test with center as a stratification variable. Time to meaningful (≥30%) reduction in pain intensity and time to administration of rescue medication were analyzed with Kaplan-Meier survival analysis techniques. Descriptive statistics were used for AEs, laboratory test results, vital signs, thrombophlebitis, and ECG results. Logistic regression was used to estimate the relative risk of cardiovascular events.

**II. Results**

Altogether, 348 patients were randomly assigned to a treatment arm following surgery (≥85 subjects per treatment group) and 331 received ≥1 dose of study drug (see Figure 7). Figure 7 shows the distribution of patients in study groups and reasons for study withdrawal. Of the 17 subjects who were randomized but did not receive study drug, the main reason for exclusion was a failure to meet eligibility criteria, as outlined above (12/17 subjects (70.6%)). Of these

12 subjects, insufficient pain on the VAS scale was the predominant reason for exclusion (9/12 (75%)). All 331 patients receiving ≥1 treatment dose were included in the ITT population and were assessed for demographics, efficacy, and safety. Distribution of the ITT population between treatment groups was as follows: placebo, n=76; diclofenac 18.75 mg, n=86; diclofenac 37.5 mg, n=87; ketorolac, n=82. The majority of patients (80.1%, n=265) completed the study. The median number of doses received across treatment groups was 8 (range, 1-13). Forty-nine patients (14.8%) received study drug for 1 day, 267 (80.6%) for 2 days, and 15 (4.5%) for ≥3 days.

Most patients were female (81%) and Caucasian (77%; Table 4). The mean age in each treatment group was 43 years, and mean subject body weight was 84 kg. There were no significant differences across treatment groups for any baseline characteristic (all p > 0.05). The aggregate mean baseline pain intensity was 68.4 mm, within the moderate-to-severe range. At baseline, 60% of patients had moderate pain (50 < VAS < 70) and 40% had severe pain (VAS >70). Pain intensity at baseline was not significantly different among treatment groups.

*1. Efficacy*

1.1. *Primary efficacy measure*

Over the first 48 hours after study drug initiation, mean SPID was significantly greater for both doses of HPβCD diclofenac (18.75 mg, p = 0.032; 37.5 mg, p = 0.0001), and for ketorolac (p < 0.0001), than for placebo (see Figure 8). Figure 8 shows the sum of pain intensity differences (SPID) from 0-24 hours and 0-48 hours. Visual analog scale (VAS) pain intensity was assessed at baseline and at specified intervals in the 48 hours subsequent to first drug dose. Pain intensity difference was calculated as the baseline pain intensity minus pain intensity at each scheduled assessment (larger numbers indicate greater pain relief). SPID was shown for the 0-24 and 0-48 hour time periods for placebo, ketorolac 30 mg, diclofenac 18.75 mg, and diclofenac 37.5 mg (error bars indicate standard error (SE)). There were no significant differences in SPID between active treatments. *$p$ < 0.05, **$p$ < 0.0001 v.s. placebo. These results were consistent regardless of baseline pain intensity. There were no statistically significant differences in efficacy between the three active treatment groups.

1.2. *Secondary efficacy measures*

Similar to the 0-48 hour interval, SPID over the 0-24 hour interval was significantly greater than placebo for both HPβCD diclofenac doses (18.75 mg, p = 0.015; 37.5 mg, p < 0.0001) and ketorolac, (p < 0.0001) (Figure 3). For the 0-72 hour period, 18.75 mg diclofenac did not lead to a significantly greater SPID than placebo (p = 0.08), but 37.5 mg diclofenac (p=0.0010) and ketorolac (p=0.0018) did significantly improve SPID. Mean PID was consistently greater with the active treatments than with placebo over the first 45 hours, with the exception of the 6-hour and 30-hour assessments.

The criterion for meaningful pain relief (≥30% reduction) was based on the threshold previously reported as meaningful for acute pain in the postoperative setting. During the first 6-hour dosing period, 55.3% (n=42) of patients receiving placebo had a ≥30% reduction in pain intensity, while 76.8% (n=63) of patients on ketorolac, 64.3% (n=54) of patients on 18.75 mg HPβCD diclofenac, and 69.8% (n=60) of patients on 37.5 mg HPβCD diclofenac reported a ≥30% reduction. The mean time to ≥30% pain intensity reduction among subjects reporting this decline within 6 hours following first study drug dose was rapid across all treatment groups (27-33 minutes for the modified ITT population). Median times to ≥30% pain intensity reduction did not differ between any of the active treatment groups and placebo (all p > 0.05).

Total pain relief was significantly greater with active treatment than with placebo over the 0-24 and 0-48 hour time intervals (p = 0.0002 and 0.0008, respectively). Use of both 18.75 mg and 37.5 mg diclofenac resulted in significantly greater mean total pain relief than placebo (18.75 mg: p=0.037 for the 0-24 hour interval and 0.038 for the 0-48 hour interval; 37.5 mg: p = 0.0018 for the 0-24 and 0-48 hour intervals), as did use of 30 mg ketorolac (p < 0.0001 for the 0-24 hour interval and p = 0.0001 for the 0-48 hour interval). There were no significant differences between active treatments.

Median time to rescue morphine administration in the ITT population was 2:07 (hours:minutes) (95% CI: 1:15 - 2:40) for placebo, but was significantly longer with 18.75 mg diclofenac (3:14, 95% CI: 2:10 - 5:05; p = 0.014 vs. placebo) and ketorolac (4:15, 95% CI: 3:05 - not estimable; p = 0.0007 vs. placebo). Time to rescue morphine administration did not meet statistical significance versus placebo with 37.5 mg diclofenac. (2:24, 95% CI: 1:50 - 4:23; p = 0.0574).

Amount and frequency of rescue opioid administration: Active treatment decreased the frequency of rescue morphine administration, and for all time intervals studied, patients on active treatments required significantly less morphine compared with the placebo group (see Figure 9). Figure 9 shows the mean amount of rescue morphine administered. Rescue medication (intravenous morphine) was available any time after the initial dose of study drug. Subjects, however, were encouraged to wait at least 1 hour after the initial study drug dosing. Mean rescue morphine administered per day post-surgery is shown for days 1 (0-24 hours), 2 (24-48 hours), and 3 (48-72 hours). The total cumulative dose received

post-surgery (0-72 hours) was 15.9 mg for placebo, 8.5 mg for ketorolac (30 mg), 8.8 mg for the 18.75 mg dose of diclofenac, and 7.4 mg for the 37.5 mg dose of diclofenac. ***p* < 0.0001 v.s. placebo for 0-24, 0-48, and 0-72 hour intervals. For the 0-24 hour interval, patients receiving 18.75 mg diclofenac, 37.5 mg diclofenac, or 30 mg ketorolac experienced 39%, 44%, and 40% reductions in rescue morphine dosage, respectively, as compared to those treated with placebo (all $p \leq 0.0001$). All active treatments led to a significant reduction in morphine dosage over the 0-48 and 0-72 hour intervals, as well (all $p \leq 0.0001$).

Patient global evaluations in each of the active treatment groups were significantly superior to placebo ($p < 0.001$) at both 24 and 48 hours, with no significant differences between active treatment groups. Altogether, 83%-87% of patients in the active treatment groups assessed their study drug as "good," "very good," or "excellent" at 48 hours.

2. *Safety*

Overall, 84.6% (280/331) of patients experienced ≥1 AE. Most events were mild-to- moderate in severity. Nausea, flatulence, and injection site pain/irritation were the most commonly-reported AEs among patients receiving active treatments (Table 6). Moderate-to-severe pain has been shown to be a risk factor for postoperative nausea and vomiting, 17 both of which were most commonly reported in the placebo group (Table 6).

**Table 6. Summary of Adverse Events**

| AE (n=331 total subjects) | Placebo, n=76 n (%) | Ketorolac 30 mg, n=82 n (%) | Diclofenac 18.75 mg, n=86 n (%) | Diclofenac, 37.5 mg, n=87 n (%) | Total Diclofenac, n=173 n (%) |
|---|---|---|---|---|---|
| Nausea | 29 (38.2%) | 22 (26.8%) | 26 (30.2%) | 22 (25.3%) | 48 (27.7%) |
| Flatulence | 19 (25.0%) | 22 (26.8%) | 22 (25.6%) | 12 (13.8%) | 34 (19.7%) |
| Injection site pain, irritation | 5 (6.6%) | 17 (20.7%) | 19 (22.1%) | 14 (16.1%) | 33 (19.1%) |
| Constipation | 11 (14.5%) | 8 (9.8%) | 17 (19.8%) | 16 (18.4%) | 33 (19.1%) |
| Headache | 15 (19.7%) | 14 (17.1%) | 9 (10.5%) | 7 (8.0%) | 16 (9.2%) |
| Insomnia | 9 (11.8%) | 7 (8.5%) | 9 (10.5%) | 7 (8.0%) | 16 (9.2%) |
| Vomiting | 11 (14.5%) | 7 (8.5%) | 7 (8.1%) | 5 (5.7%) | 12 (6.9%) |
| Blood Creatine phosphokinase (CPK) increased | 3 (3.9%) | 12 (14.6%) | 7 (8.1%) | 6 (6.9%) | 13 (7.5%) |
| Pyrexia | 8 (10.5%) | 9 (11.0%) | 2 (2.3%) | 6 (6.9%) | 8 (4.6%) |
| Thrombophlebitis | 9 (11.8%) | 6 (7.3%) | 6 (7.0%) | 3 (3.4%) | 9 (5.2%) |
| Pruritus | 5 (6.6%) | 3 (3.7%) | 4 (4.7%) | 6 (6.9%) | 10 (5.8%) |
| Tachycardia | 5 (6.6%) | 4 (4.9%) | 2 (2.3%) | 2 (2.3%) | 4 (2.3%) |
| Diarrhea | 3 (3.9%) | 6 (7.3%) | 2 (2.3%) | 0 (0.0%) | 2 (1.2%) |
| **Number of patients experiencing ≥1 AE** | **62 (81.6%)** | **72 (87.8%)** | **73 (84.9%)** | **73 (83.9%)** | **146 (84.4%)** |

Sixty-seven patients (20.2%) across the entire study population experienced at least one AE considered treatment-related by the investigator. The incidence of treatment-related AEs was 23.2% (19/82) in the ketorolac 30 mg group, 19.8% (17/86) in the diclofenac 18.75 mg group, 19.7% (15/76) in the placebo group, and 18.4% (16/87) in the diclofenac 37.5 mg group). One serious AE (SAE, abdominal hematoma) occurred in the ketorolac group and was considered possibly treatment-related. Of nine AEs that prompted withdrawal from the study, one (moderate peripheral edema, in the diclofenac 18.75 mg group) was suspected of being treatment-related. There were no deaths.

The incidence of cardiovascular AEs was 5.4% (18/331) overall, 9.2% (7/76) in the placebo group, 6.1% (5/82) in the ketorolac group, 4.6% (4/87) in the diclofenac 37.5 mg group, and 2.3% (2/86) in the diclofenac 18.75 mg group. No cardiovascular AE was considered treatment-related. Blinded third-party analysis of ECGs revealed no clinically meaningful findings. Injection site pain/irritation was more common in active treatment groups than with placebo (Table

6). Mild-to-moderate borderline elevations of liver enzymes were reported for 2%-5% of patients across all four groups. There were no reported hepatic or renal-related AEs or acute hepatic or renal impairment.

The incidence of bleeding-related AEs was 6.6% (5/76) in the placebo group, 6.1% (5/82), in the ketorolac group, 5.7% (5/87) in the diclofenac 37.5 mg group, and 2.3% (2/86) in the diclofenac 18.75 mg group (Table 7). There were no declines in hemoglobin or platelets between baseline and follow-up in any treatment group. Among subjects receiving anticoagulants or medications with anticoagulant properties, post hoc analysis revealed that 4/105 (3.8%) subjects receiving either dose of diclofenac reported ≥1 bleeding-related AE, while 3/49 (6.1%) and 4/47 (8.5%) from the ketorolac and placebo groups, respectively, had bleeding-related AEs.

**Table 7. Bleeding- and Wound-Healing-Related Adverse Events**

| **Patients, n=331 total** | **Placebo, n=76 n (%)** | **Ketorolac 30 mg, n=82 n (%)** | **Diclofenac 18.75 mg, n=86 n (%)** | **Diclofenac 37.5 mg, n=87 n (%)** | **Total Diclofenac, n=173 n (%)** |
|---|---|---|---|---|---|
| Patients on Concomitant Anticoagulants | 47 (62%) | 49 (60%) | 55 (64%) | 50 (58%) | 105 (61%) |
| Type of Bleeding-Related AE[a] | | | | | |
| Anemia | 3 (3.9%) | 2 (2.4%) | 0 (0.0%) | 4 (4.6%) | 4 (2.3%) |
| Rectal Hemorrhage | 0 (0.0%) | 1 (1.2%) | 1 (1.2%) | 0 (0.0%) | 1 (0.6%) |
| Vaginal Hemorrhage | 1 (1.3%) | 0 (0.0%) | 1 (1.2%) | 0 (0.0%) | 1 (0.6%) |
| Hematoma | 0 (0.0%) | 0 (0.0%) | 0 (0.0%) | 1 (1.1%) | 1 (0.6%) |
| Abdominal Hematoma | 0 (0.0%) | 1 (1.2%) | 0 (0.0%) | 0 (0.0%) | 0 (0.0%) |
| Incision site complication | 1 (1.3%) | 0 (0.0%) | 0 (0.0%) | 0 (0.0%) | 0 (0.0%) |
| Wound complication | 0 (0.0%) | 1 (1.2%) | 0 (0.0%) | 0 (0.0%) | 0 (0.0%) |
| Wound dehiscence | 0 (0.0%) | 1(1.2%) | 0 (0.0%) | 0 (0.0%) | 0 (0.0%) |
| **Total patients with a Bleeding-Related AE** | **5 (6.6%)** | **5 (6.1%)** | **2 (2.3%)** | **5 (5.7%)** | **7(4.0%)** |
| **Total Patients on Concomitant Anticoagulants with a Bleeding-Related AE** | **4 (8.5%)** | **3 (6.1%)** | **2 (3.6%)** | **2 (4.0%)** | **4 (3.8%)** |

[a] Adverse event

The results of this study establish the analgesic efficacy of multiple-dose injectable HPβCD diclofenac for the treatment of acute postoperative pain, confirming and extending data from two randomized, double-blind trials establishing the efficacy of single-dose HPβCD diclofenac after third-molar extraction (Leeson et al., Reg Anesth Pain Med (2007);32:303-10; Christensen et al., Anesth Prog (2011);58:73-81). Leeson *et al.,* found that both HPβCD diclofenac 75 mg and the original injectable 75 mg diclofenac formulations were superior to placebo regarding the primary endpoint of total pain relief over 4 hours, and demonstrated similar AE profiles. In a second study, 14 in which patients were eligible only if they had a baseline VAS-rated pain intensity of moderate-to-severe, HPβCD diclofenac was superior to placebo for total pain relief over 6 hours for 4 of 5 doses tested (75, 37.5, 18.75, and 9.4 mg). In addition, the 37.5 mg and 75 mg HPβCD diclofenac doses were superior to placebo at the earliest assessment of pain relief (5 minutes), whereas a standard 30-mg dose of ketorolac was not.

Injectable diclofenac formulations containing propylene glycol and benzyl alcohol, the form heretofore available outside the U.S. for the prevention or treatment of postoperative pain, require slow infusion over a period of 30-120 minutes. This study demonstrates that small IV bolus delivery of HPβCD diclofenac without a loading dose is efficacious for the treatment of acute moderate-to-severe pain following abdominal or pelvic surgery. Delivery of 18.75 mg or 37.5 mg dosages every 6 hours provided significant analgesic efficacy over placebo. Analgesic efficacy was also significant in subjects receiving a standard dose of 30 mg ketorolac. All three active drugs were significantly more effective than placebo as measured by the sum of pain intensity differences, total pain relief, and average amount of rescue morphine. No treatment-related SAEs were reported in either diclofenac dose group, while one SAE (abdominal hematoma) reported in the ketorolac group was suspected of being treatment-related. Neither diclofenac nor ketorolac was associated with an increased incidence of bleeding-related AEs.

Confirming clinical experience, the use of rescue medication in this study was greatest in the first 24 hours postoperatively. The opioid-sparing effect of the active treatments, compared with placebo, was ≥40% during every time interval studied, a key finding given that meta-analysis reveals that morphine reduction of this magnitude significantly

decreases the incidence of postoperative vomiting and sedation. Significant opioid-sparing effects were previously noted with injectable diclofenac in a study that compared a single dose of IV diclofenac 75 mg with IV ketorolac 60 mg and placebo in 102 patients undergoing orthopedic surgery (Alexander et al., J Clin Anesth (2002);14:187-92). NSAIDs reduced morphine requirements versus placebo by up to 29% over 24 hours, and significantly reduced postoperative nausea, vomiting, and pruritus.

The vast majority of literature on NSAID use for multimodal acute postoperative pain control describes NSAIDs as generally useful for mild-to-moderate, but not severe pain. In this study, however, bolus IV injection of diclofenac (and the active comparator ketorolac) proved efficacious for severe, as well as moderate pain, thereby extending the clinical applicability of NSAIDs to a pain intensity not previously thought to be routinely controllable with an NSAID plus minimal amounts of rescue opioid medication. In addition, the ability to administer this formulation by bolus as opposed to a prolonged infusion offers the opportunity for a more rapid onset of pain relief, as well as reduced time that the IV line cannot be used to deliver concomitant, potentially incompatible agents.

In conclusion, this study demonstrates that a novel IV formulation of diclofenac, a well-established NSAID with a known safety profile, provides a high degree of efficacy for the treatment of acute moderate and severe pain following abdominal or pelvic surgery. Within the patient population studied, both HPβCD diclofenac doses (18.75 mg and 37.5 mg) provided significantly greater analgesic efficacy than placebo, as did the active comparator ketorolac. For pain management, as with pharmacotherapy in general, it is recommended that clinicians use the lowest effective dose for the shortest necessary time. HPβCD diclofenac's ability to be used as a primary analgesic option for patients arriving in the PACU with moderate or severe pain, as demonstrated in this study, may offer advantages over other parenteral non-narcotic analgesic formulations, particularly when exposure to high dosages of NSAIDs and/or opioids pose significant risk to the patient.

## EXAMPLE 11: Analgesic Efficacy and Safety of a Novel Injectable Formulation of Diclofenac Compared with Intravenous Ketorolac and Placebo after Orthopedic Surgery: A Multicenter, Randomized, Double-blinded, Multiple-Dose Trial.

Example 1 was further developed as provided below.

### I. Materials and Methods

*1. Patients*

Following institutional review board (IRB) approval and IRB-approved written informed consent, patients were screened at 12 study sites. Key inclusion criteria included age 18-85 years, weight 36-136 kg, and expectation of moderate to severe postsurgical pain requiring continuous IV analgesia following elective orthopedic surgery. Key exclusion criteria included dehydration in the elderly, recent history of cardiovascular events, history of gastric ulcers, severe renal or hepatic impairment, and recent use of other analgesics (Table 8).

**Table 8. Exclusion criteria**

| |
|---|
| • Dehydrated and age >65 years |
| • Recent history (≤6 months) of a cardiovascular event (e.g., myocardial infarction, stroke) |
| • History of uncontrolled conditions, such as gastric erosion/ulceration or bleeding diathesis, renal impairment, or cardiac failure that required hospitalization in the month prior to screening or in the opinion of the investigator made participation in the study inadvisable |
| • Recent use (≤2 weeks) of a monoamine oxidase inhibitor, tryptophan, carbamazepine, or valproate |
| • Recent use (≤24 hours) of aspirin (except 325 mg or less of cardiac-protective daily dosing), other NSAIDs, or other common analgesics; centrally acting analgesic adjuvants; tranquilizers; or antihistamines, except medications administered during surgery. All opioids, long-acting NSAIDs or cyclooxygenase-2 inhibitors must have been discontinued ≤3 days prior to surgery. |
| • Hepatic dysfunction determined by Child-Pugh score >9 |
| • Screening serum creatinine >3 mg/dL |
| • Intra-articular corticosteroid injection within the last 3 months unless the operation was to replace a single injected joint; then the exclusion was within the last month |

2. *Study Design*

This was a multicenter, multiple-dose, multiple-day, randomized, double-blind, active- and placebo-controlled, parallel-group study. Patients with moderate or severe pain within 6 hours postoperatively, defined as pain intensity of ≥50 mm on a 0-100 mm visual analog scale (VAS), were randomly assigned to IV HPβCD diclofenac, ketorolac, or placebo (2:1:1 ratio).

Assignments were made according to a computer-generated random-number code. Randomization was also stratified by risk cohort (high-risk, non-high-risk, or high-weight [≥95 kg]) at baseline (see Figure 10), and by anticipated long stay (>24 hours) versus shorter stay, resulting in six strata within the randomization schedule. Figure 10 shows the distribution of patients randomized. A total of 277 patients received at least one dose of study drug and composed the intent-to-treat and safety populations. Overall, 239 (86%) patients completed the study (51/72 on placebo, 71%; 56/60 on ketorolac, 93%; 132/145 on diclofenac, 91%). "High-risk" patients were defined as those who weighed <50 kg, were ≥65 years of age, were undergoing medical ulcer therapy, had a renal (a serum creatinine 1.9-3.0 mg/dL) or hepatic insufficiency (Child-Pugh score 6-9), or a history of gastrointestinal bleeding or perforation.

The HPβCD diclofenac dose was 37.5 mg in the non-high-risk cohort, 18.75 mg in the high-risk cohort, and 50 mg in the high-weight cohort. The ketorolac dose was 30 mg in the non-high-risk and high-weight cohorts and 15 mg in the high-risk cohort. Patients in all treatment groups received a bolus IV injection every 6 hours until discharge. Patients were observed for at least 24 hours from baseline (study drug initiation) and for up to 5 days. All study drugs were blinded to the investigator and the patient; dose levels of the individual study treatments were not blinded.

Rescue IV morphine was available if the primary study drug did not provide relief and was given as needed in 2.5 mg increments and not exceeding a total of 7.5 mg every three hours. Patients were encouraged to wait at least 30 minutes after study drug initiation to request morphine, to allow primary study drug to begin to exert analgesic effects but were never denied.

3. *Outcome Measures and Assessments*

The primary efficacy measure was the sum of pain intensity differences (SPID) over five intervals: 0-24, 0-48, 0-72, 0-96, and 0-120 hours which was calculated by summing the difference in pain intensity (by a 0-100 visual analog scale) from baseline at designated timepoints. The secondary efficacy measures were: total pain relief over 0-24, 0-48, 0-72, 0-96, and 0-120 hours (overall and in the subgroup of patients with severe pain at baseline), the proportion of patients attaining clinically meaningful reduction in pain intensity (≥30%), pain intensity difference (PID) at each scheduled assessment, the amount and frequency of rescue morphine administration, and patient global evaluation on a 5-point scale (0, Poor; 1, Fair; 2, Good; 3 Very good; 4 Excellent). Patients rated the study drug every 24 hours after the start of dosing and at completion/early termination.

Patients assessed their pain at baseline and at specified time points through the next 24 hours (5, 10, 15, 30, 45 minutes, and 1, 2, 3, 5, 6, 9, 12, 15, 18, 21, 24 hours following the first dose). Those who remained at the site longer assessed their pain every 3 hours until discharged. Safety assessments included physical examinations, laboratory testing, vital signs, 12-lead electrocardiography, evaluation of thrombophlebitis at injection site, and a prospective investigator-administrated wound-healing questionnaire. Adverse events (AEs) were recorded between surgery and randomization, not just after baseline, in order to distinguish treatment-emergent AEs.

4. *Statistical Analysis*

A sample of 120 patients on HPβCD diclofenac (including both doses) and 60 patients on placebo were needed to provide 95% power to detect a clinically significant difference for each time interval in the primary efficacy measure. This calculation was based on an estimated standard deviation of 468 for the 0-24 hour interval obtained from a randomized, placebo-controlled pivotal trial the sponsor conducted in an orthopedic surgical population.

Efficacy analyses were conducted using Statistical Analysis Software®, and unless otherwise noted refer to the intent-to-treat population. The analysis of SPID, pain intensity difference, total pain relief, patient global evaluation, and amount of rescue medication were based on analysis of covariance (ANCOVA) models, with treatment and center as factors and baseline pain as a covariate. Confidence intervals were based on the pooled standard deviation obtained from an ANCOVA model. Treatment differences were tested with linear contrasts.

All testing of statistical significance was 2-sided unless the test performed was inherently 1-sided. Interaction *P*-values <0.1 were considered significant; otherwise, *P*-values <0.05 were considered significant. For SPID, comparisons between HPβCD diclofenac and placebo were performed in the following order: 0-24, 0-48, 0-72, 0-96, and 0-120 hours. If any of the comparisons failed to demonstrate statistical significance, no further comparisons were made. Total pain relief was analyzed similarly.

The proportion of patients attaining at least 30% reduction in pain intensity and the frequency of rescue medication use were analyzed with Cochran-Mantel-Haenszel tests, with center as a stratification variable.

For summed calculations, evaluations after the administration of rescue medication or after withdrawal due to adverse events (AEs) or lack of efficacy were imputed in accordance with prespecified rules. For subgroup analyses of pain intensity, a more conservative and simplified set of imputation rules was applied.

## II. Results

### 1. *Patients*

A total of 277 patients were randomized and received ≥1 dose of study drug (see Figure 10). Altogether, 239 patients (86%) completed the study. The most frequent reason for withdrawal was lack of efficacy (31 patients, 11%), which was most common in the placebo group and the long-stay population. Most study participants were female (178 patients, 64%) and Caucasian (255 patients, 92%) (see Table 9). The mean age was 55 years, and 82 patients (30%) were ≥65 years of age. Six patients (2%) were <50 kg and 66 (24%) were >95 kg.

**Table 9. Baseline Demographic and Clinical Characteristics**

| Parameter | HPβCD diclofenac (N=145) | Ketorolac (N=60) | Placebo (N=72) | Total (N=277) |
|---|---|---|---|---|
| Gender, n (%) | | | | |
| Male | 53 (36.6) | 20 (33.3) | 26 (36.1) | 99 (35.7) |
| Female | 92 (63.4)) | 40 (66.7) | 46 (63.9) | 178 (64.3) |
| Age, years | | | | |
| Mean (SD) | 55.9 (14.35) | 54.9 (15.77) | 54.5 (15.67) | 55.3 (14.97) |
| Weight, kg | | | | |
| Mean (SD) | 88.86 (21.79) | 87.42 (18.89) | 87.12 (22.99) | 88.10 (21.45) |
| Range | 45.0-143.2 | 53.2-136.4 | 47.7-138.3 | 45.0-143.2 |
| Risk cohort, n (%) | | | | |
| Non-high-risk | 63 (43.4) | 28 (46.7) | 32 (44.4) | 123 (44.4) |
| High-risk | 46 (31.7) | 18 (30.0) | 24 (33.3) | 88 (31.8) |
| High-weight | 36 (24.8) | 14(23.3) | 16(22.2) | 66 (23.8) |
| Risk category, n (%) | | | | |
| Age ≥65 years | 42 (29.0) | 17 (28.3) | 23 (31.9) | 82 (29.6) |
| Weight <50 kg | 5 (3.4) | 0 | 1 (1.4) | 6 (2.2) |
| Hepatic impairment | 3 (2.1) | 0 | 1 (1.4) | 4 (1.4) |
| Ulcer history | 0 | 1 (1.7) | 0 | 1 (0.4) |
| Length of stay, n (%) | | | | |
| Short stay (<24 hours) | 62 (42.8) | 28 (46.7) | 32 (44.4) | 122 (44.0) |
| Long stay (>24 hours) | 83 (57.2) | 32 (53.3) | 40 (55.6) | 155 (56.0) |
| Surgical procedure, n (%) | | | | |
| Bunionectomy, foot other | 46 (31.7) | 20 (33.3) | 23 (31.9) | 89 (32.1) |
| Knee replacement | 38 (26.2) | 16(26.7) | 22 (30.6) | 76 (27.4) |
| Knee surgery other | 23 (15.9) | 5 (8.3) | 6 (8.3) | 34 (12.3) |
| Hip replacement | 19 (13.1) | 6 (10.0) | 7 (9.7) | 32 (11.6) |
| Spine surgery | 4 (2.8) | 2 (3.3) | 5 (6.9) | 11 (4.0) |
| Lower-extremity soft tissue excision or repair | 6 (4.1) | 2 (3.3) | 3 (4.2) | 11 (4.0) |
| Shoulder surgery other | 3 (2.1) | 5 (8.3) | 2 (2.8) | 10 (3.6) |
| Ankle surgery | 3 (2.1) | 2 (3.3) | 2 (2.8) | 7 (2.5) |
| Baseline pain intensity, VAS | | | | |
| Mean (SD) | 69.54 (14.23) | 72.17 (15.19) | 66.83 (13.12) | 69.40 (14.23) |

(continued)

| Parameter | HPβCD diclofenac (N=145) | Ketorolac (N=60) | Placebo (N=72) | Total (N=277) |
|---|---|---|---|---|
| Median | 66.00 | 71.00 | 64.00 | 66.00 |
| Range | 50-100 | 50-100 | 50-100 | 50-100 |
| Severity | | | | |
| Moderate (≥50-70 mm VAS) | 84 (57.9%) | 29 (48.3%) | 46 (63.9%) | 159 (57.4%) |
| Severe (≥70 mm VAS) | 61 (42.1%) | 31 (51.7%) | 26 (36.1%) | 118 (42.6%) |
| NSAID = nonsteroidal anti-inflammatory drug; SD = standard deviation; VAS = 0-100 mm visual analog scale. | | | | |

There were no significant differences across treatment groups for any baseline characteristic, either overall or within any risk cohort, in duration of surgery, duration of anesthesia, or time from end of surgery until study dose initiation. Most of the patients received study drug for ≤3 days (≤1 day, 153 patients, 55%; ≤2 days, 169 patients, 61%; ≤3 days, 252 patients, 91%; ≤5 days, 277 patients, 100%). Of the 277 patients, 169 (61%) had 1-8 doses, 83 (30%) had 9-12 doses, and 25 (9%) had >13 doses.

The overall mean pain intensity on VAS was 69 mm at baseline (see Table 9), and did not differ across treatment groups or risk cohorts. Altogether, 157 (57%) of the 277 patients had moderate pain (50 mm ≤ VAS < 70 mm) and 118 (43%) had severe pain (VAS ≥ 70 mm). Eleven patients were assigned to the wrong risk cohort at baseline, and in 4 cases the dosage was adjusted. The subgroup analyses presented here are based on dose levels received.

*2. Efficacy*

In all time intervals, the mean sum of pain intensity differences (SPID) was significantly greater for diclofenac and ketorolac than for placebo (*P* < 0.0001) (see Figure 1). Figure 1 shows the sum of pain intensity differences (SPID) over time. Mean SPID scores and standard errors are shown for five time intervals for placebo, ketorolac, and HPβCD diclofenac. Larger values indicate greater reduction of pain from baseline. The percentages of imputed SPID values over 0-48, 0-72, 0-96, and 0-120 hours were 47%, 54%, 62%, and 68%, respectively. "*" represents that the *P* value is < 0.0001 versus placebo. These results were consistent across baseline pain intensities. There were no significant differences in SPID values based on length of stay.

Total pain relief over 0-24, 0-48, 0-72, 0-96, and 0-120 hours was significantly better with HPβCD diclofenac and ketorolac than with placebo (*P* < 0.0001). In the subgroup of 118 (43%) of 277 patients who had severe pain at baseline, total pain relief over all time intervals was significantly better with HPβCD diclofenac and ketorolac than with placebo (*P* ≤ 0.05).

Clinically meaningful pain reduction (≥30% reduction in intensity) was achieved by 117 (81%) of the 145 patients on HPβCD diclofenac and 45 (75%) of the 60 patients on ketorolac, compared with 31 (43%) of the 72 patients on placebo. The proportion of patients who had meaningful pain reduction with HPβCD diclofenac was significantly superior to the proportion with ketorolac at 10 minutes, 42 hours, 48 hours, and 60 hours (*P* ≤ 0.05 for all comparisons).

HPβCD diclofenac evidenced a faster onset of analgesia as measured by pain intensity difference. HPβCD diclofenac was significantly greater than placebo at 10 minutes (*P* = 0.03), while ketorolac required 30 minutes (*P* = 0.006). For active treatments, statistical separation from placebo was maintained for 120 hours. Patient global evaluations for the active treatments were significantly higher than for placebo (*P* < 0.0001) (see Figure 11). Figure 11 shows the patient global evaluation at last assessment. At last assessment, mean scores for the active treatments were significantly higher than for placebo (*P* < 0.0001), with 99 (70.2%) of 141 patients on HPβCD diclofenac and 34 (57.6%) of 59 patients on ketorolac rating their drug as "very good" or "excellent," versus 16 (22.9%) of the 70 patients on placebo.

*3. Rescue*

HPβCD diclofenac-treated patients required significantly less morphine than the placebo control group (*P* < 0.0001) (see Figure 2). Figure 2 shows the cumulative amount of rescue medication administered over time. The total morphine requirement over the first 5 days was 42% lower with HPβCD diclofenac than with placebo (11.8 v.s. 20.5 mg), and in every time interval the opioid-sparing effect was ≥40% with HPβCD diclofenac compared with placebo. "*" represents that the *P* value is < 0.0001 versus placebo. "#" represents that the *P* value is < 0.05 versus ketorolac. Over the 5 days of treatment, patients on HPβCD diclofenac also used significantly less morphine than those receiving ketorolac (11.8 v.s. 18.1 mg, 35% reduction v.s. ketorolac, *P* = 0.008) (see Figure 3). Figure 3 shows the cumulative proportions

of subjects in each treatment group who required rescue medication. Of the patients who required rescue morphine, more than half required rescue ≤2 times in the HPβCD diclofenac and ketorolac groups, compared with ≤6 times in the placebo group. The median time from administration of study drug to administration of rescue medication (tm) was greatest for HPβCD diclofenac (tm=220.0 minutes, P < 0.0001 compared to placebo) followed by ketorolac (tm=137.0 minutes, P < 0.0001 compared to placebo) and placebo (tm=51.0 minutes).

*4. Risk cohorts*

SPID scores for HPβCD diclofenac were significantly higher than placebo across risk and weight cohorts, and significantly higher than ketorolac in the high risk cohort at the 0-24 and 0-48 hour intervals (see Figure 12). Figure 12 shows the sum of pain intensity differences (SPID) by risk cohort. Mean SPID scores for 5 time intervals for placebo, ketorolac, and HPβCD diclofenac. "*" represents that the P value is < 0.05 versus placebo. "#" represents that the P value is < 0.05 versus ketorolac. The efficacy of diclofenac in high-risk elderly patients over time compared to that of placebo and ketorolac is shown in Figure 13. One subgroup of high-risk patients, those ≥65 years of age (n=80), who received low-dose HPβCD diclofenac had a greater response rate than those receiving low-dose ketorolac or placebo, as defined by 30% reduction in pain intensity (see Figure 13A). Elderly HPβCD diclofenac-treated patients needed less rescue medication than those given ketorolac or placebo (P = 0.05), and less frequently (see Figure 13B). Figure 13A shows the percentage of patients attaining at least 30% reduction in pain intensity across treatments. "*" represents the *P* value is < 0.05 versus placebo. Figure 13B shows the mean amount of rescue medication in milligrams by treatment versus placebo over time. The total amount of morphine used was 35.5% less in elderly patients treated with low-dose HPβCD diclofenac versus those treated with low-dose ketorolac (9.6 v.s. 14.9 mg).. "#" represents the P value is < 0.05 versus. ketorolac.

*5. Safety*

No deaths were reported. Overall, 216 (78%) of the 277 patients studied reported one or more AEs, with a similar incidence in the active treatment groups (Table 10), and risk cohorts. Most AEs (92%) were mild to moderate in severity. Nausea was the most common AE overall and in each risk cohort. Within the high-weight cohort, use of HPβCD diclofenac 50 mg did not increase the risk of an AE compared with placebo. Forty-seven (17%) of the 277 patients reported one or more AEs thought to be treatment-related (Table 10). This included 7 (8%) of the 88 patients in the high-risk cohort, 27 (22%) of the 123 patients in the non-high-risk cohort, and 13 (20%) of the 66 patients in the high-weight cohort.

**Table 10. Summary of Adverse Events (AEs)**

| | **HBβCD diclofenac (n=145)** | **Ketorolac (n=60)** | **Placebo (n=72)** |
|---|---|---|---|
| **Total GI-Related Adverse Events (n (%))** | **48 (33.1%)** | **24 (40.0%)** | **40 (57.1%)** |
| **Most Common GI-Related** | | | |
| Nausea | 36 (24.8%) | 18 (30.0%) | 26 (36.1%) |
| Vomiting | 11 (7.6%) | 6 (10.0%) | 14 (19.4%) |
| Constipation | 19 (13.1%) | 6 (10.0%) | 11 (15.3%) |
| | | | |
| **Total Renal-Related Adverse Events (n (%))** | **5 (3.5%)** | **1 (1.7%)** | **1 (1.4%)** |
| Acute Renal Failure / Renal Insufficiency | 1 (0.7%) * | 0 | 1 (1.4%) † |
| Acute Tubular Necrosis | 0 | 0 | 1 (1.4%) † |
| Nephrolithiasis | | | 1 (1.4%) † |
| Urinary Retention | 3 (2.1%) | 0 | 0 |
| Dysuria (burning, difficulty urinating) | 1 (0.7%) | 1 (1.7%) | 0 |
| | | | |
| **Total Bleeding related adverse events (n (%))** | **23 (15.9%)** | **13 (21.7%)** | **12 (16.7%)** |

(continued)

| | | | |
|---|---|---|---|
| **Most Common Bleeding-related** | | | |
| Anemia | 16 (11.0%) | 9 (15.0%) | 11 (15.3%) |
| Epistaxis | 2 (1.4%) | 0 | 0 |
| Contusion | 1 (0.7%) | 2 (3.3%) | 1 (1.4%) |
| | | | |
| **Other AEs Occurring in >10% Patients** | | | |
| Blood creatine phosphokinase (CPK) increase | 21 (14.5%) | 8 (13.3%) | 9 (12.5%) |
| Dizziness | 16 (11.0%) | 5 (8.3%) | 5 (6.9%) |
| Fever / increase body temp. | 6(4.1%) | 5 (8.3%) | 14 (19.4%) |

* 81 year obese male undergoing hip replacement. P reoperative history of renal insufficiency, anemia, pulmonary HTN and CHF. Patient experienced postoperative anemia and hypotension and increase in creatine.(1.5 to 2.6). Patient received hydration with normal saline, 1 unit packed red blood cells and d/c furosemide, valsartan and HPβCD diclofenac. Mild renal insufficiency resolved after 1 day.
† 63 year obese female undergoing knee replacement. Preoperative history of kidney stones and urinary incontinence. Patient experienced postoperative hypotension that did not respond to fluid boluses, creatinine increased from normal to 2.5 mg/dL. Patient transferred to ICU and treated with vasopressors. Patient responded and weaned from vasopressors after 2 days. Creatinine returned to normal 3 days after its initial increase. Acute renal failure, tubular necrosis resolved after 5 days

Five patients had serious cardiovascular events: three (2.1%) of the 145 patients on HPβCD diclofenac developed deep vein thrombosis (2 of these patients were >95 kg), one (1.7%) of the 60 patients on ketorolac developed congestive heart failure, and one (1.4%) of the 72 patients on placebo developed hypotension. None of these events was considered treatment-related.

The incidence of bleeding-related AEs was similar in the active treatment groups (HPβCD diclofenac, 23/145 patients, 16%; ketorolac, 13/60 patients, 22%) and not significantly greater than in the placebo group (12/72 patients, 17%). Likewise, in the subset of patients who received anticoagulants (n=197), there were no clinically meaningful differences in bleeding-related AEs across treatment groups. There were no major differences in renal or liver function tests between the active treatment groups and placebo.

This study establishes the safety and efficacy of IV HPβCD diclofenac for managing acute moderate and severe pain alone or in combination with opioids in patients recovering from orthopedic surgery. HPβCD diclofenac 18.75 mg, 37.5 mg, or 50 mg every 6 hours was found to provide superior analgesia to those receiving placebo and rescue morphine and greater effects than ketorolac beginning at 0-48 hours. During the trial, all treatment groups, including placebo, could receive supplemental morphine as needed to help manage pain. HPβCD diclofenac-treated patients required 35% less rescue morphine than those treated with ketorolac (P = 0.008). When rescue morphine was needed, the median time until administered was 220 minutes for HPβCD diclofenac, more than four times longer than placebo (51 minutes) and 83 minutes longer than in those receiving ketorolac (137 min).

Elderly patients receiving low-dose HPβCD diclofenac (18.75 mg) had statistically significant better outcomes than those receiving low-dose ketorolac (15 mg). This included a higher likelihood of analgesic response, better analgesic efficacy, and lower overall opioid requirements than those receiving ketorolac. These are important findings given that the elderly are at greater risk of opioid- and NSAID-induced side effects and lower dosages are warranted of each to minimize their exposure and risk.

In the perioperative period, side effects of particular concern with NSAIDs are renal impairment and increased risk of bleeding. Safety data was consistent with what has been demonstrated in a randomized, multi-dose trial of HPβCD diclofenac following major abdominal or pelvic surgery (Gan et al., Acute Pain, (2008);10: 165-166). Blood loss in this study was slightly greater with the active treatments than with placebo.

In this study, HPβCD diclofenac proved efficacious in the immediate postoperative period for the 118 (43%) of the 277 patients who had severe pain at baseline, as well as for those with moderate pain. The results suggest giving HPβCD diclofenac to patients with a pain severity not previously thought to be controllable with an NSAID alone (plus minimal amounts of rescue medication). This could have particularly important implications for the elderly patient populations that routinely undergo orthopedic procedures.

In conclusion, this study demonstrates that a novel IV formulation of diclofenac, a long-trusted NSAID with a

well-characterized safety profile, is safe and efficacious for treatment of acute moderate and severe pain following commonly performed orthopedic surgeries, particularly in the elderly. While renal insufficiency and bleeding complications were infrequent, awareness of risk factors associated with their origins should be carefully assessed. Furthermore, the data suggest using HPβCD diclofenac as a default primary postoperative analgesic, with morphine added as necessary, rather than the reverse.

**EXAMPLE 12: Safety of a Novel Parenteral Formulation of Diclofenac after Major Orthopedic or Abdominal Surgery: An Open-Label, Multi-Day, Repeated-Dose Clinical Trial.**

Example 3 was further developed as provided below.

**I. Materials and Methods**

*1. Study subjects*

The study protocol and informed consent were reviewed and approved by each site's participating Institutional Review Board prior to subject enrollment. After providing written consent, subjects were screened at 52 sites, with 51 sites enrolling ≥1 subject. Key inclusion criteria were age 18-85 years and the expectation that, within three weeks after the screening evaluation, the subject would undergo abdominal (laparoscopic or non-laparoscopic), orthopedic, abdominal/pelvic or any other surgery that would qualify for ≥ 2 days of scheduled parenterally-administered NSAIDs over multiple days.

Subjects were excluded if they had a history or evidence of significant cardiovascular, respiratory, renal, hepatic, or other gastrointestinal disease, or a psychiatric disorder that would make study participation unacceptably risky. Other key exclusion criteria were a history of coronary artery bypass graft surgery, hepatic insufficiency at screening (serum bilirubin >2.5 mg/dL), prothrombin time (PT) >20% above the upper limit of normal), significant renal insufficiency at screening (serum creatinine >1.9 mg/dL), warfarin treatment ≤1 week prior to surgery (or the expectation that warfarin treatment would begin before last study drug dose), intraoperative IV NSAID use, known or suspected drug or alcohol abuse, allergy or hypersensitivity to diclofenac, other NSAIDS, or any of the excipients of the study drug preparation, and pregnancy or lactation. Qualifying individuals with serum creatinine > 1.1 mg/dL (women) or >1.3 mg/dL (men) but less than 1.9 mg/dL at screening were identified as having impaired renal function but were not excluded from entry into the clinical trial.

*2. Study Design and Procedures*

This was a multicenter, open-label, repeated-dose, multiple-day, single-arm safety study. Administration of HPβCD diclofenac, given as an IV bolus, began in the immediate post-operative period, as soon as the patient was stable following surgery according to the study site's usual practice. The study drug was administered every 6 hours (±15 minutes) around the clock as the primary postoperative analgesic until the subject was either completely transitioned to oral analgesics, discharged from the institution, received treatment for 5 days, or was discontinued from the study. Most patients (65%) received 37.5 mg doses of HPβCD diclofenac, and dosage was not reduced for elderly patients or for those with hepatic or renal impairment. Based on a previous pharmacokinetic study and efficacy findings from a multiple-day, randomized, controlled trial, patients ≥95 kg (35%) received 50 mg HPβCD diclofenac, in order to maintain equivalent exposure and efficacy. Excepted from this dosage adjustment were subjects ≥95 kg with >1 NSAID-related risk factor. The 37.5 mg dose was administered as a 1.0-mL IV bolus and the 50-mg dose as a 1.33-mL IV bolus.

Opioids or other standard postoperative analgesics (except for other NSAIDs or controlled-release opioids), were allowed on an as-needed basis to supplement the primary analgesic effects of IV HPβCD diclofenac, and were given according to the institution's standard of care. Concomitant treatment with commonly used drugs with anticoagulant properties was permitted during the study. Coumadin/warfarin was not permitted during co-administration of the study drug, but, was allowed to be given beginning on the last day of study drug administration.

Subjects were considered to have completed the study if they received ≥8 consecutive doses of diclofenac (≥2 days of continuous treatment) and completed all follow-up requirements (in-clinic follow-up 10 days following last study drug dose and follow-up telephone call 30-37 days following last dose).

*3. Assessments*

Clinical laboratory tests (hematology, biochemistry, urinalysis) and 12-lead electrocardiography (ECG) were completed at screening, baseline (immediately prior to beginning treatment), and discharge/early termination. For laboratory analyses, baseline values were defined as the most recent value obtained postoperatively and prior to first dose

of study drug. Vital signs were collected at screening, baseline, discharge/early termination, and upon follow-up. Physical examinations were conducted at screening and upon follow-up. Vital signs were collected at screening, baseline, discharge/early termination, and follow-up.

3.1 Adverse Events

AEs were recorded from the signing of the informed consent through the follow-up telephone call, and were followed through resolution or to 30 days following administration of the last dose of study drug, whichever occurred first. Treatment-emergent AEs were defined as those which first occurred or worsened in severity during the course of the study, regardless of their relationship to study drug. The relationship of AEs to treatment was defined by investigators as "not related," "unlikely," "suspected," or "probable," and AEs classified as "treatment-related" were those categorized by the investigator as having a "suspected" or "probable" relationship to study drug.

3.1.1 Bleeding-related events

Events categorized as 'Bleeding-Related AEs' were those classified by the clinical investigator as a clinically-relevant bleeding AE. Changes in laboratory values such as prolonged prothrombin time (PT), prolonged activated partial thromboplastin time (PTT), international normalized ratio (INR) increase, and hematocrit decrease in a given patient were not classified as bleeding-related AEs unless the investigator also noted recorded an event that was significant enough to be coded as a clinical AE according to MEDRA conventions in the same individual. Post-operative anemia was not classified as a bleeding related AE unless the clinical investigator classified the anemic event as likely being caused by the study drug versus an expected outcome of the surgical procedure. Bleeding related AEs included the following verbatim descriptions recorded by clinical investigators, regardless of the investigator's classification of likelihood of the adverse event being caused by HPβCD diclofenac: rectal hemorrhage, hemorrhagic anemia, small foul smelling hematoma inferior to surgical site, anal hemorrhage, ecchymosis bilateral forearms at injection site, vaginal hemorrhage, blood loss from surgical incision, hematoma posterior to left colon at surgical site, postoperative wound bleeding, excessive blood drainage at level of left knee at surgical incision, GI bleeding at the level of surgical anastomosis, upper GI bleeding, acute blood loss anemia, blood emesis, hematuria, superficial clot at IV site, infected left hip hematoma, hematochezia, and hematoma right knee.

3.1.2 Renal-related AEs

Events categorized as "significant renal AEs" were those classified as a clinically significant renal AE. Changes in laboratory values such as increased blood creatinine, decreased renal creatinine clearance, and increased blood urea in a given patient were not classified as significant renal AEs unless the investigator also noted a clinical renal AE in these patients that could be coded within recognized MEDRA conventions in the same individual. Significant renal AEs included the following verbatim descriptions regardless of likelihood of the adverse event being caused by HPβCD diclofenac: decreased urine output, acute renal failure, renal tubular necrosis, acute renal insufficiency, oliguria, azotemia, and anuria.

3.2 Patient global evaluation of treatment efficacy

An overall global evaluation of treatment was obtained at discharge/early termination. Patients were asked to rate their treatment experience as "Excellent," "Very good," "Good," "Fair," or "Poor."

**II. Results**

*1. Demographics /Characteristics*

Altogether, 1171 patients were screened for the study, 1050 were enrolled, and 971 received HPβCD diclofenac and were included in the safety population analysis (Figure 14). Figure 14 depicts the study subject disposition. As shown in Figure 14, a total of 971 subjects received ≥1 dose of intravenous (IV) HPβCD diclofenac for acute postoperative pain, and were included in the safety analysis. In total, 943 (97.1%) of subjects receiving ≥1HPβCD diclofenac dose completed the study. For the 79 patients who were enrolled but did not receive the study drug, the most common reasons were patient withdrawal of consent (n=32), cancellation of surgery (n=13), AEs occurring after informed consent and before the time of initiation of study drug (n=8), and the use of prohibited medication (n=8).

Table 11 provides detailed the demographics for the 971 post surgical patients studied. The mean age was 58.8 years with a significant proportion of patients greater than 65 (38%) and 75 (12%) years old. Most subjects underwent

major surgical procedures (e.g., total knee or total hip replacement, open hysterectomy, laparotomy), and 85% of patients studied received HPβCD diclofenac for two to three days following surgery at a dosing schedule of every six hours. Over 60% of the patients studied also received concomitant anticoagulants for DVT prophylaxis. The most common medications used included heparin (7.8%) and low molecular weight heparins (LMMW) (51.3%). Warfarin use on the last day of treatment was permitted and was identified in 134 patients (13.8%) of those studied. Many patients remained on low-dose aspirin (81 mg to 325 mg/QD) or clopidogrel for cardiac prophylaxis in addition to the anticoagulants used for DVT prophylaxis. A total of 12 patients (1.2%) were treated with aspirin or clopidogrel. Patients with mild pre-existing renal impairment were not excluded from the study, and a total of 57 (5.9%) of patients with renal impairment were evaluated. Two different dosage levels of HPβCD diclofenac were also evaluated 37.5 mg and a higher 50 mg dose for patients weighing ≥95 kg each delivered every 6 hours.

**Table 11. Demographics and clinical characteristics of study patient population**

| Demographic Parameter | Total n=971[a]; n (%) |
|---|---|
| Age | |
| <65 years | 604 (62.2) |
| 65-75 years | 250 (25.7) |
| >75 years | 117 (12.0) |
| Mean (SD) | 58.8 (13.4) |
| Median (range) | 60 (18-87) |
| Gender | |
| Male | 354 (36.5) |
| Female | 617 (63.5) |
| Dose received | |
| 37.5 mg | 634 (65.3) |
| 50 mg | 335 (34.5) |
| 18.75 mg (in error) | 2 (0.2) |
| Length of exposure to study drug | |
| 1 day | 41 (4.2) |
| 2 days | 607 (62.5) |
| 3 days | 220 (22.7) |
| 4-5 days | 103 (10.6) |
| Mean (SD) | 2.4 (0.8) |
| Median (range) | 2.0 (1-5) |
| Major surgical procedure(s) performed Orthopedic | 676 (69.6) |
| Total knee replacement | 450 (46.3) |
| Total hip replacement | 137(14.1) |
| Other[b] | 89 (9.2) |
| Abdominal / pelvic | 293 (30.2) |
| Gynecologic / Genitourinary | 152 (15.6) |
| Abdominal | 141 (14.5) |
| Renal function at enrollment | |
| Renal impairment[c] | 57 (5.9) |
| Normal renal function | 914 (94.1) |
| Concomitant anticoagulant use | |
| Receiving concomitant DVT anticoagulant | 602 (62.0) |
| Heparin | 76 (7.8) |
| LMWH | 498 (51.3) |
| Warfarin[d] | 134 (13.8) |
| Aspirin / clopidogrel | 12 (1.2) |

(continued)

| Demographic Parameter | Total n=971[a]; n (%) |
|---|---|
| Not receiving concomitant DVT anticoagulant | 369 (38.0) |

[a] 51 study sites; highest-enrolling sites had 124 (12.8% of total), 87 (9.0%), and 80 (8.2%) subjects, respectively
[b] Includes spinal fusion, rotator cuff repair, laminectomy, fracture repair, discectomy, and other, unspecified
[c] Serum creatinine >1.1 mg/dL (women) >1.3 mg/dL (men) or urine creatinine >300 mg/dL
[d] Beginning on the last day of study drug administration

*2. Safety*

A total of 823 (84.8%) patients reported a treatment-emergent AE and 85 (8.8%) reported a treatment-related adverse event considered by the clinical investigator as "suspected" or "probably" related to HPβCD diclofenac administration.

Table 12 presents the most common treatment-emergent AEs in the study population (occurring in >5% of the patients studied). Also presented in Table 12 are the treatment-emergent AEs that were reported in >5% of patients AND were considered as "suspected" or "probably" related to the administration of the study drug. Table 13 presents treatment-emergent and treatment-related bleeding -related AEs reported by patients in the study population, and Table 14 presents treatment-emergent and treatment-related significant renal AEs that occurred during the study period.

**Table 12. Treatment-emergent and treatment-related adverse events (AEs) in the study population**

| Adverse Event | Treatment-Emergent Events [a] (n=971 total subjects) | | | Treatment-Related Events[b] (n=971 total subjects) | |
|---|---|---|---|---|---|
| | n | % | | n | % |
| Nausea | 361 | 37.2% | | 6 | 0.6% |
| Postoperative anemia | 218 | 22.5% | | 0 | 0.0% |
| Constipation | 181 | 18.6% | | 0 | 0.0% |
| Insominia | 130 | 13.4% | | 0 | 0.0% |
| Pruritus | 125 | 12.9% | | 3 | 0.3% |
| Vomiting | 83 | 8.5% | | 2 | 0.2% |
| Blood CPK increase | 63 | 6.5% | | 11 | 1.1% |
| Hypotension | 60 | 6.2% | | 0 | 0.0% |
| Pyrexia | 58 | 6.0% | | 0 | 0.0% |
| Headache | 56 | 5.8% | | 1 | 0.1% |
| Infusion site pain / irritation | 50 | 5.1% | | 35 | 3.6% |
| Dizziness | 49 | 5.0% | | 0 | 0.0% |

[a] Adverse events occurring in ≥ 5% of study population
[b] Adverse Events considered by investigator as "suspected" or "probably" caused by HPβCD diclofenac and occurring in > 1 individual Other treatment-related events: acute renal failure (6 patients), dyspepsia (5 patients), blood creatinine increase (4 patients), abnormal liver function test (4 patients), urine output decreased (2 patients) and infusion site thrombosis (2 patients)

**Table 13. Treatment-emergent and treatment-related bleeding-related adverse events (AEs) and postoperative anemia in the study population**

| Adverse Event | Treatment-Emergent Events (n=971 total subjects) | | | Treatment-Related Events [a] (n=971 total subjects) | |
|---|---|---|---|---|---|
| | n | % | | n | % |
| Hemorrhagic anemia | 3 | 0.3% | | 0 | 0.0% |
| Hematochezia | 3 | 0.3% | | 0 | 0.0% |

(continued)

| Adverse Event | Treatment-Emergent Events (n=971 total subjects) | | | Treatment-Related Events [a] (n=971 total subjects) | |
|---|---|---|---|---|---|
| | n | % | | n | % |
| Hematemesis | 1 | 0.1% | | 0 | 0.0% |
| Rectal hemorrhage | 1 | 0.1% | | 0 | 0.0% |
| Upper GI hemorrhage | 1 | 0.1% | | 1 | 0.1% |
| Anal hemorrhage | 1 | 0.1% | | 1 | 0.1% |
| Infusion site bruising | 1 | 0.1% | | 0 | 0.0% |
| Infusion site hematoma | 1 | 0.1% | | 0 | 0.0% |
| Infusion site hemorrhage | 1 | 0.1% | | 1 | 0.1% |
| Injection site hemorrhage | 1 | 0.1% | | 0 | 0.0% |
| Hematoma infection | 1 | 0.1% | | 0 | 0.0% |
| Incision site hemorrhage | 7 | 0.7% | | 0 | 0.0% |
| Postprocedural hemorrhage | 4 | 0.4% | | 0 | 0.0% |
| Incision site hematoma | 2 | 0.2% | | 0 | 0.0% |
| Anastomotic hemorrhage | 1 | 0.1% | | 0 | 0.0% |
| Vaginal hemorrhage | 1 | 0.1% | | 0 | 0.0% |
| Ecchymosis | 1 | 0.1% | | 0 | 0.0% |
| Wound hemorrhage | 3 | 0.3% | | 0 | 0.0% |
| Hematoma | 2 | 0.2% | | 0 | 0.0% |
| Postoperative anemia | 218 | 22.5% | | 0 | 0.0% |
| [a] Adverse Events considered by investigator as "suspected" or "probably" caused by HPβCD diclofenac and occurring in > 1 individual | | | | | |

**Table 14. Treatment-emergent and treatment-related significant renal adverse events (AEs) in the study population**

| Adverse Event | Treatment-Emergent Events (n=971 total subjects) | | | Treatment-Related Events [a] (n=971 total subjects) | |
|---|---|---|---|---|---|
| | n | % | | n | % |
| Decreased urinary output | 9 | 0.9% | | 2 | 0.2% |
| Acute renal failure | 8 | 0.8% | | 4 | 0.4% |
| Oliguria | 2 | 0.2% | | 1 | 0.1% |
| Anuria | 1 | 0.1% | | 1 | 0.1% |
| Azotemia | 1 | 0.1% | | 1 | 0.1% |
| Renal tubular necrosis | 1 | 0.1% | | 1 | 0.1% |
| Acute renal insufficiency | 1 | 0.1% | | 0 | 0.0% |
| Increased blood creatinine | 10 | 1.0% | | 4 | 0.4% |

(continued)

| Adverse Event | Treatment-Emergent Events (n=971 total subjects) | | | Treatment-Related Events [a] (n=971 total subjects) | |
|---|---|---|---|---|---|
| | n | % | | n | % |
| Increased blood urea | 2 | 0.2% | | 1 | 0.1% |
| Decreased renal creatinine clearance | 1 | 0.1% | | 0 | 0.0% |

[a] Adverse Events considered by investigator as "suspected" or "probably" caused by HPβCD diclofenac and occurring in > 1 individual

2.1 Bleeding-related AEs

Thirty-two patients (3.3%) in the study population reported an AE that was described by the investigator as a clinical bleeding-related event (Table 13). In addition, 218 (22.5%) patients reported postoperative anemia of mild to moderate severity. Of these 218 incidences of postoperative anemia, none were considered related to study drug by the clinical investigator responsible (Table 13). Given the frequency of mild-to-moderate anemia that occurs as part of recovery from major surgical procedures, the lack of severity of these events in the patients studied, as well as, the lack of association of this event by clinical investigators with HPβCD diclofenac co-administration, the balance of safety results will focus on AEs described by clinical investigators as bleeding-related and having the potential for significant medical consequences.

Table 15 presents the incidence of bleeding-related AEs in various well-recognized at-risk patient population categories, such as patients with advanced age, concomitant anticoagulant use, prolonged duration of dosing, elevated NSAID dosage, and patients undergoing major surgical procedures. A statistically significant difference in the incidence of bleeding-related AEs was observed noted between patients receiving HPβCD diclofenac following major abdominal surgery (5.7%) and those receiving HPβCD diclofenac follwing gynecological or genitourinary procedures (1.3%) p = 0.04. No significant difference between orthopedic and abdominal / pelvic or within orthopedic specific procedures (*e.g.*, total knee versus total however (Table 15). In addition, no other risk factor examined was associated with a significant increase in the incidence of bleeding-related AEs in the study population.

**Table 15. Incidence of bleeding-related adverse events (AEs) in at-risk postsurgical patient populations**

| Postsurgical patient population | Bleeding-related adverse events | | | |
|---|---|---|---|---|
| | **n** | **%** | **95% CI** | **P** |
| **Total (n=971)** | **32** | **3.3%** | **2.26; 4.62** | |
| **Age** | | | | |
| <65 years (n = 604) | 19 | 3.1% | 1.90 ; 4.87 | NS [a] |
| 65 to 75 years (n = 250) | 11 | 4.4% | 2.22 ; 7.74 | |
| > 75 years (n = 117) | 2 | 1.7% | 0.21 ; 6.04 | |
| **Concomitant Anticoagulant Use** | | | | |
| With Anticoagulants (n = 602) | 22 | 3.7% | 2.30 ; 5.48 | NS |
| Without Anticoagulants (n = 369) | 10 | 2.7% | 1.31 ; 4.93 | |
| **Duration of Dosing** | | | | |
| 2 days (n = 648) | 21 | 3.2% | 2.02 ; 4.91 | NS |
| > 2 but ≤ 5 days (n = 323) | 11 | 3.4% | 1.71 ; 6.01 | |
| **Dose Received** | | | | |
| Normal Dose (37.5 mg; n = 634) | 22 | 3.5% | 2.19 ; 5.21 | NS |
| High Dose (50 mg; n = 355) | 10 | 3.0% | 1.44 ; 5.42 | |

(continued)

| Type of Surgery | | | | |
| --- | --- | --- | --- | --- |
| Orthopedic (n = 676) | 22 | 3.3% | 2.05 ; 4.89 | NS |
| Total Knee Replacement (n = 450) | 10 | 2.2% | 1.07 ; 4.05 | |
| Total Hip Replacement (n = 137) | 7 | 5.1% | 2.08; 10.24 | |
| Other Orthopedic (n = 89) | 5 | 5.6% | 1.85 ; 12.63 | |
| | | | | |
| Abdominal / Pelvic (n = 293) | 10 | 3.4% | 1.65 ; 6.19 | NS |
| Gynecological / Genitourinary ( n = 152) | 2 | 1.3% | 0.16; 4,67 | |
| Abdominal (n = 141) | 8 | 5.7% | 2,48 ; 10.87 | p = 0.04 [b] |

[a] NS = non-significant differences between groups
[b] Versus gynecological / genitourinary group

Two observations are particularly notable with respect to bleeding-related AEs in at-risk populations. First, it is of interest to note that age as a single risk factor did not contribute to the incidence of bleeding-related AEs. In fact, the lowest incidence of bleeding-related AEs was observed for patients >75 years old (1.7% (2/117 patients) versus 3.1% and 4.4% in patients <65 and 65-75 years old, respectively). Additionally, concurrent use of anticoagulants with HPβCD diclofenac did not increase the risk of bleeding-related AEs.

2.3 Renal AEs

The risk of acute renal failure associated with NSAID use is another well-recognized clinical concern with regard to postsurgical patient populations. Thus, reports of acute renal failure and decreased urinary output were of particular interest in this study, and the effect of HPβCD diclofenac on the incidence of these renal AEs was closely evaluated within numerous patient subpopulations with and without recognized risk factors for NSAID-induced renal failure. The incidences of acute renal failure and decreased urinary output were evaluated in patients with and without the following risk factors associated with NSAID-induced decreases in renal function: prior history of compromised renal function, advanced age, surgery ≥2 hours in duration, >2 days drug exposure, elevated drug dosage, and major surgical procedures (Table 16).

A total of 17 patients experienced either acute renal failure (n=8) or decreased urinary output (n=9). Patients ≥75 years of age were at a significantly higher risk of developing one of these renal AEs following surgery than those < 75 years old (p = 0.001) (Table 16). While patients > 75 years old had a significant p-value noted, the confidence intervals (indication of similarity with small numbers) did overlap suggesting no difference.

**Table 16. Incidence of acute renal failure and decreased urinary output adverse events (AEs) in at-risk postsurgical populations**

| Postsurgical patient population | Acute renal failure or decreased urinary output adverse events | | | |
| --- | --- | --- | --- | --- |
| | n | % | 95% CI | P |
| **Total** (n=971) | 17 | 1.8 | 1.02; 2.79 | |
| Acute renal failure | 8 | 0.8 | | |
| Decreased urinary output | 9 | 0.9 | | |
| **Renal function at enrollment [a]** | | | | |
| Renal impairment (n=57) | 5 | 8.8 | | |
| Normal renal function (n=914) | 12 | 1.3 | | |
| **Age** | | | | |
| <65 years (n = 604) | 6 | 1.0 | 0.37; 2.15 | NS [b] |
| 65 to 75 years (n = 250) | 5 | 2.0 | 0.65; 4.61 | |
| > 75 years (n = 117) | 6 | 5.1 | 1.90; 10.83 | 0.001 [c] |

(continued)

| | | | | |
|---|---|---|---|---|
| **Duration of surgery** | | | | |
| < 2 hours (n=634)<br>≥ 2 hours (n=215) | 7<br>10 | 0.9<br>4.7 | 0.37; 1.90<br>2.25; 8.39 | < 0.001 [d] |
| **Duration of Dosing** | | | | |
| 2 days (n = 648)<br>> 2 but ≤ 5 days (n = 323) | 10<br>7 | 1.5<br>2.2 | 0.74; 2,82<br>0.88; 4.41 | NS |
| **Dose Received** | | | | |
| Normal Dose (37.5 mg; n = 634)<br>High Dose (50 mg; n = 355) | 14<br>3 | 2.2<br>0.9 | 1.21; 3.68<br>0.19; 2.59 | NS |
| **Type of Surgery** | | | | |
| Orthopedic (n = 676)<br>Total Knee Replacement (n = 450)<br>Total Hip Replacement (n = 137)<br>Other Orthopedic (n = 89) | <br>13<br>2<br>2 | <br>2.9<br>1.5<br>2.2 | <br>1.55; 4.89<br>0.18; 5.17 | |
| | | | | |
| Abdominal / Pelvic (n = 293)<br>Gynecological / Genitourinary (n = 152)<br>Abdominal (n = 141) | 0<br>0<br>0 | 0<br>0<br>0 | | |
| [a] Serum creatinine >1.1 mg/dL (women) >1.3 mg/dL (men) or urine creatinine >300 mg/dL<br>[b] NS = non-significant differences between groups<br>[c] Versus subjects <65 years old<br>[d] Versus subjects undergoing surgical procedures <2 hours in duration | | | | |

Patients who developed acute renal; failure or decreased urinary output were evaluated by type of surgery and duration of surgery. None of the patients undergoing abdominal or pelvic procedures developed these acute events (Table 16). Patients undergoing orthopedic procedures were found to exhibit these events in 1.5-2.9% of cases. There was no overlap in confidence intervals suggesting a difference in hip, knee and other orthopedic procedures, but the variability of the data makes it difficult to draw any firm conclusions. To further examine these orthopedic patients, the mean time in surgery and pre-existing renal impairment were reviewed. Patients undergoing procedures ≥ 2 hours long were also at a significantly greater risk of experiencing acute renal failure or decreased urinary output AEs than those undergoing procedures <2 hours in duration (p<0.001; non-overlapping 95% confidence intervals; Table 16). The incidence of acute renal failure specifically was 2.4% (n=5/215) in patients who underwent procedures ≥2 hours long and 0.5% (n=3/634) in those who underwent shorter procedures. There were no significant differences in the incidences of these AEs based on duration of HPbCD diclofenac exposure (2 days versus 2-5 days) or the dose received (37.5 mg versus 50 mg/dose).

In the 17 patients experiencing acute renal failure or decreased urinary output, treatment consisted of the administration of fluids and diuretics and discontinuation of HPβCD diclofenac. None of the patients studied had to undergo dialysis as treatment for renal failure. Of the eight patients experiencing an episode of acute renal failure the median duration for this AE was 3 days, 18 hours (range 1 day, 4 hours to 9 days, 12 hours)

Of the 8 patients who experienced acute renal failure, 7 (88%) also experienced an episode of hypotension preceding the acute renal failure event. The remaining acute renal failure patient, who did not experience a preceding hypotensive event, was identified as having renal insufficiency prior to surgery and treatment with study drug. Acute renal failure occurred in 3.5% (2/57) of patients with elevated serum creatinine levels prior to surgery but only 0.7% (6/914) of those with normal pre-surgical serum creatinine levels. The median age of those experiencing acute renal failure was higher at 79.5 years old (60-84 years) versus those that experience a decrease in urinary output 60.0 years old (23-80 years). In total the median age of all patients studied that experienced a renal adverse event was 68 years old (22-84 years) and significantly higher than the median age of those patients that did not experience a renal adverse event at 58.5 years (18-87 years).

2.4 Patient Global Evaluation of Treatment Efficacy

Of the subjects completing the global efficacy evaluation, 932/958 (97.3%) indicated that their experience with the study medication was "Excellent," "Very good," or "Good," with 839 (86.4%) assessing their experience as "Excellent" or "Very good."

The primary purpose of this study was to examine a large, multicenter, repeated-dose, multiple-day trial to characterize the bleeding and renal adverse events that occurred in a large population of post -surgical patients receiving HPβCD diclofenac. The study was designed to include large at-risk cohorts, including the elderly (367 subjects ≥65 years old), those receiving concomitant anticoagulants (n=602 subjects), as well as individuals with pre-existing renal impairment (n=57). This study characterizes the safety profile of IV HPβCD diclofenac when administered every six hours following surgery for a 2 to 5 day post-operative recovery period.

The risk of unexpected severe postsurgical bleeding associated with the use of NSAIDs as postoperative analgesics is a well-recognized clinical concern. Because of this, the propensity of HPβCD diclofenac to cause such bleeding was analyzed in a variety of subpopulations with and without well-recognized and accepted risk factors for NSAID-induced complications. Few differences were observed between at-risk patient subpopulations with respect to bleeding-related AEs other than anemia. In total, 3.3% of all study subjects reported a bleeding-related AE. In addition, mild to moderate postoperative anemia was reported in 22.5% of all participants. Anemic events, however, were not considered to be treatment-related in any patient. With respect to at-risk patient groups in this study, the incidence of clinically-significant bleeding-related AEs such as GI bleeding, anastomotic hemorrhage, post operative bleeding from the surgical incision site, hematoma, hematemesis, hematuria and others was significantly elevated only in patients undergoing abdominal surgical procedures (laparotomy, incision, excision, and anastomosis of the intestine, as well as operations of the gallbladder, biliary tract, and pancreas), when compared to those undergoing gynecologic or genitourinary procedures (mostly open hysterectomies). The greater incidence of bleeding-related events with abdominal versus gynecologic/genitourinary procedures is in agreement with previous evidence indicating increased incidence of bleeding events with organ and general surgical procedures versus those surgical procedures involving the reproductive organs (Stokes et al., BMC Health Serv Res (2011); 11:135).

Importantly, no statistically significant differences in the incidence of bleeding-related AEs occurred between those patients receiving HPβCD diclofenac in addition to anticoagulants for DVT prophylaxis versus those not receiving anticoagulants. Likewise, no differences were observed when patients ≥ 65 years old and < 65 years old, or patients receiving two days of treatment versus those receiving up to 5 days of continuous treatment with HPβCD diclofenac, were compared.

Although the post-surgical population is already at s greater risk for acute renal failure and other renal adverse events due to fluid shifts, compromised hemodynamic balance, and possibly impaired renal function, multiple-dose IV HPβCD diclofenac was associated with a relatively low incidence of postoperative renal failure in this trial. A total of 8 reports of acute renal failure (n=8/971, (0.8%)) occurred in this study which is consistent with the 1% incidence reported in national surgical data sets for postoperative acute renal failure in all post surgical patients, not just those also receiving postoperative IV or oral NSAIDs (Ghaferi et al., New Engl J Med (2009);1361:1368-1375; Kheterpal et al., Anesthesiology (2009);110:505-515).

It is important to note that this trial did demonstrate an increase in the incidence of decreased urinary output and acute renal failure in patients >75 years of age (5.1% (n=6/604)) versus those < 65 years old (1.0% (6/604) or those 65-75 years old (2.0%, (5/250)). There was no statistical difference in the incidence of these AEs when patients 65-75 and < 65 years of age were compared. Acute renal failure occurred in 4.3% (5/117) of those patients aged greater than 75 years (range 76 - 85 years of age). However, these numbers are small and unable to detect a clinically or statistically meaningful signal. In addition, patients undergoing surgical procedures >2 hours in duration had a greater incidence of acute renal failure or decreased urinary output AEs than those undergoing procedures <2 hours in total length. In those undergoing procedures >2 hours long, the incidence of acute renal failure was 2.4% (n=5/215), versus only 0.5% (n=3/634) in those undergoing shorter surgical procedures. This increase in renal adverse events may reflect the likelihood of more extensive fluid shifts, and corresponding periods of hypotension that tend to occur with more extensive procedures lasting a longer period.

Finally, patients with serum creatinine levels greater than normal prior to surgery also experienced a significantly greater incidence of renal adverse events in this trial than those with normal serum creatinine levels, and acute renal failure occurred in 3.5% of patients with elevated serum creatinine levels prior to surgery, but only 0.7% of those with normal pre-surgical serum creatinine levels.

With respect to duration of treatment, continuous treatment with HPβCD diclofenac for 2-5 days did not result in a significantly greater increase in renal AEs, as compared to incidence in patientstreated for only two days.

In conclusion, this study demonstrated that 37.5 mg IV HPβCD diclofenac was safe and well-tolerated in a large patient population with few exclusion criteria that was reflective of the types of postsurgical patients treated in clinical practice. In addition, this study revealed a similar safety profile with the 50 mg dose, which was given to patients

of >95 kg weight. Overall, the safety profile of IV HPβCD diclofenac established herein, as well as its analgesic efficacy, as demonstrated in previous studies (Christensen et al., Anesth Prog (2011);58:73-81; Leeson et al., Reg Anesth Pain Med (2007);32:303-310) indicates that this new diclofenac formulation can serve as an important new role in the management of postoperative pain.

**EXAMPLE 13: The pharmacokinetics (PK) of HBβPD-Diclofenac, a novel parenteral Diclofenac formulation, in special populations.**

**I. Methods and Subjects**

*1. Subjects:*

There were 128 total participants in the two studies (Table 17). Study 1 was conducted at two sites, in Baltimore, MD and Miramar, Florida. Study 2 was conducted at four sites: Minneapolis, Minnesota; Knoxville, Tennessee; Orlando, Florida; and Mid Glamorgan, UK.

**Table 17. Demographic characteristics of participants**

| Study | Description | Dose, route | No. of subjects | Age range | Mean Weight, kg (SD) | Mean BMI, kg/m2, (SD) | Male/female |
|---|---|---|---|---|---|---|---|
| **1** | **Effect of age, weight and BMI on pharmacokin etics of HPβCD-diclofenac** | **Weight cohort:** 37.5 mg HPβCD-diclofenac, IV | **Weight cohort:** 54 | **Weight cohort** 18-54 | **Weight cohort:**<br>Underweight: 52.00 (7.16)<br>Small: 54.27 (3.79)<br>Large: 81.53 (10.29) | **Weight cohort:**<br>Underweight: 18.00 (0.628)<br>Small: 21.19 (1.769)<br>Large: 27.08 (2.013)<br>Obese: 34.21 (2.367) | **Weight cohort:** 23/31 |
| | | **Age cohort:** 18.75 mg HPβCD-diclofenac, IV | **Age cohort:** 34 | **Age cohort:** 55-82 | Obese: 99.14 (11.13)<br>Extremely Obese: 126.82 (20.64)<br>**Age cohort:**<br>55 ≤ Age < 65 years: 68.65 (9.77)<br>65 ≤ Age < 75 years: 67.01 (8.93)<br>Age ≥ 75 years: 72.20 (6.42) | Extremely Obese: 43.66 (2.472)<br>**Age cohort:**<br>55 ≤ Age < 65 years: 25.95 (2.804)<br>65 ≤ Age < 75 years: 26.32 (2.422)<br>Age ≥ 75 years: 27.78 (1.774) | **Age cohort:** 13/21 |
| **2** | **Pharmacoki netics of HPβCD-diclofenac in patients with renal and hepatic insufficiency** | 37.5 mg HPβCD-diclofenac, IV 200 mg itraconazole, IV | Renal: 13 | Renal: 50-75 | Renal: 79.8 (20.2) | Renal: 28.3 (5.4) | Renal: 5/8 |
| | | | Hepatic: 8 | Hepatic: 40-61 | Hepatic: 76.4 (12.2) | Hepatic: 25.1 (4.4) | Hepatic: 8/0 |
| | | | Healthy: 19 | Healthy: 33-65 | Healthy: 74.9 (10.0) | Healthy: 25.5 (3.0) | Hepatic: 13/6 |

*2. Procedure*

2.1 Study 1 - Effect of age, weight / BMI on pharmacokinetics of HPβCD-diclofenac

2.1.1 Subject Criteria

This open-label, single dose study assessed the effects of age, weight, and body composition on the pharmacokinetics, safety, and tolerability of HPβCD-diclofenac in adult volunteers. This study was conducted in two cohorts: weight-based and age-based. Subjects enrolling into the weight-based cohort were required to have a BMI greater than or equal to 15 kg/$m^2$, and body weight in the range of 40-159 kg (88-350 pounds). Subjects enrolling into the age-based cohort were required to be 55 years of age or older, and have a BMI 19 or more, but less than 30 kg/$m^2$, and body weight between 45 kg and 95 kg (99-209 pounds). All volunteers were required to be non-smokers, in good general health and able to communicate with study personnel. Pre-menopausal female volunteers were required to have a negative pregnancy test, be non-lactating, and practice an approved form of contraception. Subjects were excluded if they had any significant medical history or clinically relevant laboratory test results; were serologically positive for the human immunodeficiency virus, hepatitis B virus, or hepatitis C virus; had hypersensitivity to NSAIDs or diclofenac; or were substance abusers.

Because it was anticipated that these populations might have health problems, it was not possible to restrict this study to healthy subjects. Restrictions were placed on certain disease states, such as current intestinal disorders or infections, peptic ulcers, GI bleeding or cerebral hemorrhage, and specific previous surgeries, such as bariatric surgery and bowel resection. Only specified concomitant medications resulted in exclusion from the study, such as appetite suppressants or herbal medications for subjects in the weight-based cohort. However, volunteers with a history of cardiovascular events, diabetes, high blood pressure, and/or hypercholesterolemia were allowed to enroll in the study, providing that the investigator judged that it would not put their health at risk.

2.1.2. Procedure

Subjects in the weight-based cohort were stratified into five groups: Underweight: weight not defined, BMI $\geq$ 15 and $\leq$ 18.9 kg/m2; Small: weight $\geq$ 45 and < 60 kg, BMI $\geq$ 19 and $\leq$ 24.9 kg/$m^2$; Large: weight $\geq$ 60 and < 100 kg, BMI $\geq$ 19 and $\leq$ 30 kg/$m^2$; Obese: weight not defined, BMI $\geq$ 30 and $\leq$ 40 kg/$m^2$; Extremely Obese: weight not defined, BMI > 40 kg/$m^2$. Subjects in the age-based cohort were stratified into 3 groups: 55 $\leq$ Age < 65 years, 65 $\leq$ Age < 75 years, and Age $\geq$ 75 years (as shown in Figure 15). Figure 15 represents the distribution of patients assigned to age and weight cohorts in Study 1. A total of 88 patients were assigned to a cohort. All 88 patients (100%) patients completed the study.

Subjects were fasted overnight before a single dose of IV HPβCD-diclofenac was administered in the morning. Participants later received a standardized breakfast, lunch and dinner at the study site. Subjects in the weight-based cohort received an IV bolus of HPβCD-diclofenac 37.5 mg. Subjects in the age-based cohort received an IV bolus of HPβCD-diclofenac 18.75 mg. Blood samples for PK analysis were obtained via an indwelling IV cannula or by direct venipuncture at the following time points: Time 0 (pre-dose), 5, 10, 20, 30, and 45 minutes and 1, 1.5, 2, 2.5, 3, 4, 6, 8, 10, 12, and 18 hours post-dose. For samples from an indwelling catheter, the first 2 mL of blood withdrawn were drawn into a separate syringe and discarded to clear saline diluent in tubing dead space and the last 3 mL were used for analysis.

2.2 Study 2 - Pharmacokinetics of HPβCD-diclofenac in patients with renal and hepatic insufficiency

2.2.1 Subject Criteria

Study 2 consisted of an open-label, single-dose study of the pharmacokinetics of HPβCD-diclofenac and its excipient HPBCD in subjects with renal or hepatic impairment, and a randomized, open-label, single-dose, two-way, crossover study of the pharmacokinetics of HPβCD-diclofenac and of HPBCD used in another approved drug in healthy volunteers. Subjects with stable mild chronic renal insufficiency (defined as creatinine clearance (CrCl) $\geq$ 50 and $\leq$ 80 mL/min) or stable moderate chronic renal insufficiency (CrCl $\geq$ 30 and < 50 mL/min) were recruited. CrCl for healthy subjects and subjects with mild or moderate chronic renal insufficiency was estimated using the following formula: Glomerular filtration rate (GFRmL/min/1.73 m2) =186 x serum creatinine (SCr)-1.154 x (age)-0.203 x (0.742 if female) x (1.212 if African American) (Levey et al., Annals of internal medicine. (1999);130(6):461-470). Subjects with mild chronic hepatic impairment, as defined by Child-Pugh Classification A, Score of 5 to 6 and a bilirubin of $\leq$ 2.5 mg/dl, were recruited. Subjects with renal or hepatic impairment were included if they were between 18-75 years of age, and healthy subjects between 18 and 65 years of age.

Inclusion criteria for healthy subjects were similar to those for Study 1. Healthy subjects were required to have normal renal function, defined as CrCl > 80 mL/min, and normal hepatic function. The healthy adult subjects were

matched by age (±10 years), gender, and body weight (±10 kg) to the subjects with mild chronic renal insufficiency and the subjects with mild chronic hepatic impairment.

Similar to Study 1, there were allowances for a greater breadth of co-morbid conditions in the renal and hepatic groups. Subjects were allowed to enroll if they had a history of cardiovascular events, diabetes, high blood pressure, and/or hypercholesterolemia providing these conditions were stable, well-controlled and did not pose a significant safety risk, as determined by medical history, physical examination, electrocardiogram, and clinical laboratory evaluations (complete blood count (CBC), chemistry, platelet function, urinalysis, hepatitis B surface antigen, HIV antibodies, hepatitis C antibodies, breath alcohol, and urine drug screen tests). Diabetic patients must have been on a stable therapeutic regimen for four weeks.

Exclusion criteria included pregnancy, uncontrolled or poorly controlled diabetes, use of dialysis, fluctuating or rapidly deteriorating hepatic function, hepatic or other cancers, organ transplantation or immunosuppression, acute infections, or asthma. Subjects were excluded for any serious cardiovascular events, such as myocardial infarction, coronary artery bypass surgery or percutaneous coronary intervention, unstable angina, stroke or congestive heart failure. Restrictions were also placed on certain disease states, such as current intestinal disorders or infections, peptic ulcers, GI bleeding or cerebral hemorrhage. Subjects who were positive for hepatitis B or hepatitis C were excluded from the healthy and renal groups, but were included in the subjects with mild chronic hepatic impairment. History and current use of other prescription or OTC drugs was monitored, evaluated for potential interference, and may have resulted in exclusion, depending on the medication. Hypersensitivity to NSAIDs or diclofenac also prompted exclusion.

2.2.2 Procedure

Subjects with renal or hepatic impairment reported to the study site on study day 0 and remained at the site for two nights and two days. HPβCD-diclofenac 37.5 mg was given to each subject as an IV bolus injection over 15 seconds on study day one. Blood samples were obtained via an indwelling IV cannula or by direct venipuncture at the following times: Time 0 (pre-dose), 5, 10, 20, 30, and 45 minutes; 1, 1.5, 2, 2.5, 3, 4, 6, 8, 10, 12, 18, and 24 hours post dose. Subjects were discharged on study day 2 after being assessed by the investigator, and returned 7 ± 3 days after dosing for final safety assessments. If there were no abnormal findings at discharge, the follow-up visit was completed via telephone.

Healthy subjects reported to the study site on study day 0 and remained at the site for three nights and three days. HPβCD-diclofenac 37.5 mg was given as an IV bolus over 15 seconds, and the comparator, an approved antifungal drug solubilized with HPβCD (Sporanox, itraconazole for injection, 200 mg) was given as an IV infusion over 60 minutes) were administered on study day 1 and study day 2 according to randomization codes. Blood samples were obtained as described above for renal and hepatic subjects. When the subjects received HPβCD-diclofenac, two blood samples were drawn at each time point for diclofenac and HPβCD concentration measurements. For itraconazole, one blood sample was to be drawn at each time point to assay for HPβCD concentrations. Subjects were discharged on study day 3 after being assessed by the investigator, and returned 7 ± 3 days after receiving the last dose of study drug for safety assessments.

*3. Pharmacokinetic and statistical analysis*

Diclofenac plasma concentrations were measured by liquid chromatography with validated tandem mass spectrometry detection (LC-MS-MS) (limit of quantitation (LOQ) curve range 5-2000 ng/ml) performed by CEDRA Clinical Research, LLC (8609 Cross Park Drive, Austin, Texas 78754). Plasma concentrations of HPβCD were determined using a validated assay by Eurofins Medinet (12635 East Montview Blvd., Suite 214, Aurora, Colorado 80045). The LOQ for the HPβCD assay was 100 ng/ml.

Pharmacokinetic parameters were calculated using non-compartmental analysis. Only those plasma concentrations equal to or greater than the lower limit of quantitation were used in the analysis. Actual sampling times were used in all pharmacokinetic analyses. Per protocol times were used to calculate mean plasma concentrations for graphical displays.

The maximum serum concentration ($C_{max}$) and time to $C_{max}$ ($T_{max}$) were taken directly from the data. The elimination rate constant, λz, was calculated as the negative of the slope of the terminal log-linear segment of the serum concentration-time curve. The range of data used for each subject and treatment was determined by visual inspection of a semi-logarithmic plot of concentration vs. time. Elimination half-life (t½) was calculated according to the following equation;

$$t_{1/2} = 0.693/\lambda z$$

Area under the curve from zero to the final sample with a concentration ≥: LOQ (AUC(0-τ)) was calculated using the linear trapezoidal method and extrapolated to infinity using:

$$AUC\infty = AUC(0\text{-}\tau) + Ctf/\lambda z,$$

where Ctf is the final concentration ≥: LOQ. Total plasma clearance (CL) was calculated as Dose/ AUC, and volume of distribution (Vz) was calculated as Dose/λz AUC.

For Study 1, the potential relationships between the independent PK parameters CL and Vz and the dependent parameter t½ and the demographic variables age, total body weight, and BMI were examined using linear regressions. Since age, body weight, and BMI are continuous variables, data from the weight and age cohorts were combined for these analyses.

For Study 2, glomerular filtration rate (GFR), in mL/min/1.73 $m^2$, was estimated for subjects with mild or moderate renal impairment and healthy controls using the Modification of Diet in Renal Disease formula:

$$GFR = 186 \text{ x } Scr^{-1.154} \text{ x } Age^{-0.203} \text{ x } (0.742 \text{ if female}) \text{ x } (1.212 \text{ if African American})$$

where Scr is serum creatinine in mg/dL. The effect of renal impairment on the pharmacokinetics of diclofenac and HPβCD on the parameters $C_{max}$, AUC∞ , CL, Vz, and t½ was examined using an analysis of variance statistical model (ANOVA) with subject type as the classification variable using the natural logarithms of the data. Comparisons among the three subject types were done using paired t-tests. The same model was used to test for the effect of hepatic impairment on the pharmacokinetics of diclofenac and HPβCD but without additional comparisons, as there were only two groups. Potential relationships between the independent pharmacokinetic parameters CL and Vz and the dependent parameter t1/2 and renal function were examined using linear regressions of each pharmacokinetic parameter against GFR.

Comparison of the pharmacokinetic parameters Cmax, AUC(0-t), and AUC∞ for HPβCD between HPβCD-diclofenac (test) and itraconozole (reference) was done using an ANOVA model with sequence, subject within sequence, treatment, and period as the classification variables, using the natural logarithms of the data. Confidence intervals (90%) were constructed for the ratio, test to-reference, of the three parameters using the log-transformed data and the two one-sided t-tests procedure. The point estimates and confidence limits were exponentiated back to the original scale. All pharmacokinetic calculations were done and individual subject plasma concentration vs. time graphs were prepared using SAS® for Windows® Version 9.1.3.

## II. Results

*1. Study 1 - Effect of age, weight / BMI on pharmacokinetics of HPβCD-diclofenac*

All 88 subjects who enrolled in the age and weight cohorts and were randomized completed the studies. Demographic characteristics of subjects are detailed in Table 17.

Pharmacokinetic cohort studies in 34 subjects showed no effect of age (55-82 years) on the exposure of IV HPβCD-diclofenac (Table 18, and as shown in Figure 16A). Figure 16A shows the mean plasma concentrations over time of diclofenac after IV administration of 18.75 mg of HPβCD-diclofenac to three age-based cohorts. Examination of the relationships between CL, Vz, and t½ and age indicated significant inverse relationships between the volume of distribution (Vz) and age ($p = 0.0183$), and t½ and age ($p = 0.0480$) (Figures 17A-B). Figures 17A-B show the relationships between age, and diclofenac PK parameters. Figure 17A shows the relationship between volume of distribution and age after intravenous administration of 18.75 mg or 37.5 mg of HPβCD-diclofenac. Figure 17B shows the relationship between terminal elimination half-life and age after intravenous administration of 18.75 mg or 37.5 mg of HPβCD-diclofenac. There was a significant decrease in Vz with age, which resulted in a significant inverse relationship between t½ and age. In a pooled regression of both age and weight cohorts, the clearance of diclofenac did not appear to be affected by age ($p= 0.1779$).

Mean values for PK parameters showed a decrease in exposure with increasing body weight (44-162 kg) (Table 18, and as shown in Figure 16B). Figure 16B shows the mean plasma concentrations over time of diclofenac after IV administration of 37.5 mg of HPβCD-diclofenac to five weight-based cohorts. Examination of the relationships between CL, Vz, and t½ and total body weight and BMI indicated significant relationships between CL and weight ($p < 0.0001$), Vz and weight ($p<0.0001$). Relationships with BMI were consistent with those for weight, a major component of BMI.

There were essentially no relationships between t½ and either total body weight or BMI, with p-values of 0.4872 and 0.8384, respectively, due to proportional increases in both CL and Vz with increased body weight, resulting in no change, in their ratio, which determines t½.

## Table 18. Pharmacokinetic parameters measured in studies

| Parameter[1] | $C_{max}$, ng/mL ± SD (n) | $T_{max}$, h (n) | AUC(0-t), h x ng/mL ± SD (n) | AUC∞, h x ng/mL ± SD (n) | t½, h ± SD (n) | λz, $h^{-1}$ ± SD (n) | CL, mL/min ± SD (n) | Vz, L ± SD (n) |
|---|---|---|---|---|---|---|---|---|
| Study 1 | | | | | | | | |
| 55 ≤ Age < 65 yrs | 3,439 ± 855 (12) | 0.083 (12) | 1,087 ± 288 (12) | 1,126 ± 291 (11) | 1.39 ± 0.43 (11) | 0.5437 ± 0.1728 (11) | 274 ± 70.6 (11) | 32.7 ± 13.3 (11) |
| 65 ≤ Age < 75 yrs | 3,465 ± 738 (13) | 0.083 (13) | 1,143 ± 261 (13) | 1,178 ± 263 (12) | 1.42 ± 0.34 (12) | 0.5165 ± 0.1351 (12) | 257 ± 52.2 (12) | 31.5 ± 9.26 (12) |
| Age ≥ 75 yrs | 3,257 ± 750 (3) | 0.083 (3) | 1,200 ± 95.9 (3) | 1,220 ± 96.8 (3) | 2.14 ± 0.61 (3) | 0.3438 ± 0.1035 (3) | 239 ± 19.8 (3) | 44.3 ± 13.1 (3) |
| Underweight: | 6,594 ± 2,258 (5) | 0.083 (5) | 2,190 ± 609 (5) | 2,103 ± 651 (4) | 2.03 ± 0.47 (4) | 0.3593 ± 0.1011 (4) | 297 ± 92.4 (4) | 50.4 ± 14.0 (4) |
| Small: | 8,212 ± 1,952 (11) | 0.083 (11) | 2,413 ± 616 (11) | 2,429 ± 616 (11) | 1.67 ± 0.34 (11) | 0.4321 ± 0.0962 (11) | 255 ± 71.4 (11) | 36.1 ± 10.1 (11) |
| Large | 5,903 ± 1,060 (16) | 0.083 (16) | 1,916 ± 411 (16) | 1,933 ± 412 (16) | 1.79 ± 0.44 (16) | 0.4095 ± 0.0997 (16) | 314 ± 69.3 (16) | 47.9 ± 13.6 (16) |
| Obese | 5,103 ± 0,672 (13) | 0.083 (13) | 1,740 ± 265 (13) | 1,757 ± 266 (13) | 1.56 ± 0.25 (13) | 0.4568 ± 0.0847 (13) | 338 ± 53.0 (13) | 45.5 ± 9.60 (13) |
| Extremely Obese | 4,616 ± 1,639 (8) | 0.083 (8) | 1,569 ± 316 (8) | 1,640 ± 302 (7) | 1.81 ± 0.57 (7) | 0.4205 ± 0.1437 (7) | 363 ± 56.0 (7) | 56.4 ± 19.0 (7) |
| Study 2 | | | | | | | | |
| Diclofenac | | | | | | | | |
| Mild Renal | 7,286 ± 1,430 (8) | 0.083 (8) | 1,927 ± 409 (8) | 1,943 ± 409 (8) | 1.89 ± 0.46 (8) | 0.3856 ± 0.088 (8) | 312 ± 73.0 (8) | 49.8 ± 12.1 (8) |
| Moderate renal | 5,332 ± 1,629 (5) | 0.083 (5) | 1,531 ± 418 (5) | 1,550 ± 422 (5) | 2.10 ± 0.44 (5) | 0.3427 ± 0.080 (5) | 401 ± 126 (5) | 69.7 ± 9.22 (5) |
| Healthy matches | 7,163 ± 950 (7) | 0.083 (7) | 1,947 ± 313 (7) | 1,968 ± 315 (7) | 1.90 ± 0.30 (7) | 0.3725 ± 0.055 (7) | 303 ± 55.6 (7) | 50.2 ± 14.1 (7) |
| Mild Hepatic | 5,648 ± 709 (8) | 0.083 (8) | 1,641 ± 179 (8) | 1,663 ± 179 (8) | 1.97 ± 0.67 (8) | 0.3793 ± 0.098 (8) | 353 ± 40.7 (8) | 60.1 ± 21.5 (8) |
| Healthy matches | 5,884 ± 897 (7) | 0.083 (7) | 1,618 ± 333 (7) | 1,640 ± 335 (7) | 1.92 ± 0.28 (7) | 0.3678 ± 0.050 (7) | 367 ±74.7 (7) | 59.9 ± 9.4 (7) |
| HPβCD | | | | | | | | |
| Mild Renal | 60,750 ± 16,275 (8) | 0.083 (8) | 127,141 ± 90,489 (8) | 128,349 ± 91,132 (8) | 2.87 ± 0.69 (8) | 0.2549 ± 0.068 (8) | 59.0 ± 31.3 (8) | 13.6 ± 5.38 (8) |
| Moderate renal | 52,700 ± 18,565 (5) | 0.083 (5) | 169,042 ± 52,722 (5) | 165,728 ± 60,386 (4) | 6.04 ± 1.94 (4) | 0.1226 ± 0.033 (4) | 36.2 ± 10.0 (4) | 17.7 ± 1.88 (4) |
| Healthy matches | 50,329 ± 7,731 (7) | 0.083 (7) | 66,449 ±12,642 (7) | 67,316 ± 12,615 (7) | 3.29 ±1.66 (7) | 0.2510 ± 0.095 (7) | 85.2 ± 16.5 (7) | 23.3 ± 9.84 (7) |
| | | | | | | | | |
| Mild Hepatic | 44,813 ± 14,985 (8) | 0.083 (8) | 55,946 ± 17,233 (8) | 56,802 ±17,412 (8) | 2.28 ± 0.60 (8) | 0.3226 ± 0.084 (8) | 107 ± 33.8 (8) | 20.0 ± 4.19 (8) |
| Healthy matches | 40,917 ± 4,975 (7) | 0.083 (7) | 52,982 ± 11,267 (7) | 53,651 ± 11,321 (7) | 2.28 ± 0.42 (7) | 0.3127 ± 0.052 (7) | 107 ± 21.2 (7) | 20.6 ± 2.45 (7) |
| | | | | | | | | |
| Healthy-HPβCD-diclofenac | 44,331 ± 10,004 (13) | 0.083 (13) | 58,994 ± 14,123 (13) | 59,709 ± 14,217 1 (13) | 2.74 ± 1.35 (13) | 0.2909 ± 0.0860 (13) | 98.0 ± 22.7 (13) | 21.8 ± 7.36 (13) |
| Healthy - itraconazole | 557,538 ± 105,477 (13) | 1.083 (13) | 1,300,356 ± 264,445 (13) | 301,283 ± 264,630 (13) | 2.54 ± 0.25 (13) | 0.2752 ± 0.0244 (13) | 106 ± 19.0 (13) | 23.5 ± 5.65 (13) |

$C_{max}$ = Maximum observed plasma concentration; $T_{max}$ = Time at which $C_{max}$ is observed; AUC(0-t) = AUC up to the last quantifiable concentration; AUC∞ = AUC from time zero to infinite time; λz = Terminal elimination rate constant; t½= Apparent elimination half-life; Vz = Volume of distribution; CL = Clearance

[1]Arithmetic mean ± standard deviation except for $T_{max}$ for which the median is reported.

One subject in the weight-based cohort and six subjects in the age-based cohort had plasma concentrations

of HPβCD-diclofenac that were considered aberrant. The PK analyses were performed both including and excluding these subjects. The overall conclusions of the study and relationship between the PK parameters CL, t½, and Vz, and weight or age did not change as a result of including subjects with apparent aberrant plasma concentrations. The PK results presented here have excluded these subjects.

*1.1 Safety*

There were no deaths, no serious adverse events (AEs), and no AEs that led to discontinuation from the study in either the weight-based or the age-based cohort during this study. Nine subjects (16.7%) in the weight-based cohort reported 14 treatment-emergent AEs. None of the subjects in Treatment Groups A (Underweight) and E (Extremely Obese) experienced a treatment emergent AE during the study. All events were considered to be mild and transient, resolved without medical intervention, and included gastrointestinal events, administration site conditions and headache. Eight of 14 events (57.1%) were considered unrelated to study drug.

Three subjects (8.8%) in the age based cohort reported a total of 13 treatment emergent AEs. All events were considered to be mild and transient, and all but 2 resolved without medical intervention. Mild events included constipation, administration site conditions, increased blood amylase, increased lipase, pruritus, and hypertension. Two events, pruritus and hypertension required medication. Approximately half (54.5%) were considered by the PI to be unrelated to study drug.

*2. Study 2 - Pharmacokinetics of HPβCD-diclofenac in patients with renal and hepatic insufficiency*

A total of 13 subjects with renal insufficiency (mean CrCl 56 mL/min), 8 subjects with hepatic impairment (mean ALT 52.4 IU/l, mean AST 43.6 IU/l, mean bilirubin 0.59 mg/dl, Child-Pugh Score 5.5), and 14 matching healthy subjects completed the study successfully (Table 18). Demographic characteristics of subjects are detailed in Table 17.

The overall exposures for HPβCD-diclofenac, as measured by AUC(0-τ) and AUC∞, did not differ significantly between groups with mild renal impairment, moderate renal impairment and healthy matched controls (Table 18, as shown in Figure 18A). Figure 18A shows the mean plasma concentrations over time of diclofenac after IV administration of 37.5 mg of HPβCD-diclofenac to subjects with mild or moderate renal impairment and to healthy subjects. The elimination rate of diclofenac did not differ significantly as a function of the degree of renal insufficiency. There was a statistically insignificant trend toward an increase in the CL and Vz of diclofenac in subjects with moderate renal insufficiency ($30 \leq$ CrCl $< 50$ mL/min) compared to those with mild renal insufficiency ($50 \leq$ CrCl $\leq 80$ mL/min) and matched healthy controls (CrCl > 80 mL/min) (Table 18), however, this may be an artifact of the small sample sizes. The elimination rate of the excipient, HPβCD, varied with the degree of renal insufficiency Figure 18B. Figure 18B shows the mean plasma concentrations over time of the excipient, HPβCD, after IV administration of 37.5 mg of HPβCD-diclofenac to subjects with mild or moderate renal impairment and to healthy subjects. There was a decrease in the CL of HPβCD, with corresponding increases in AUC∞ and t½ with decreased renal function. A 2.4-fold decrease in CL and a 1.8-fold increase in t½ were observed in subjects with moderate renal insufficiency when compared to healthy subjects (Table 18).

There were no differences in the PK of diclofenac or HPβCD in subjects with mild hepatic impairment versus matched healthy controls (Table 18, and as shown in Figure 19A). Figure 19A shows the mean plasma concentrations over time of diclofenac after IV administration of 37.5 mg of HPβCD-diclofenac to subjects with mild hepatic impairment and to healthy subjects.

Following administration of HPβCD-diclofenac, exposure to HPβCD was markedly reduced compared to itraconazole in healthy controls (as shown in Figure 19B). Figure 18B shows the mean plasma concentrations over time of the excipient, HPβCD, after IV administration of 37.5 mg of HPβCD-diclofenac containing 333.3 mg of HPβCD and itraconazole 200 mg containing 8000 mg of HPβCD to healthy subjects. Exposure was equal to $1/20^{th}$ that of itraconazole after a single dose and $1/8^{th}$ after steady state. Exposure to HPβCD, based on the geometric least squares mean ratio of AUC∞, after administration of 333.3 mg as HPβCD-diclofenac was 4.58% of that after administration of 8000 mg as Sporanox, essentially the same as the ratio of doses (4.17%). For subjects with moderate renal insufficiency, the exposure to HPβCD after a 37.5 mg dose of HPβCD-diclofenac would still be 7.9-fold lower based on AUC(0-τ), and 3.9-fold lower based on the average concentration at steady state ($C_{av}$), than in healthy subjects administered itraconazole.

2.1 Safety

There were no deaths, withdrawals due to AEs, or serious adverse events (SAEs) in this study evaluating the effect of renal or hepatic impairment on HPβCD-diclofenac pharmacokinetics. There were no severe AEs and all reported events were mild or moderate in intensity. The overall incidence and severity of treatment-emergent AEs was similarly low in all cohorts. Following HPβCD-diclofenac administration, two subjects (15.4%) with renal insufficiency, and one subject (12.5%) with mild hepatic impairment were recorded as having drug-related AEs and no drug-related AEs were

reported by healthy subjects. There were no reports of adverse hepatic or renal AEs in the patients with renal or hepatic impairment. No clinically significant study drug effects were observed for clinical chemistry or hematology parameters or for renal function or liver function tests. No clinically significant out-of-range vital signs or ECG results were observed during the study.

This study demonstrated that advanced age had no effect on the total exposure of IV HPβCD-diclofenac, while increasing weight was associated with decreased exposure. Renal or hepatic insufficiency did not affect the exposure or elimination of diclofenac, however renal insufficiency did decrease the clearance of the excipient HPβCD.

This study demonstrated that patient age does not affect the overall exposure of HPβCD-diclofenac. However, age-related changes in body water and body fat act to reduce the volume of distribution of drugs (Vz) in older patients. A significant decrease in Vz with increased age, which resulted in a decrease in t1/2 with increased age, was observed. This suggests that there would be a decreased risk of accumulation of HPβCD-diclofenac in elderly subjects. Therefore, modification of the dosing regimen of HPβCD-diclofenac should not be necessary for elderly subjects solely due to pharmacokinetic changes. However, NSAID dosages are typically reduced in elderly patients because they are particularly susceptible to NSAID side effects, such as bleeding, renal, and cardiovascular effects (Aubrun et al., Clinical anaesthesiology (2007);21(1):109-127). For example, ketorolac, although effective when given by IV bolus, interferes with platelet aggregation (Bauer et al., Journal of clinical anesthesia. (2010);22(7):510-518), and increases the risk of bleeding (Elia et al., Anesthesiology. (2005);103(6):1296-1304) to such extents that dosage reductions are mandatory in at-risk populations such as the elderly (Physicians' Desk Reference. 2012). However, HPβCD-diclofenac, with its balanced COX-1 and COX-2 inhibitory profile, may pose less risk of postoperative bleeding than NSAIDs such as ketorolac and aspirin, which predominantly inhibit COX-1. In a study of the platelet function of human volunteers, HPβCD-diclofenac affected clotting ability to a lesser extent than the predominantly COX-1 inhibiting NSAIDs, ketorolac and aspirin (Bauer et al., Journal of clinical anesthesia. (2010);22(7):510-518).

Consistent with the guidelines stated above, elderly patients in this study were given 18.75 mg, half of the standard dose. Other studies have demonstrated the comparable efficacy of the 18.75 mg dose, for example, the studies shown in Examples 1 and 2 above. Therefore, the safety and tolerability of HPβCD-diclofenac in elderly patients in this study and of diclofenac in others (Dilger et al., Journal of clinical pharmacology. (2002);42(9):985-994; Fredman et al., Anesthesia and analgesia. (1999);88(1):149-154; Bakshi et al., Current medical research and opinion. (1991);12(7):459-465) indicates that the analgesic and opioid-sparing benefits of this well-understood NSAID may be offered to such patients without the incremental risk that is sometimes associated with pharmacotherapy in the elderly.

Patient weight does affect the kinetics of HPβCD-diclofenac, showing a decrease in exposure with increasing body weight. There was a significant increase in CL with increased body weight, suggesting that maintaining a "standard" exposure (AUC) by varying dose in higher-weight subjects should be considered to maintain equivalent exposure and pain relief. For patients of all ages and those weighing 40-95 kg, a uniform dose offers the advantages of simplifying treatment, reducing errors, and saving time spent on dose calculations.

The elimination rate of diclofenac did not differ significantly as a function of the degree of renal insufficiency. This is consistent with studies of other diclofenac formulations, in which renal elimination was not found to be a significant pathway for clearance (Brogden et al., Drugs. (1980);20(1):24-48). However, subjects with renal insufficiency showed decreased clearance of HPβCD. The magnitude of the reduction was such that blood concentrations of HPβCD after therapeutic doses of HPβCD-diclofenac would remain well below those seen with IV itraconazole, a currently marketed antifungal product that is also solubilized with HPβCD. HPβCD does not seem to provide any additive or synergistic effect on the pharmacokinetics of diclofenac in patients with renal insufficiency, indicating that exposure to HPβCD in HPβCD-diclofenac poses no risk to patients with mild or moderate renal insufficiency.

Hepatic impairment can affect drug metabolism, so it was interesting that no differences in the PK profile of diclofenac or HPβCD in subjects with mild hepatic impairment compared to matched healthy controls were observed. Hepatic metabolism accounts for almost 100% of diclofenac elimination, while HPβCD is not extensively metabolized and 80% to 90% of the IV dose is excreted unchanged in the urine (Brewster et al., Advanced drug delivery reviews. (2007);59(7):645-666). There was no evidence to support an additive or synergistic effect of HPβCD on the pharmacokinetics in patients with hepatic impairment. HPβCD-diclofenac was safe and well tolerated in subjects with mild hepatic impairment with no notable aggravation of underlying disease or marked elevations in liver function tests. Therefore, no dose adjustment is recommended in this patient population.

HPβCD-diclofenac may have distinct advantages over other formulations of diclofenac. HPβCD-diclofenac employs HPβCD to enhance the solubility of diclofenac (Gould et al., Food and chemical toxicology: an international journal published for the British Industrial Biological Research Association. (2005);43(10):1451-1459), resulting in the reduction in dosing volume, reduction in irritation from the high or low pH or the use of organic solvents needed for solubilization, and avoiding direct venous irritation from the drug itself (Colucci et al., Acute Pain. (2009); 11:15-21). The IV bolus administration of HPβCD-diclofenac is safe, and has a faster onset of analgesia (Leeson et al., Regional anesthesia and pain medicine. (2007);32(4):303-310; Christensen et al., Anesthesia progress. (2011);58(2):73-81). The small volume of HPβCD-diclofenac has the advantages of decreasing IV infusion time and also may permit less painful

deep intragluteal IM injection. HPβCD-diclofenac does not need to be reconstituted before use, which has potential cost savings as compared to previous formulations of diclofenac (Wallerstein, International Society for Pharmacoeconomic and Outcomes Research (ISPOR), 2007). HPβCD-diclofenac was generally well tolerated in this study.

In summary, this study suggest that HPβCD-diclofenac may be administered to patients in these special populations (elderly, obese, renally or hepatically insufficient) at the usual dose and schedule without a need for dose reduction, but with upwards dose adjustment to maintain equivalent exposure for those with higher weight.

## Claims

**1.** A pharmaceutical composition comprising:

a. 50 mg or less of diclofenac or a pharmaceutically acceptable diclofenac salt; and
b. hydroxypropyl-beta-cyclodextrin (HPβCD);

for use in treating post-operative moderate to severe pain assessed by a visual analog scale (VAS), resulting from orthopedic surgery in a mammal having hepatic or renal impairment, wherein the composition is intended for parenteral administration, wherein moderate to severe pain is a pain intensity of ≥ 50 mm on a 1-100 mm VAS.

**2.** A pharmaceutical composition comprising:

a. 50 mg or less of diclofenac or a pharmaceutically acceptable diclofenac salt; and
b. hydroxypropyl-beta-cyclodextrin (HPβCD);

for use in treating post-operative moderate to severe pain assessed by a visual analog scale (VAS), resulting from abdominal or pelvic surgery in a mammal having hepatic or renal impairment, wherein the composition is intended for parenteral administration, wherein moderate to severe pain is a pain intensity of ≥ 50 mm on a 1-100 mm VAS.

**3.** The composition for use according to claim 1, wherein the mammal has a high-risk of an adverse reaction to analgesia.

**4.** The composition for use according to claim 1 or 2, wherein the mammal weighs at least 210 lbs (95 kg).

**5.** The composition for use according to claim 1 or 2, wherein the pharmaceutical composition comprises 37.5 mg or less of diclofenac or a pharmaceutically acceptable diclofenac salt; or
wherein the pharmaceutical composition comprises 18.75 mg or less of diclofenac or a pharmaceutically acceptable diclofenac salt; or
wherein the pharmaceutical composition comprises 9.375 mg or less of diclofenac or a pharmaceutically acceptable diclofenac salt.

**6.** The composition for use according to claim 1, wherein the composition is intended to be administered with an amount of rescue medication.

**7.** The composition for use according to claim 2, wherein the composition is intended to be administered with an amount of rescue medication.

**8.** The composition for use according to claim 1 or 2, wherein moderate pain is a pain intensity of ≥ 50mm and < 70 mm.

**9.** The composition for use according to claim 1 or 2, wherein severe pain is a pain intensity of ≥ 70 mm

## Patentansprüche

**1.** Pharmazeutische Zusammensetzung, umfassend:

a. 50 mg oder weniger Diclofenac oder eines pharmazeutisch verträglichen Diclofenac-Salzes; und
b. Hydroxypropyl-beta-cyclodextrin (HPβCD);

für die Verwendung bei der Behandlung von postoperativem mäßigem bis schwerem Schmerz, beurteilt auf einer

visuellen Analogskala (VAS), als Folge von orthopädischer Chirurgie bei einem Säuger mit Leber- oder Nierenfunktionsstörung, wobei die Zusammensetzung für die parenterale Verabreichung vorgesehen ist, wobei mäßiger bis schwerer Schmerz eine Schmerzintensität von ≥ 50 mm auf einer VAS von 1-100 mm ist.

**2.** Pharmazeutische Zusammensetzung, umfassend:

a. 50 mg oder weniger Diclofenac oder eines pharmazeutisch verträglichen Diclofenac-Salzes; und
b. Hydroxypropyl-beta-cyclodextrin (HPβCD);

für die Verwendung bei der Behandlung von postoperativem mäßigem bis schwerem Schmerz, beurteilt auf einer visuellen Analogskala (VAS), als Folge von Abdominal- oder Beckenchirurgie bei einem Säuger mit Leber- oder Nierenfunktionsstörung, wobei die Zusammensetzung für die parenterale Verabreichung vorgesehen ist, wobei mäßiger bis schwerer Schmerz eine Schmerzintensität von ≥ 50 mm auf einer VAS von 1-100 mm ist.

**3.** Zusammensetzung für die Verwendung gemäß Anspruch 1, wobei der Säuger ein hohes Risiko einer unerwünschten Reaktion auf Analgesie aufweist.

**4.** Zusammensetzung für die Verwendung gemäß Anspruch 1 oder 2, wobei der Säuger wenigstens 210 lbs (95 kg) wiegt.

**5.** Zusammensetzung für die Verwendung gemäß Anspruch 1 oder 2, wobei die pharmazeutische Zusammensetzung 37,5 mg oder weniger Diclofenac oder eines pharmazeutisch verträglichen Diclofenac-Salzes umfasst; oder
wobei die pharmazeutische Zusammensetzung 18,75 mg oder weniger Diclofenac oder eines pharmazeutisch verträglichen Diclofenac-Salzes umfasst; oder
wobei die pharmazeutische Zusammensetzung 9,375 mg oder weniger Diclofenac oder eines pharmazeutisch verträglichen Diclofenac-Salzes umfasst.

**6.** Zusammensetzung für die Verwendung gemäß Anspruch 1, wobei die Zusammensetzung für die Verabreichung mit einer Menge an Notfallmedikation vorgesehen ist.

**7.** Zusammensetzung für die Verwendung gemäß Anspruch 2, wobei die Zusammensetzung für die Verabreichung mit einer Menge an Notfallmedikation vorgesehen ist.

**8.** Zusammensetzung für die Verwendung gemäß Anspruch 1 oder 2, wobei mäßiger Schmerz eine Schmerzintensität von ≥ 50 mm und < 70 mm ist.

**9.** Zusammensetzung für die Verwendung gemäß Anspruch 1 oder 2, wobei schwerer Schmerz eine Schmerzintensität von ≥ 70 mm ist.

## Revendications

**1.** Composition pharmaceutique comprenant :

a. 50 mg ou moins de diclofénac ou d'un sel pharmaceutiquement acceptable de diclofénac ; et
b. de l'hydroxypropyl-bêta-cyclodextrine (HPβCD) ; pour utilisation dans le traitement d'une douleur postopératoire modérée à sévère évaluée suivant l'échelle visuelle analogique (EVA), résultant d'une opération chirurgicale orthopédique chez un mammifère souffrant d'insuffisance hépatique ou rénale, où la composition est destinée à une administration parentérale, où une douleur modérée à sévère est une intensité de douleur de ≥ 50 mm sur une EVA de 1 à 100 mm.

**2.** Composition pharmaceutique comprenant :

a. 50 mg ou moins de diclofénac ou d'un sel pharmaceutiquement acceptable de diclofénac ; et
b. de l'hydroxypropyl-bêta-cyclodextrine (HPβCD) ;

pour utilisation dans le traitement d'une douleur postopératoire modérée à sévère évaluée suivant l'échelle visuelle analogique (EVA), résultant d'une opération chirurgicale abdominale ou pelvienne chez un mammifère souffrant

d'insuffisance hépatique ou rénale, où la composition est destinée à une administration parentérale, où une douleur modérée à sévère est une intensité de douleur de ≥ 50 mm sur une EVA de 1 à 100 mm.

**3.** Composition pour utilisation selon la revendication 1, où le mammifère présente un risque élevé de réaction indésirable à l'analgésie.

**4.** Composition pour utilisation selon la revendication 1 ou 2, où le mammifère pèse au moins 95 kg (210 livres) .

**5.** Composition pour utilisation selon la revendication 1 ou 2, où la composition pharmaceutique comprend 37,5 mg ou moins de diclofénac ou d'un sel pharmaceutiquement acceptable de diclofénac ; ou
où la composition pharmaceutique comprend 18,75 mg ou moins de diclofénac ou d'un sel pharmaceutiquement acceptable de diclofénac ; ou
où la composition pharmaceutique comprend 9,375 mg ou moins de diclofénac ou d'un sel pharmaceutiquement acceptable de diclofénac.

**6.** Composition pour utilisation selon la revendication 1, où la composition est destinée à être administrée avec une quantité de médicament de secours.

**7.** Composition pour utilisation selon la revendication 2, où la composition est destinée à être administrée avec une quantité de médicament de secours.

**8.** Composition pour utilisation selon la revendication 1 ou 2, où une douleur modérée est une intensité de douleur de ≥ 50 mm et < 70 mm.

**9.** Composition pour utilisation selon la revendication 1 ou 2, où une douleur sévère est une intensité de douleur de ≥ 70 mm.

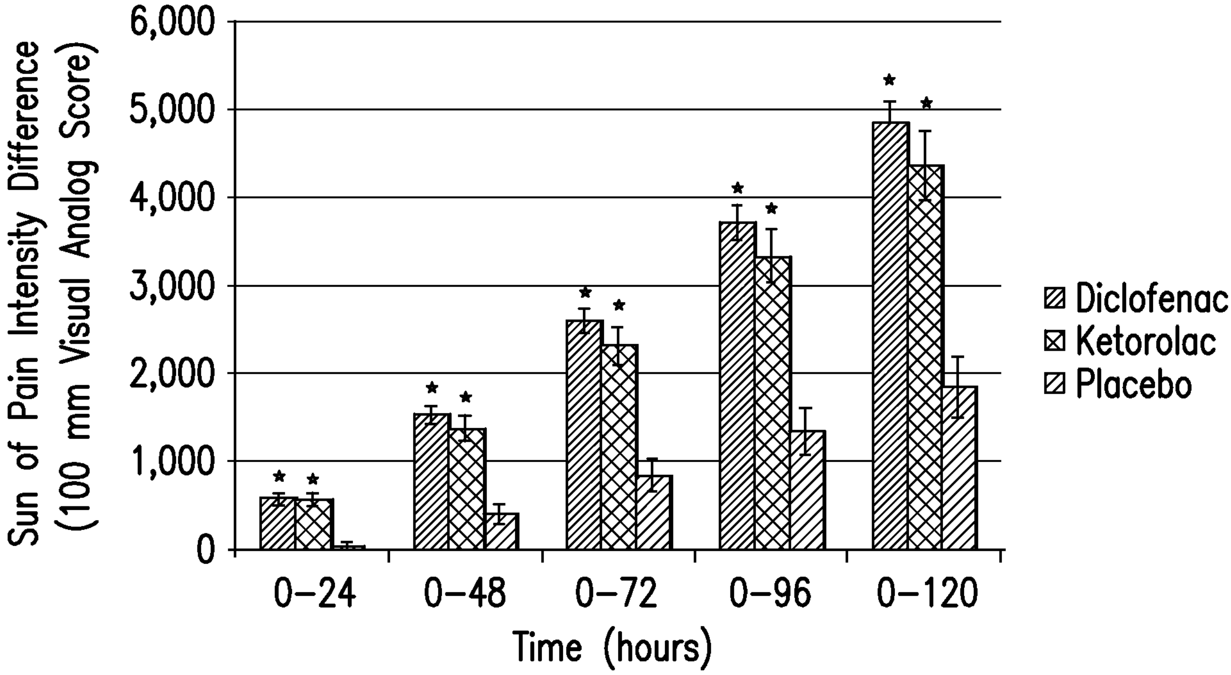
6,000
5,000
4,000
3,000
2,000
1,000
0
Sun of Pain Intensity Difference
(100 mm Visual Analog Score)
0–24
0–48
0–72
0–96
0–120
Time (hours)
Diclofenac
Ketorolac
Placebo

FIG. 1

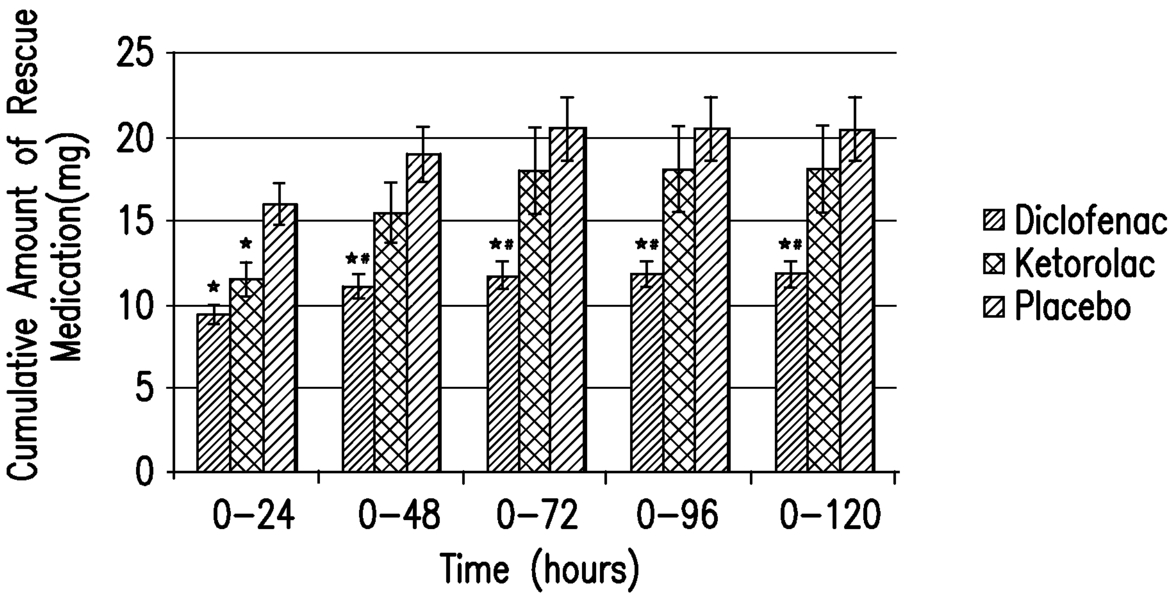
Cumulative Amount of Rescue Medication(mg)
25
20
15
10
5
0
0-24
0-48
0-72
0-96
0-120
Time (hours)
Diclofenac
Ketorolac
Placebo

FIG. 2

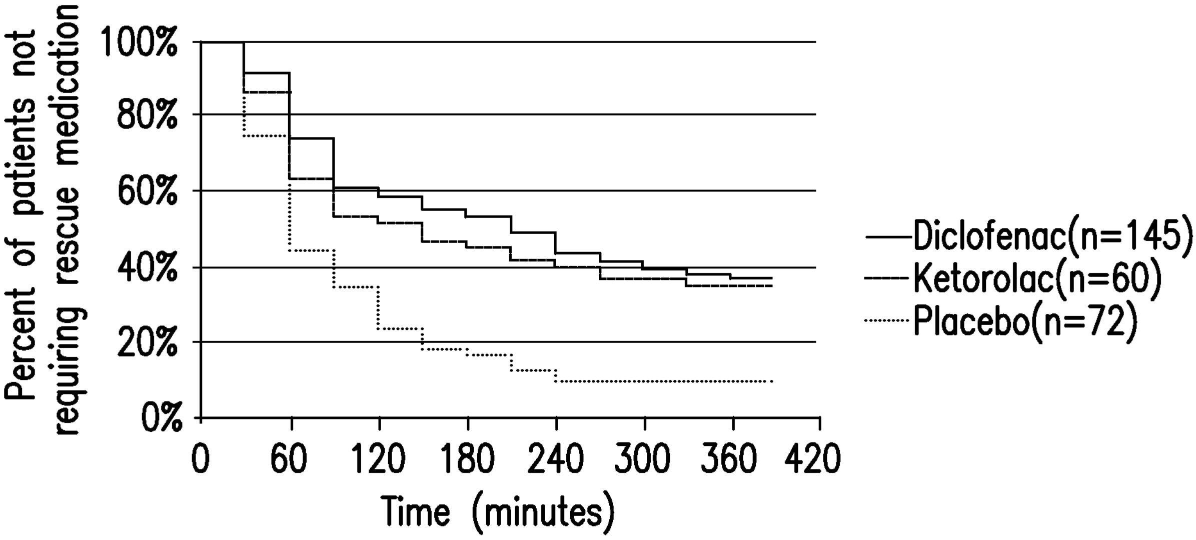
Percent of patients not requiring rescue medication
100%
80%
60%
40%
20%
0%
0
60
120
180
240
300
360
420
Time (minutes)
Diclofenac(n=145)
Ketorolac(n=60)
Placebo(n=72)

FIG. 3

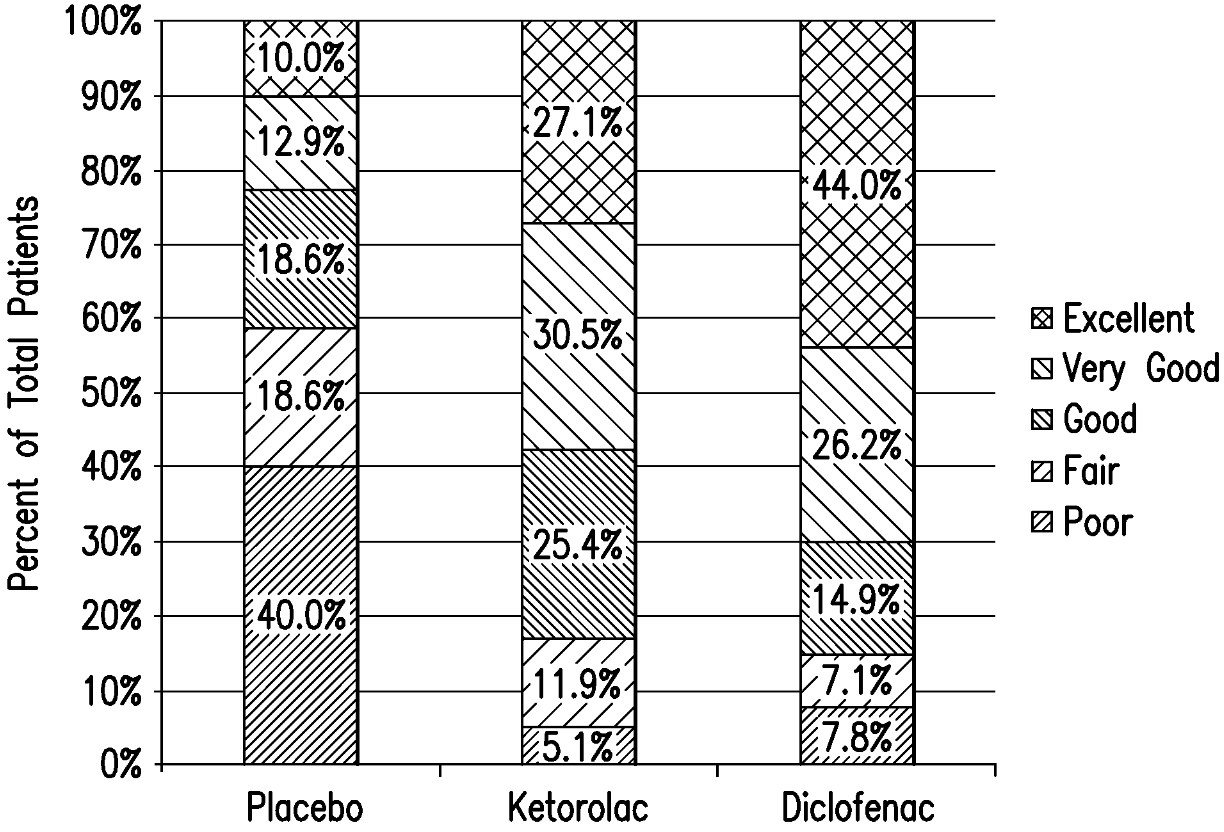
100%
90%
80%
70%
60%
50%
40%
30%
20%
10%
0%
Percent of Total Patients
10.0%
12.9%
18.6%
18.6%
40.0%
27.1%
30.5%
25.4%
11.9%
5.1%
44.0%
26.2%
14.9%
7.1%
7.8%
Placebo
Ketorolac
Diclofenac
Excellent
Very Good
Good
Fair
Poor

FIG. 4

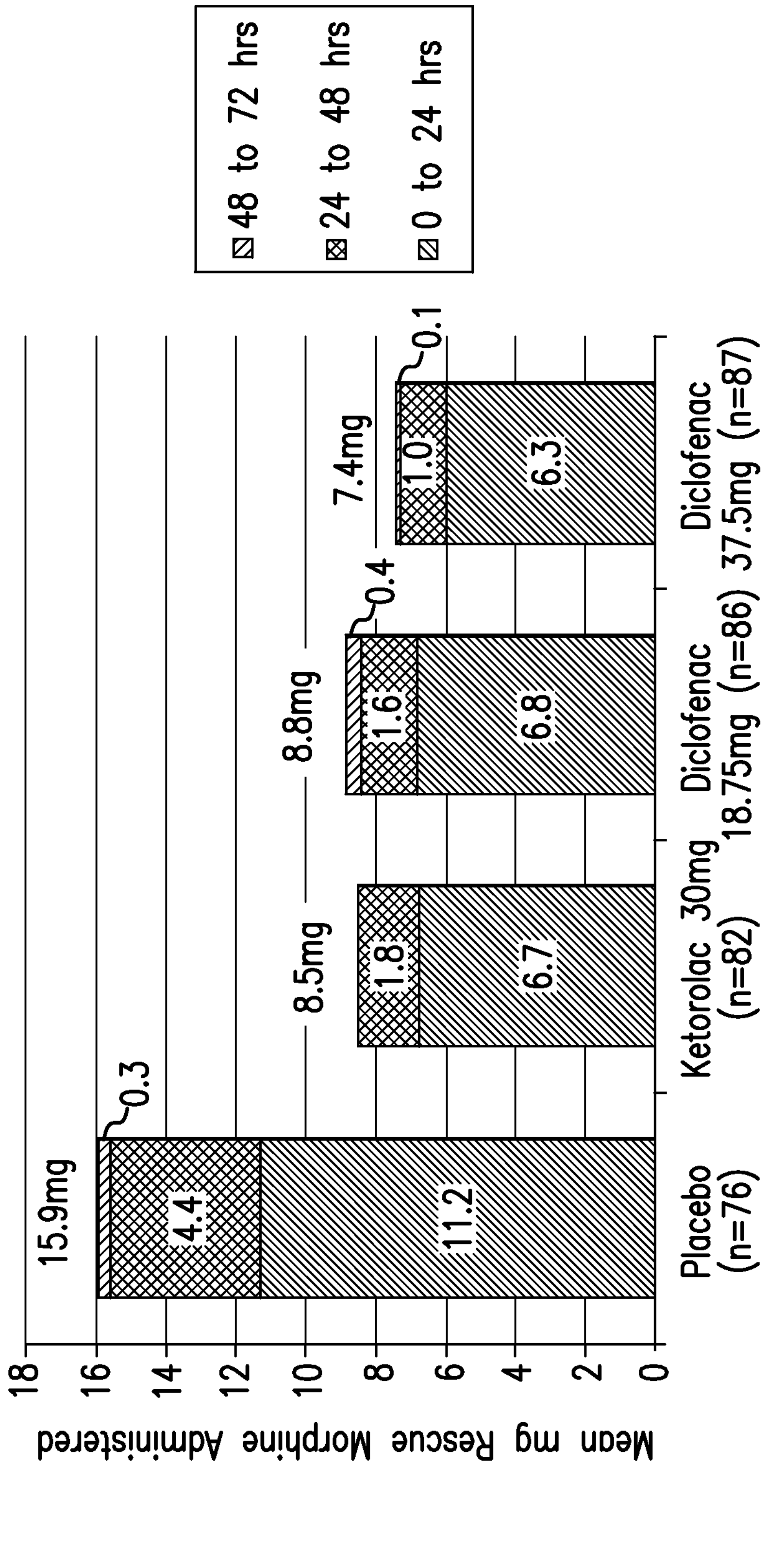

Mean mg Rescue Morphine Administered
18
16
14
12
10
8
6
4
2
0
15.9mg
0.3
4.4
11.2
8.5mg
1.8
6.7
8.8mg
0.4
1.6
6.8
7.4mg
0.1
1.0
6.3
Placebo (n=76)
Ketorolac 30mg (n=82)
Diclofenac 18.75mg (n=86)
Diclofenac 37.5mg (n=87)
48 to 72 hrs
24 to 48 hrs
0 to 24 hrs


FIG. 5

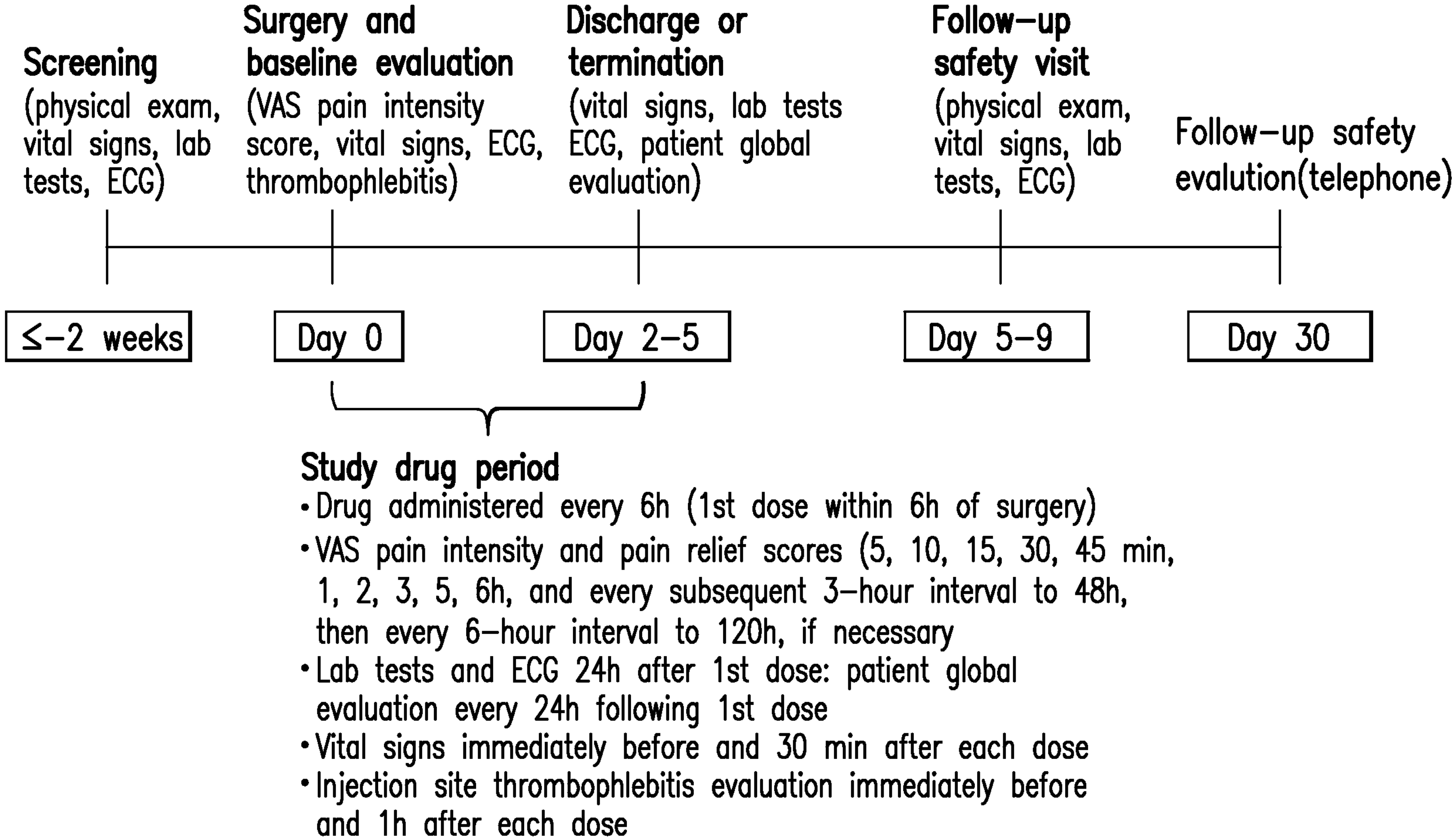
Screening
(physical exam, vital signs, lab tests, ECG)
Surgery and baseline evaluation
(VAS pain intensity score, vital signs, ECG, thrombophlebitis)
Discharge or termination
(vital signs, lab tests ECG, patient global evaluation)
Follow-up safety visit
(physical exam, vital signs, lab tests, ECG)
Follow-up safety evalution(telephone)
≤-2 weeks
Day 0
Day 2-5
Day 5-9
Day 30
Study drug period
• Drug administered every 6h (1st dose within 6h of surgery)
• VAS pain intensity and pain relief scores (5, 10, 15, 30, 45 min, 1, 2, 3, 5, 6h, and every subsequent 3-hour interval to 48h, then every 6-hour interval to 120h, if necessary
• Lab tests and ECG 24h after 1st dose: patient global evaluation every 24h following 1st dose
• Vital signs immediately before and 30 min after each dose
• Injection site thrombophlebitis evaluation immediately before and 1h after each dose

FIG. 6

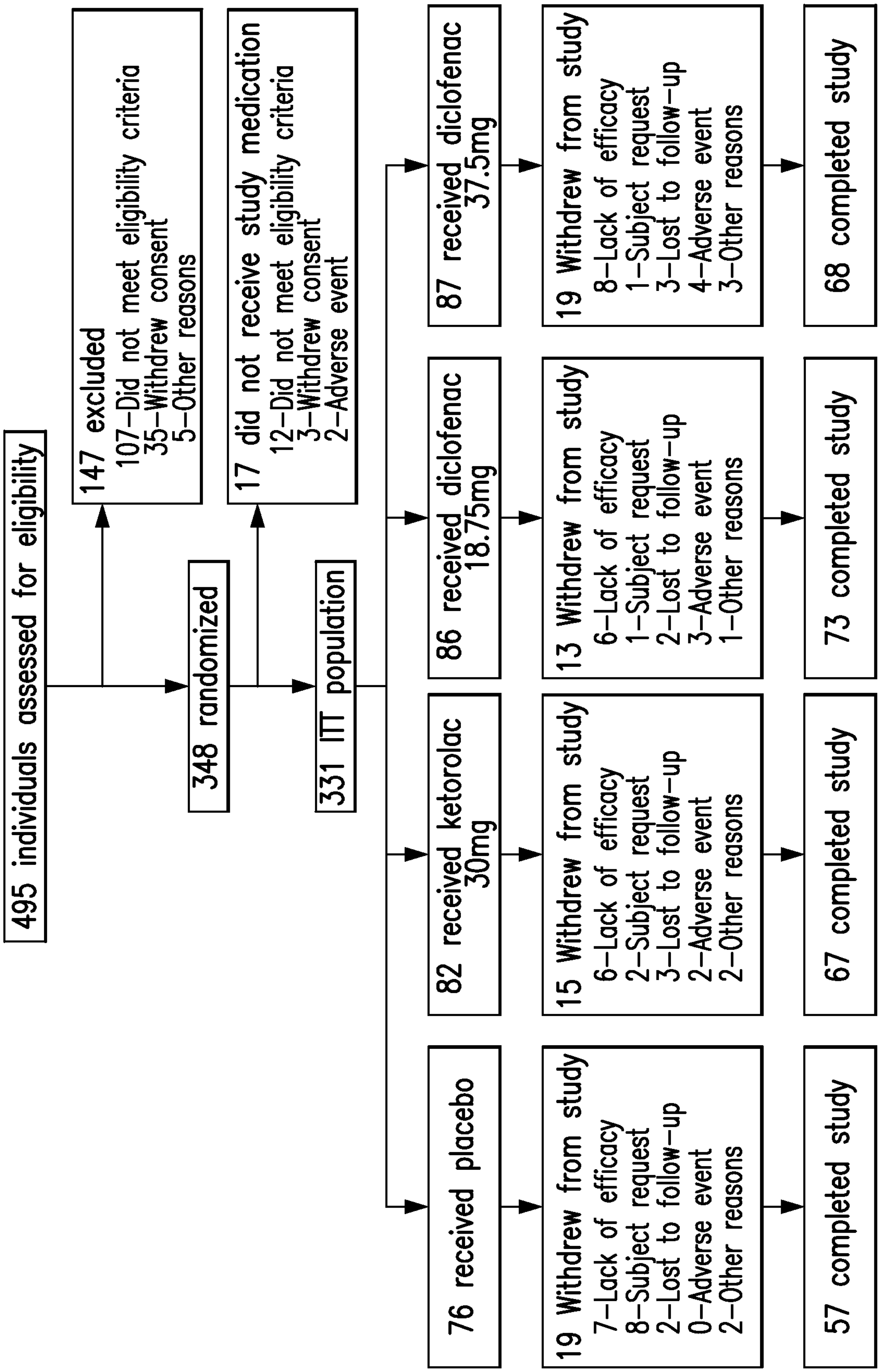
495 individuals assessed for eligibility
147 excluded
107–Did not meet eligibility criteria
35–Withdrew consent
5–Other reasons
348 randomized
17 did not receive study medication
12–Did not meet eligibility criteria
3–Withdrew consent
2–Adverse event
331 ITT population
76 received placebo
82 received ketorolac 30mg
86 received diclofenac 18.75mg
87 received diclofenac 37.5mg
19 Withdrew from study
7–Lack of efficacy
8–Subject request
2–Lost to follow–up
0–Adverse event
2–Other reasons
15 Withdrew from study
6–Lack of efficacy
2–Subject request
3–Lost to follow–up
2–Adverse event
2–Other reasons
13 Withdrew from study
6–Lack of efficacy
1–Subject request
2–Lost to follow–up
3–Adverse event
1–Other reasons
19 Withdrew from study
8–Lack of efficacy
1–Subject request
3–Lost to follow–up
4–Adverse event
3–Other reasons
57 completed study
67 completed study
73 completed study
68 completed study

FIG. 7

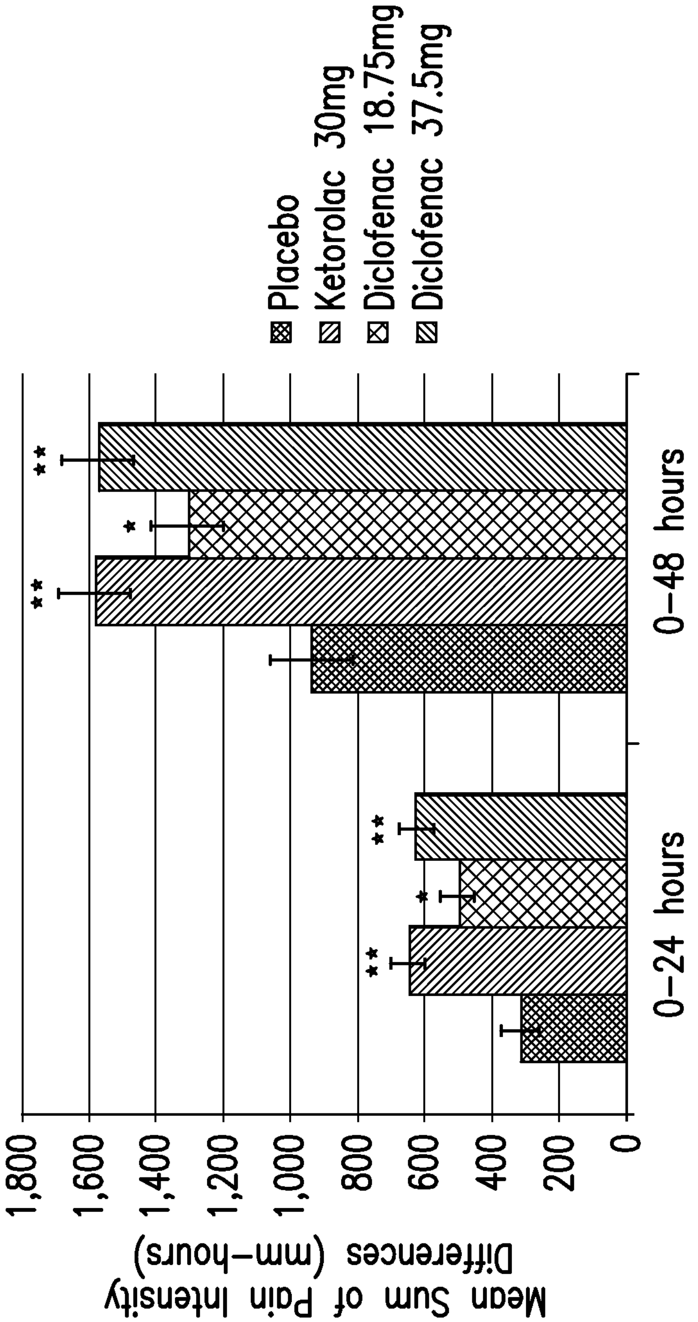
Mean Sum of Pain Intensity Differences (mm–hours)
1,800
1,600
1,400
1,200
1,000
800
600
400
200
0
0–24 hours
0–48 hours
Placebo
Ketorolac 30mg
Diclofenac 18.75mg
Diclofenac 37.5mg

FIG. 8

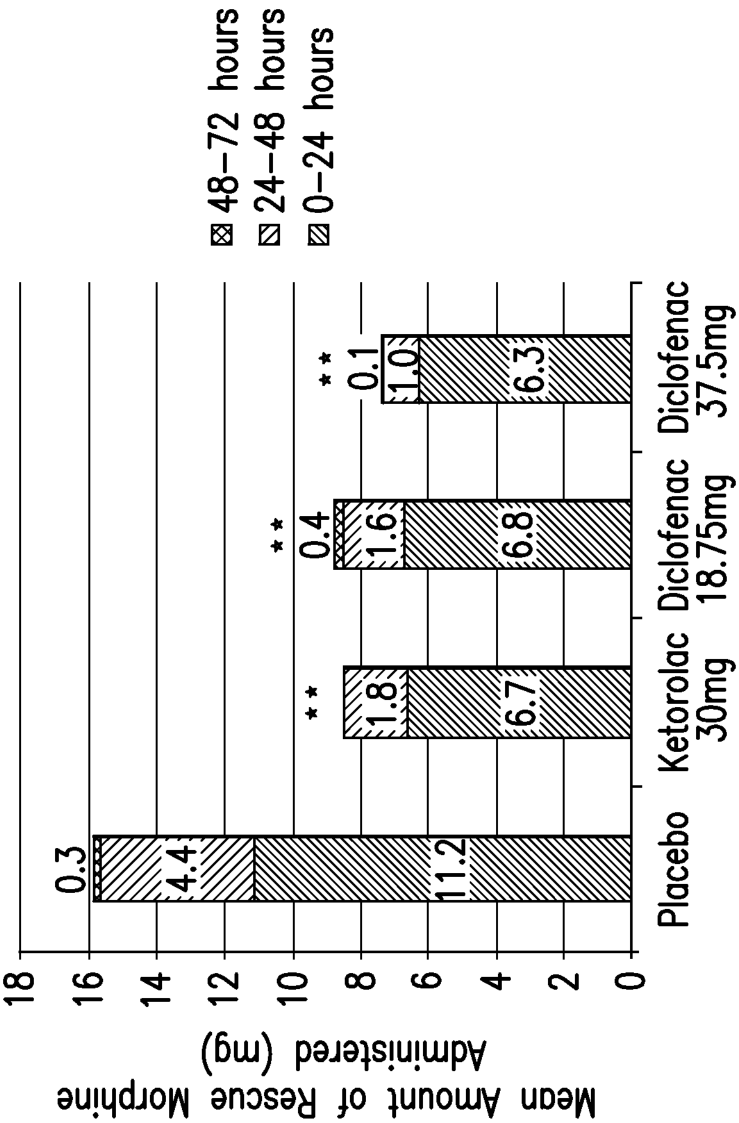
Mean Amount of Rescue Morphine Administered (mg)
18
16
14
12
10
8
6
4
2
0
Placebo
Ketorolac 30mg
Diclofenac 18.75mg
Diclofenac 37.5mg
0.3
4.4
11.2
**
1.8
6.7
**
0.4
1.6
6.8
**
0.1
1.0
6.3
48–72 hours
24–48 hours
0–24 hours

FIG. 9

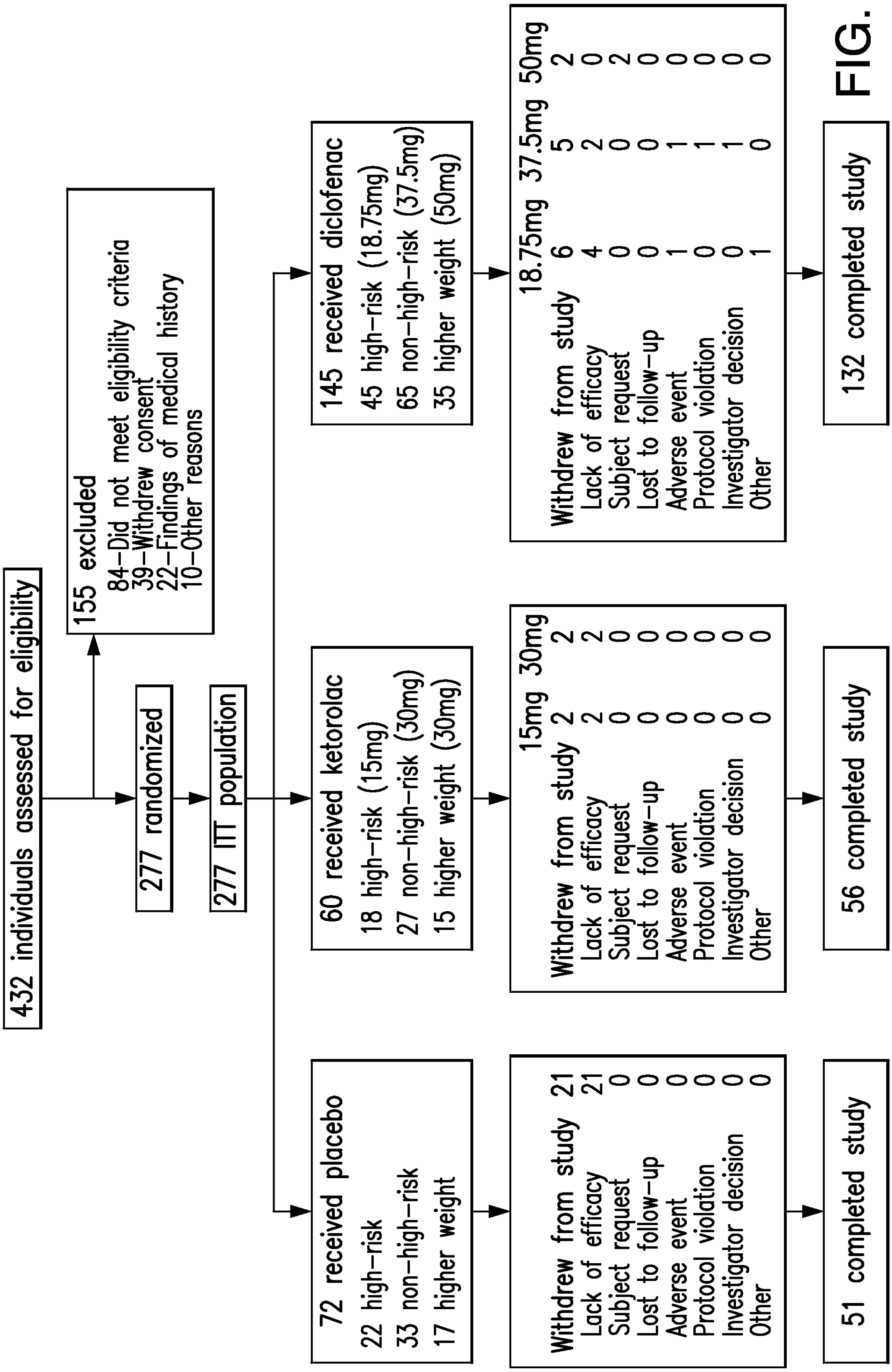

432 individuals assessed for eligibility
155 excluded
84–Did not meet eligibility criteria
39–Withdrew consent
22–Findings of medical history
10–Other reasons
277 randomized
277 ITT population
72 received placebo
22 high–risk
33 non–high–risk
17 higher weight
60 received ketorolac
18 high–risk (15mg)
27 non–high–risk (30mg)
15 higher weight (30mg)
145 received diclofenac
45 high–risk (18.75mg)
65 non–high–risk (37.5mg)
35 higher weight (50mg)
Withdrew from study 21
Lack of efficacy 21
Subject request 0
Lost to follow–up 0
Adverse event 0
Protocol violation 0
Investigator decision 0
Other 0
15mg 30mg
Withdrew from study 2 2
Lack of efficacy 2 2
Subject request 0 0
Lost to follow–up 0 0
Adverse event 0 0
Protocol violation 0 0
Investigator decision 0 0
Other 0 0
18.75mg 37.5mg 50mg
Withdrew from study 6 5 2
Lack of efficacy 4 2 0
Subject request 0 0 2
Lost to follow–up 0 0 0
Adverse event 1 1 0
Protocol violation 0 1 0
Investigator decision 0 1 0
Other 1 0 0
51 completed study
56 completed study
132 completed study


FIG. 10

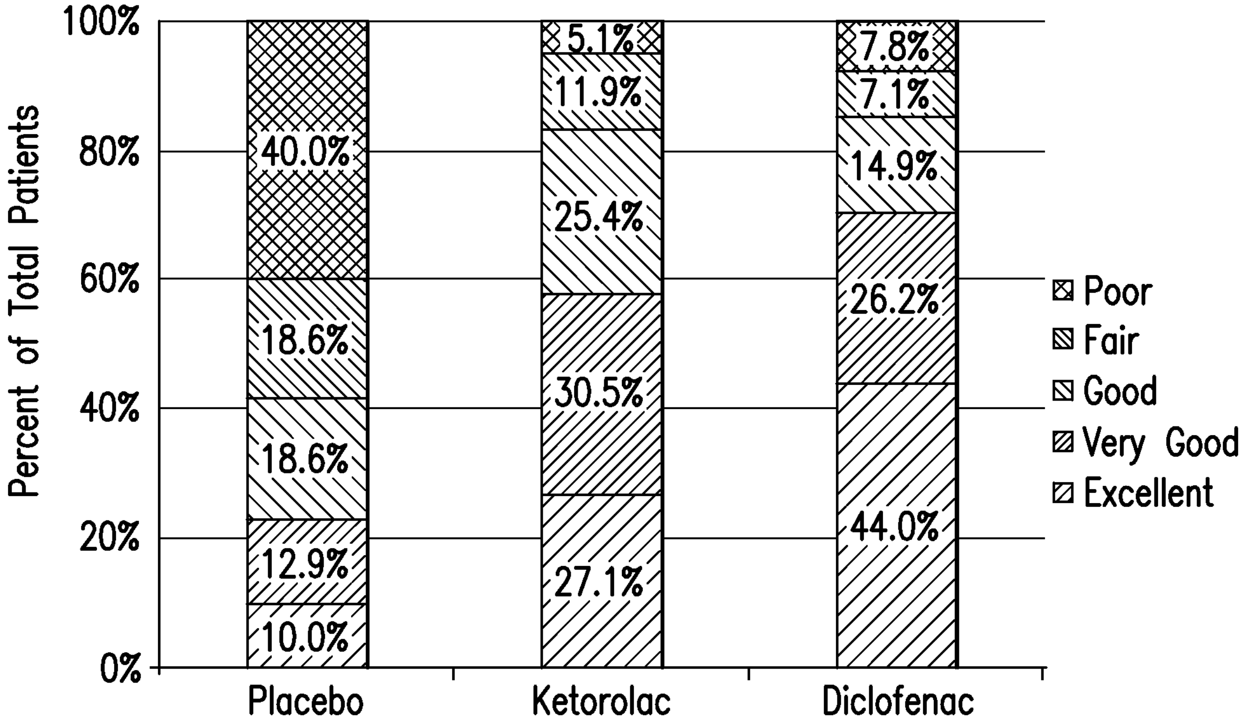
100%
80%
60%
40%
20%
0%
Percent of Total Patients
40.0%
18.6%
18.6%
12.9%
10.0%
5.1%
11.9%
25.4%
30.5%
27.1%
7.8%
7.1%
14.9%
26.2%
44.0%
Poor
Fair
Good
Very Good
Excellent
Placebo
Ketorolac
Diclofenac

FIG. 11

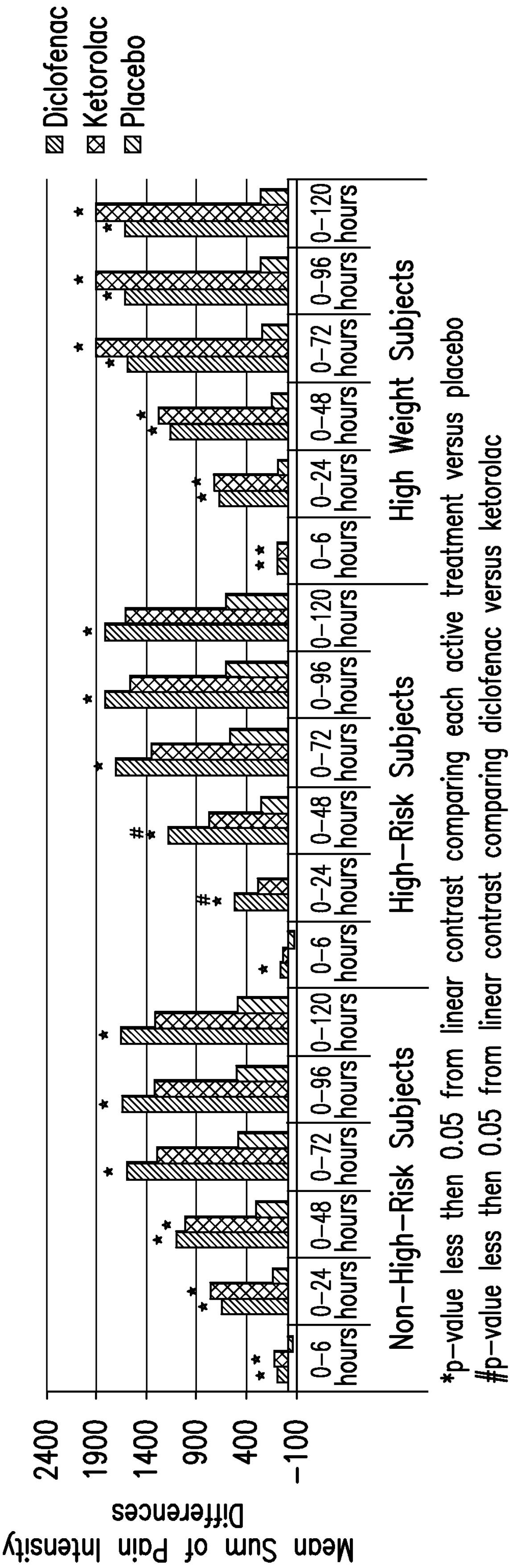
Diclofenac
Ketorolac
Placebo
Mean Sum of Pain Intensity Differences
2400
1900
1400
900
400
−100
0−6 hours
0−24 hours
0−48 hours
0−72 hours
0−96 hours
0−120 hours
Non−High−Risk Subjects
High−Risk Subjects
High Weight Subjects
*p−value less then 0.05 from linear contrast comparing each active treatment versus placebo
#p−value less then 0.05 from linear contrast comparing diclofenac versus ketorolac

FIG. 12

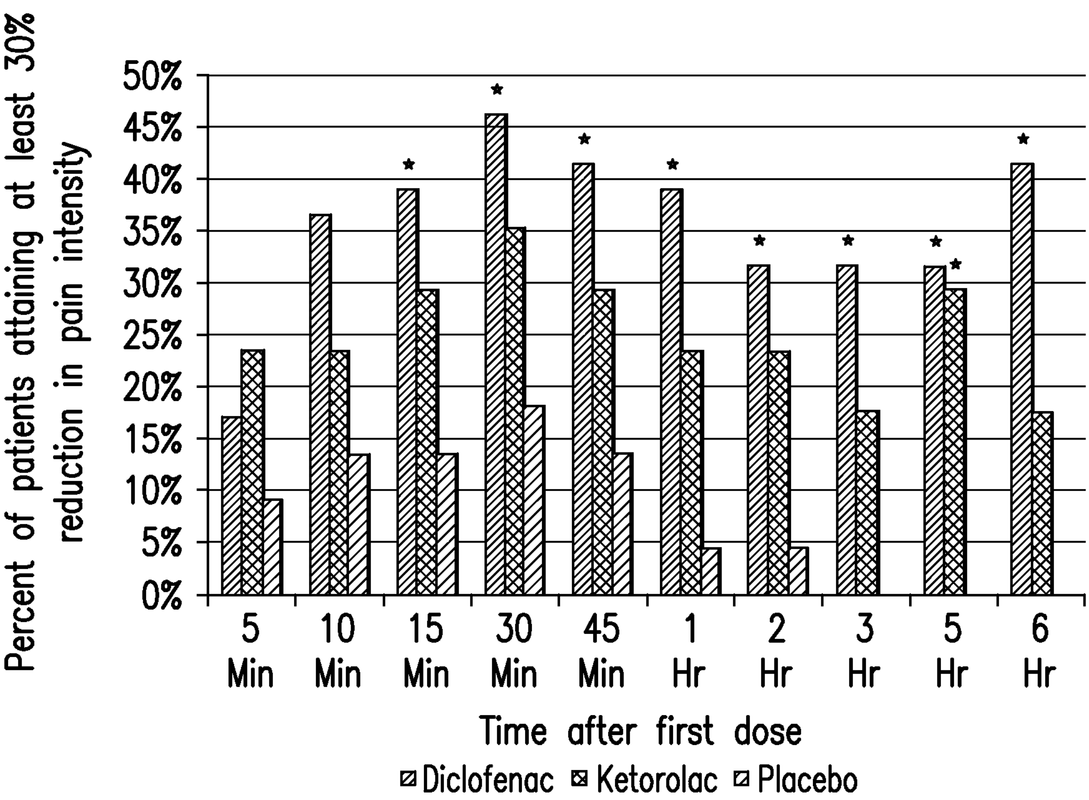


FIG. 13A

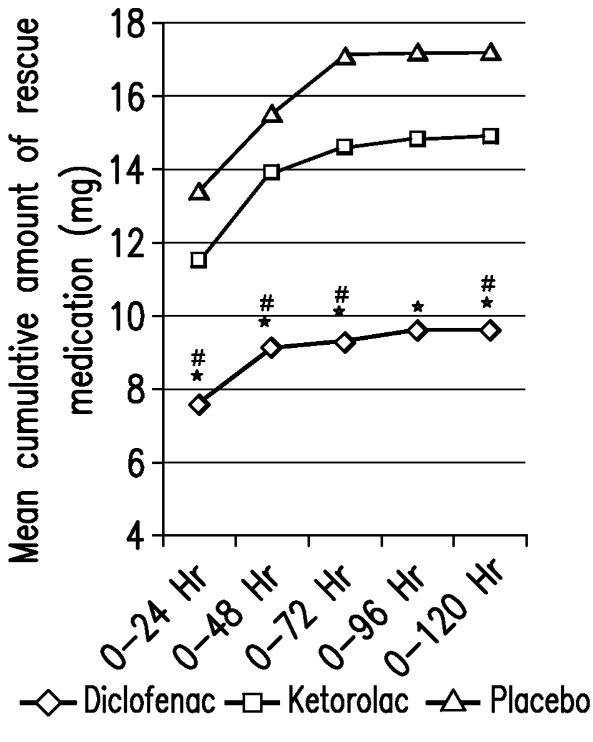


FIG. 13B

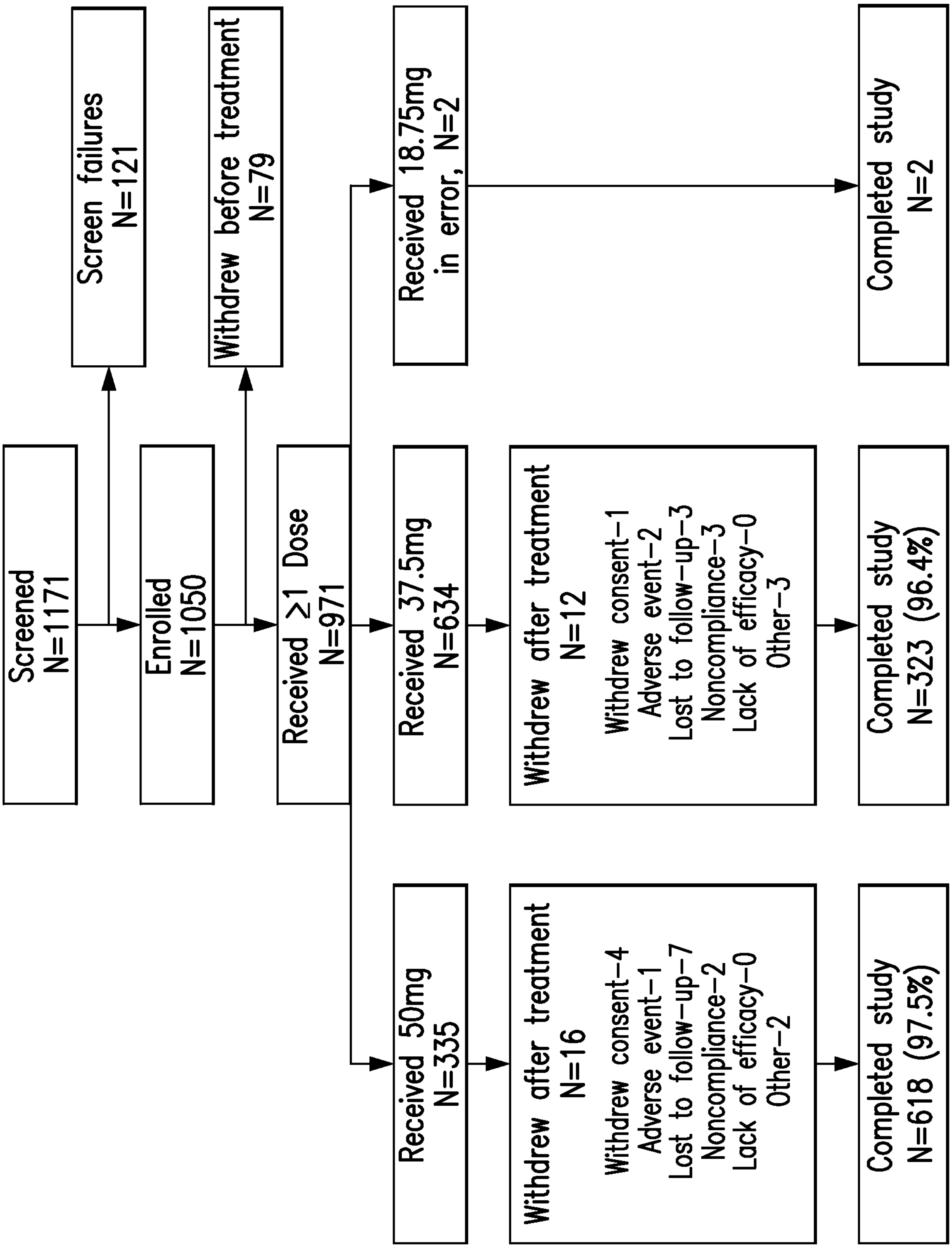
Screened
N=1171
Screen failures
N=121
Enrolled
N=1050
Withdrew before treatment
N=79
Received ≥1 Dose
N=971
Received 18.75mg
in error, N=2
Completed study
N=2
Received 37.5mg
N=634
Withdrew after treatment
N=12
Withdrew consent–1
Adverse event–2
Lost to follow–up–3
Noncompliance–3
Lack of efficacy–0
Other–3
Completed study
N=323 (96.4%)
Received 50mg
N=335
Withdrew after treatment
N=16
Withdrew consent–4
Adverse event–1
Lost to follow–up–7
Noncompliance–2
Lack of efficacy–0
Other–2
Completed study
N=618 (97.5%)

FIG. 14

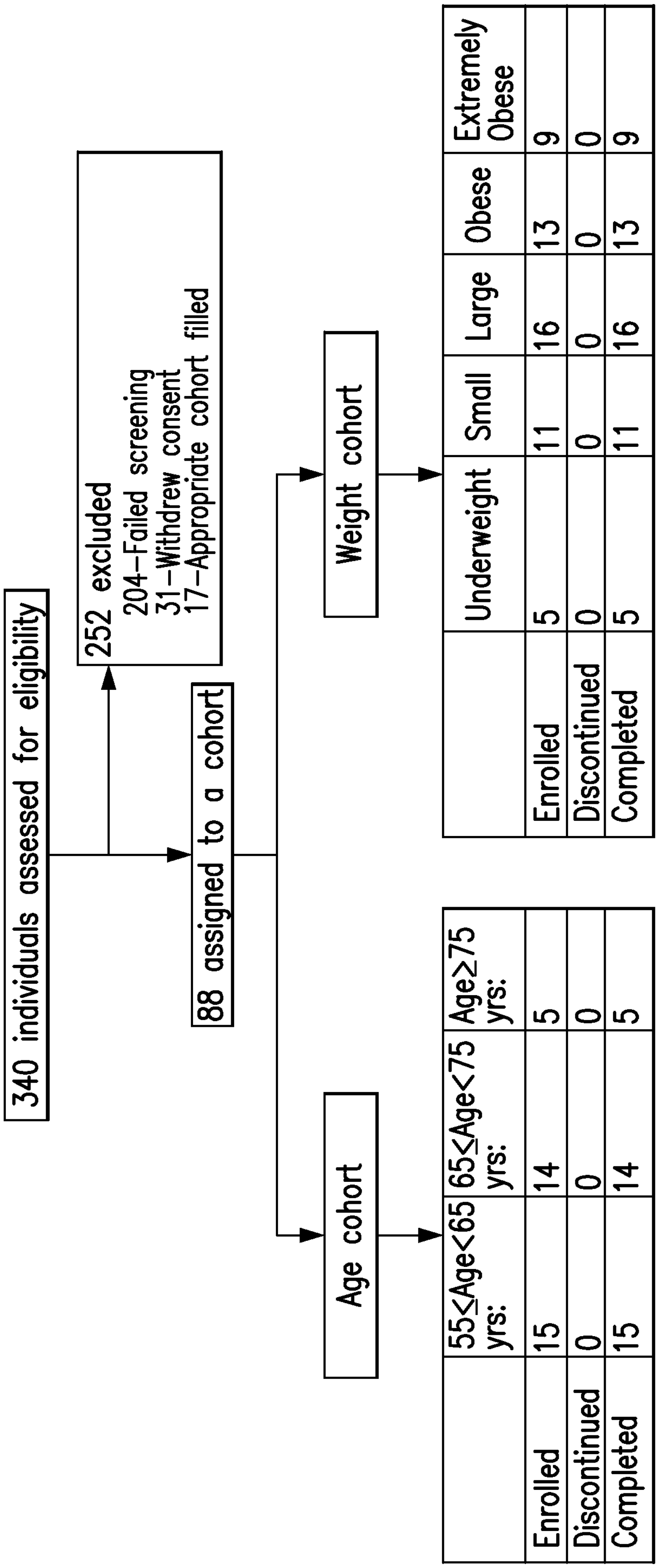


| | 55≤Age<65 yrs: | 65≤Age<75 yrs: | Age≥75 yrs: |
|---|---|---|---|
| Enrolled | 15 | 14 | 5 |
| Discontinued | 0 | 0 | 0 |
| Completed | 15 | 14 | 5 |

| | Underweight | Small | Large | Obese | Extremely Obese |
|---|---|---|---|---|---|
| Enrolled | 5 | 11 | 16 | 13 | 9 |
| Discontinued | 0 | 0 | 0 | 0 | 0 |
| Completed | 5 | 11 | 16 | 13 | 9 |

FIG. 15

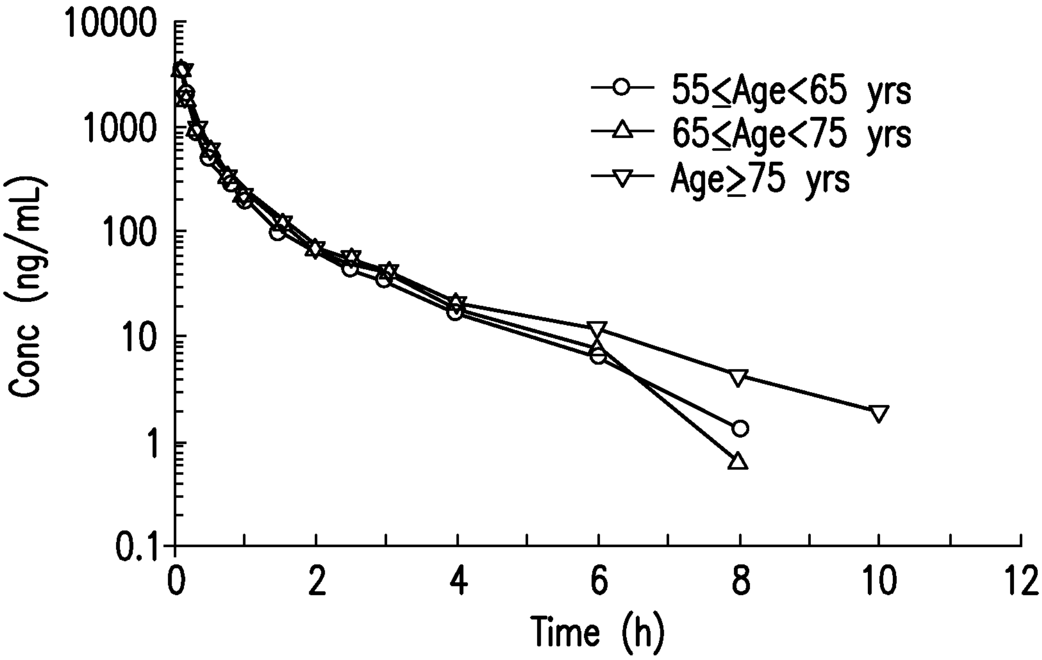


FIG. 16A

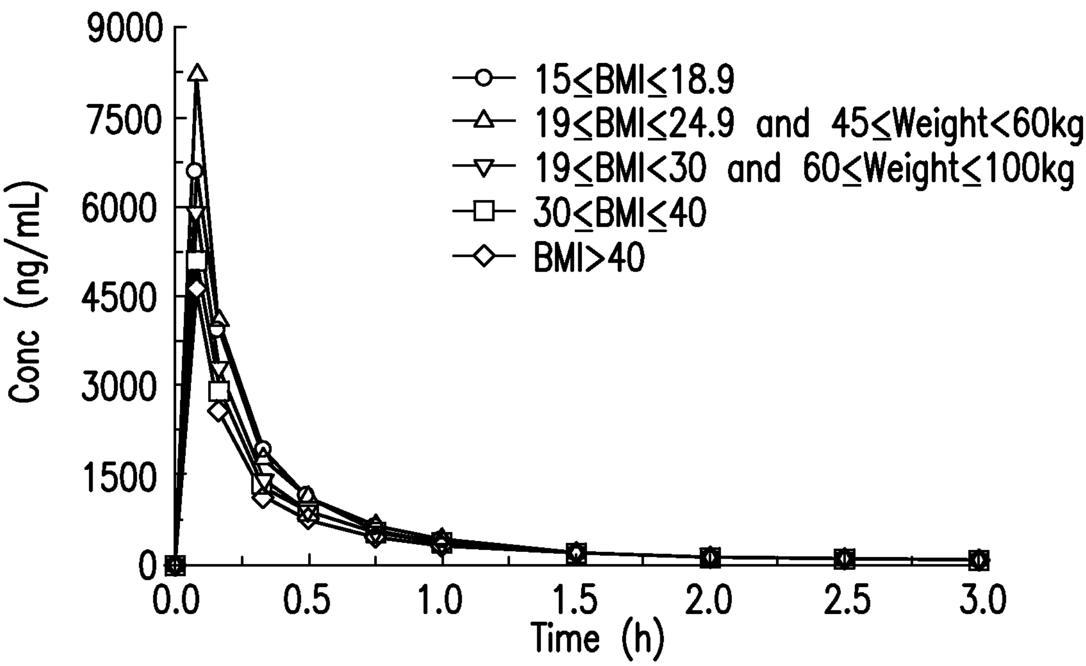


FIG. 16B

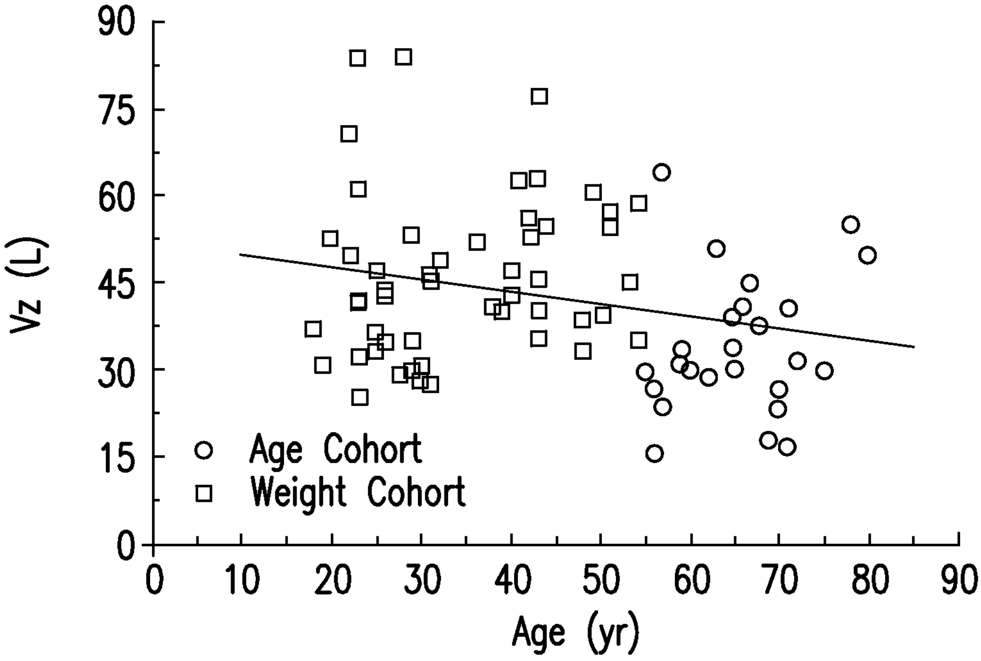


FIG. 17A

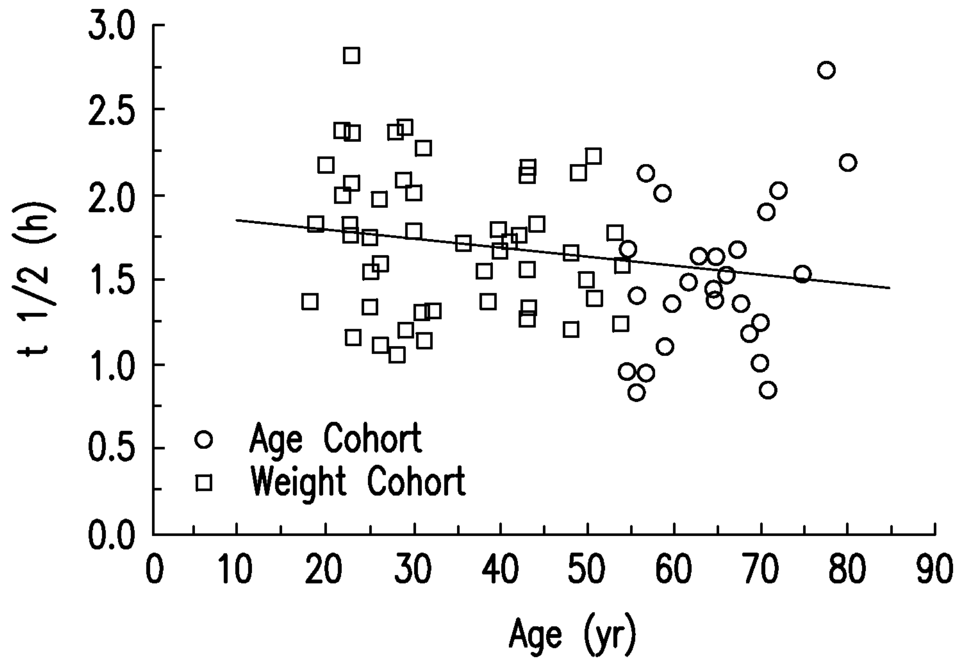


FIG. 17B

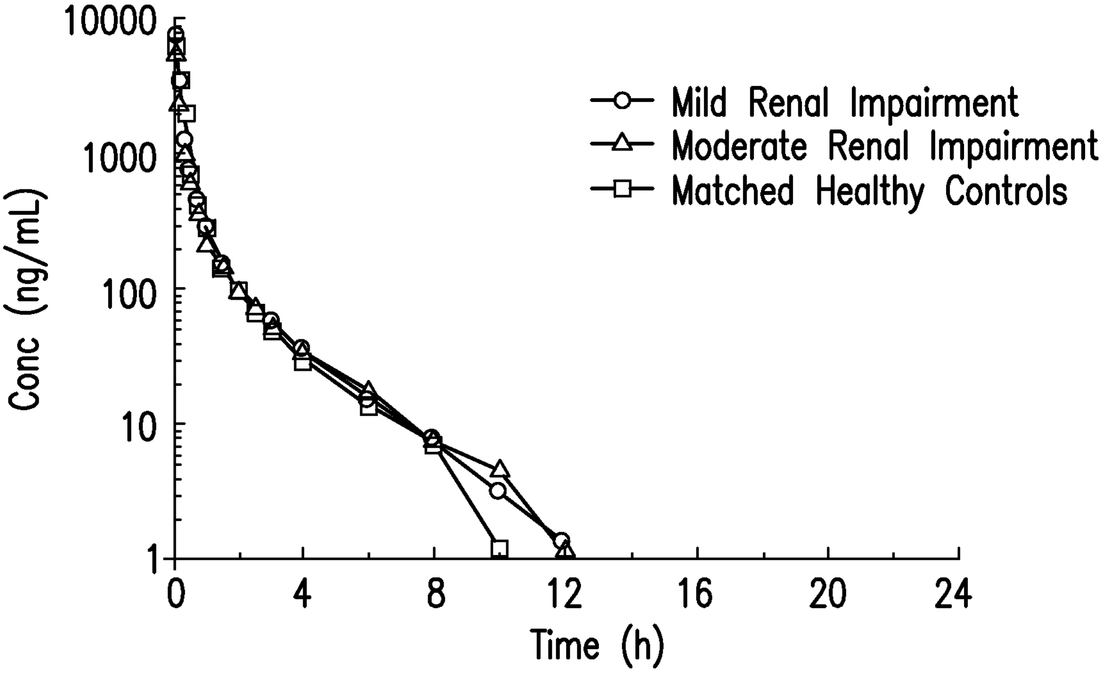


FIG. 18A

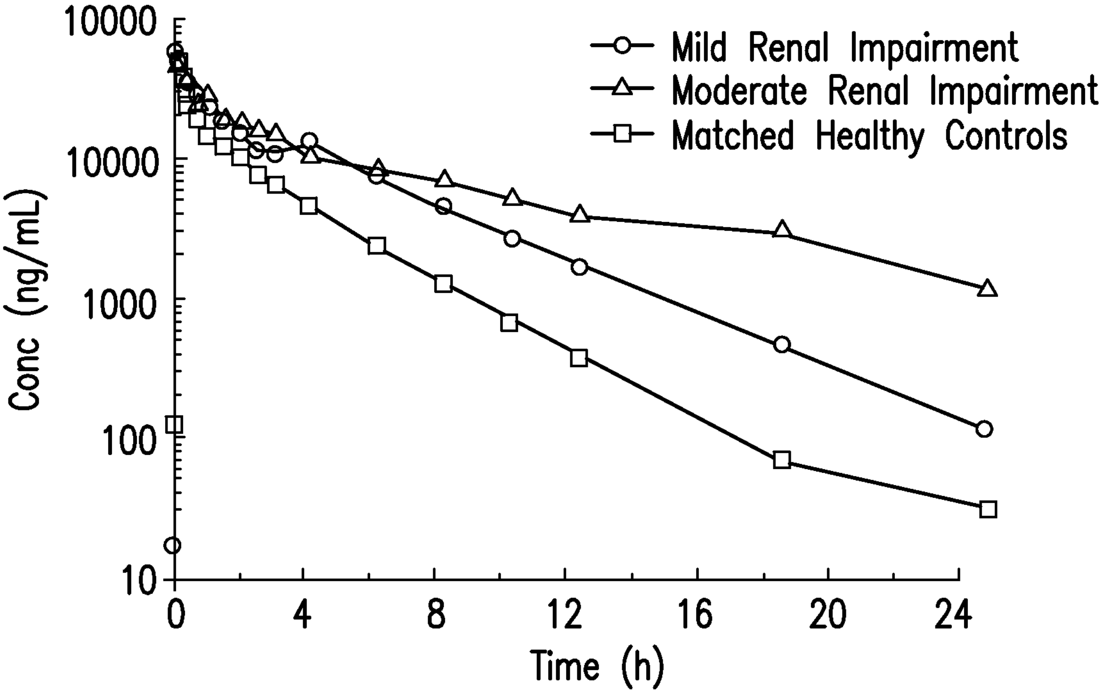


FIG. 18B

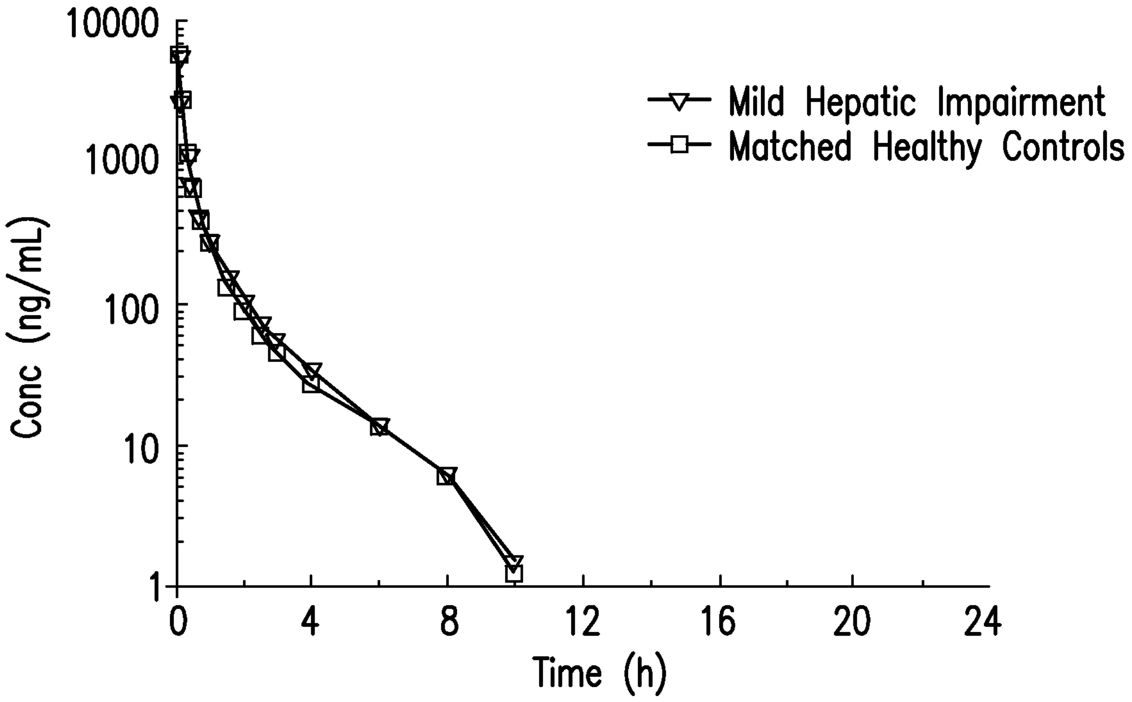


FIG. 19A

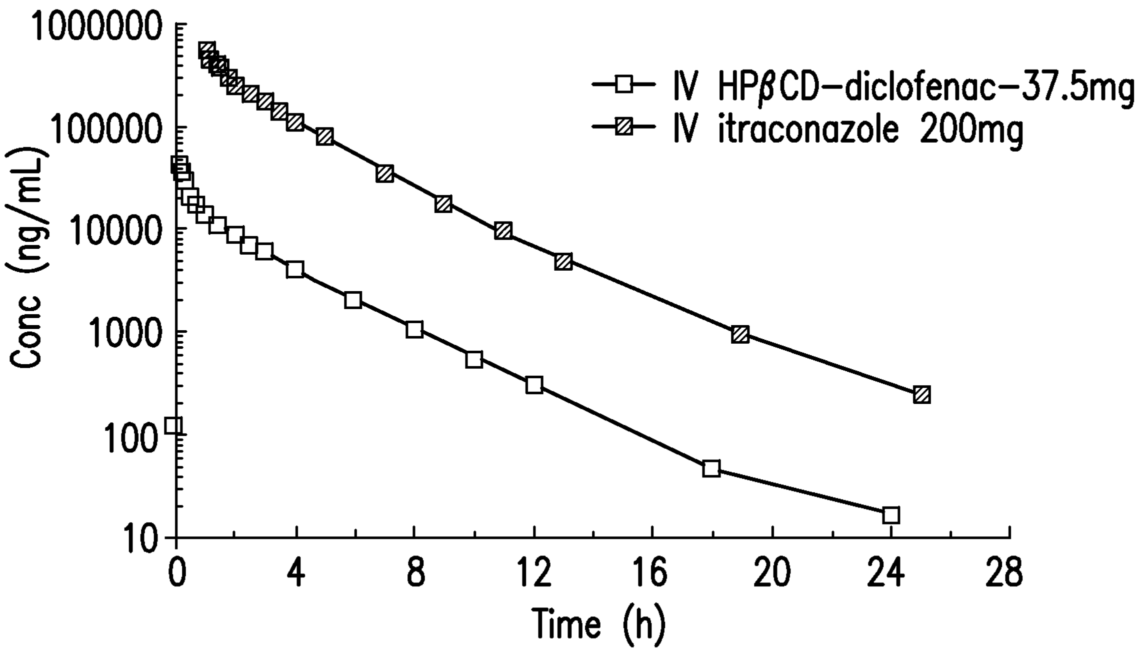


FIG. 19B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5679660 A
- US 20050238674 A1
- WO 2009089269 A
- US 2007232566 A
- EP 1574221 A
- US 5389681 A
- US 5674854 A
- US 20050238674 A
- US 6933289 B
- WO 2008110080 A
- WO 2003059393 A


### Non-patent literature cited in the description


- **LIU et al.** *Anesth. Analg.,* 2007, vol. 104 (3), 689-702
- **FALAGAS et al.** *The Lancet,* 2010, vol. 375 (9710), 248-251
- **CHELLY J et al.** *Presentation at the International Association for the Study of Pain 13th World Congress on Pain,* 2010
- **BAUER KA et al.** *J Clin Anesth.,* 2010, vol. 22 (7), 510-518
- **SIDERIS et al.** *Pharm Res,* 1994, vol. 11, 90-5
- **CHOUDHURY et al.** *Pharm Res,* 1993, vol. 10, 156-9
- **AICART et al.** *J Incl Phenom Macroc Chem,* 2003, vol. 47, 161-5
- **AL OMARI et al.** *Drug Dev Ind Pharm,* 2007, vol. 33, 1205-15
- **VOZONE et al.** *J Incl Phenom Macroc Chem,* 2002, vol. 44, 111-5
- **MORAES et al.** *Int J Pharm,* 2007, vol. 331, 99-106
- **LOFTSSON et al.** *Int J Pharm.,* 2005, vol. 302, 18-28
- **BHUTANI et al.** *JSci Ind Res,* 2007, 66830-4
- **ALEEM et al.** *J Pharm Biomed Anal,* 2008, vol. 47, 535-40
- **CHOWDARY et al.** *AAPS Pharm Sci Tech,* 2006, vol. 7 (79
- **LOFTSSON et al.** *Pharmazie,* 1994, vol. 49, 292-3
- **HIRAYAMA et al.** *Eur J Pharm Sci,* 1997, vol. 5, 23-30
- **LOFTSSON et al.** *Acta Pharm Nord,* 1989, vol. 1, 185-94
- **SOICA et al.** *Revista de Chimie,* 2007, vol. 58, 606-11
- **MALAEKEH-NIKOUEI et al.** *J Incl Phenom Macroc Chem,* 2007, vol. 59, 245-50
- **PIEL et al.** *Int J Pharm,* 2006, vol. 312, 75-82
- **MISIUK et al.** *Anal Lett,* 2008, vol. 41, 543-60
- **LOFTSSON et al.** *J Incl Phenom Macroc Chem,* 2007, vol. 57, 545-52
- **LE CORRE et al.** *Int J Pharm,* 1998, vol. 169, 221-8
- **MORIN et al.** *J Liq Chrom Rel Tech,* 2000, vol. 23, 727-39
- **LOFTSSON et al.** *J Pharm Sci,* 2002, vol. 91, 2307-16
- **CAPPELLO et al.** *Drug Dev Ind Pharm,* 2009, vol. 35, 877-86
- **PATEL et al.** *J Incl Phenom Macroc Chem,* 2008, vol. 60, 241-51
- **PALMIERI et al.** *S.T.P. Parma Sci,* 1997, vol. 7, 174-81
- **HOLVOET et al.** *Int J Pharm,* 2003, vol. 265, 13-26
- **MAITRE et al.** *Drug Dev Ind Pharm,* 2007, vol. 33, 311-26
- **VLACHOU et al.** *J Biomater Appl,* 2003, vol. 17, 197-206
- **AMMAR et al.** *Int J Pharm,* 2006, vol. 309, 129-38
- **ZHANG et al.** *Guangpuxue Yu Guangpu Fenxi,* 2008, vol. 28, 711-4
- **LOUKAS et al.** *J Pharm Biomed Anal,* 1997, vol. 16, 263-8
- **MURA et al.** *Int J Pharm,* 1998, vol. 166, 189-203
- **MASSON et al.** *Int J Pharm,* 1998, vol. 164, 45-55
- **LOFTSSON et al.** *Int J Pharm,* 1993, vol. 98, 225-30
- **BABOOTA et al.** *J Incl Phenom Macroc Chem,* 2005, vol. 51, 219-24
- **VALERO et al.** *J Incl Phenom Macroc Chem,* 1999, vol. 35, 663-77
- **MURA P et al.** *Int J Pharm,* 2003, vol. 260 (2), 293-302

- **FERNANDES et al.** *Eur J Pharm Sci,* 2002, vol. 15, 79-88
- **LOFTSSON et al.** *Int J Pharm,* 2005, vol. 302, 18-28
- **CHO et al.** *Int J Pharm,* 2008, vol. 349, 101-7
- **MAESTRELLI et al.** *J Incl Phenom Macroc Chem,* 2009, vol. 63, 17-25
- **LARSEN et al.** *J Pharm Sci,* 2005, vol. 94, 507-15
- **CHEN et al.** *Sepu,* 2004, vol. 22, 595-600
- **AL OMARI et al.** *J Pharm Biomed Anal,* 2006, vol. 41, 857-65
- **BADR-ELDIN et al.** *Eur J Pharm Biopharm,* 2008, vol. 70, 819-27
- **SHEWALE et al.** *Int J Chem Sci,* 2008, vol. 6, 1449-54
- **ZIA et al.** *Pharm Res,* 2001, vol. 18, 667-73
- **TRAPANI et al.** *J Pharm Sci,* 2000, vol. 89, 1443-51
- **CHRISTENSEN et al.** *Anesth Prog,* 2011, vol. 58, 73-81
- **LEESON et al.** *Reg Anesth Pain Med,* 2007, vol. 32, 303-10
- **ALEXANDER et al.** *J Clin Anesth,* 2002, vol. 14, 187-92
- **GAN et al.** *Acute Pain,* 2008, vol. 10, 165-166
- **STOKES et al.** *BMC Health Serv Res,* 2011, vol. 11, 135
- **GHAFERI et al.** *New Engl J Med,* 2009, vol. 1361, 1368-1375
- **KHETERPAL et al.** *Anesthesiology,* 2009, vol. 110, 505-515
- **LEESON et al.** *Reg Anesth Pain Med,* 2007, vol. 32, 303-310
- **LEVEY et al.** *Annals of internal medicine,* 1999, vol. 130 (6), 461-470
- **AUBRUN et al.** *Clinical anaesthesiology,* 2007, vol. 21 (1), 109-127
- **BAUER et al.** *Journal of clinical anesthesia,* 2010, vol. 22 (7), 510-518
- **ELIA et al.** *Anesthesiology,* 2005, vol. 103 (6), 1296-1304
- *Physicians' Desk Reference,* 2012
- **DILGER et al.** *Journal of clinical pharmacology,* 2002, vol. 42 (9), 985-994
- **FREDMAN et al.** *Anesthesia and analgesia,* 1999, vol. 88 (1), 149-154
- **BAKSHI et al.** *medical research and opinion.,* 1991, vol. 12 (7), 459-465
- **BROGDEN et al.** *Drugs,* 1980, vol. 20 (1), 24-48
- **BREWSTER et al.** *Advanced drug delivery reviews,* 2007, vol. 59 (7), 645-666
- **GOULD et al.** *Food and chemical toxicology: an international journal published for the British Industrial Biological Research Association,* 2005, vol. 43 (10), 1451-1459
- **COLUCCI et al.** *Acute Pain,* 2009, vol. 11, 15-21
- **LEESON et al.** *Regional anesthesia and pain medicine,* 2007, vol. 32 (4), 303-310
- **CHRISTENSEN et al.** *Anesthesia progress,* 2011, vol. 58 (2), 73-81
- **WALLERSTEIN.** *International Society for Pharmacoeconomic and Outcomes Research (ISPOR),* 2007